(19) Europäisches Patentamt
European Patent Office
Office européen des brevets

(11) EP 4 299 069 A1

(12) **EUROPEAN PATENT APPLICATION**
published in accordance with Art. 153(4) EPC

(43) Date of publication:
**03.01.2024 Bulletin 2024/01**

(21) Application number: **22798954.8**

(22) Date of filing: **06.05.2022**

(51) International Patent Classification (IPC):
***A61K 38/12** (2006.01)* ***A61K 47/10** (2017.01)*
***A61K 47/14** (2017.01)* ***A61K 47/26** (2006.01)*
***A61K 47/44** (2017.01)* ***A61P 35/00** (2006.01)*
***A61P 43/00** (2006.01)*

(52) Cooperative Patent Classification (CPC):
**A61K 38/12; A61K 47/10; A61K 47/14; A61K 47/26; A61K 47/44; A61P 35/00; A61P 43/00**

(86) International application number:
**PCT/JP2022/019540**

(87) International publication number:
**WO 2022/234850 (10.11.2022 Gazette 2022/45)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.05.2021 JP 2021079015**

(71) Applicant: **CHUGAI SEIYAKU KABUSHIKI KAISHA
Tokyo 115-8543 (JP)**

(72) Inventors:
- **UETO, Takamitsu
Gotemba-shi, Shizuoka 412-8513 (JP)**
- **KUBOKI, Yukari
Gotemba-shi, Shizuoka 412-8513 (JP)**

(74) Representative: **Vossius & Partner
Patentanwälte Rechtsanwälte mbB
Siebertstrasse 3
81675 München (DE)**

(54) **PREPARATION CONTAINING CYCLIC PEPTIDE COMPOUND AND METHOD FOR PRODUCING SAME**

(57) The present invention relates to a composition containing: a compound represented by the following Formula (1):

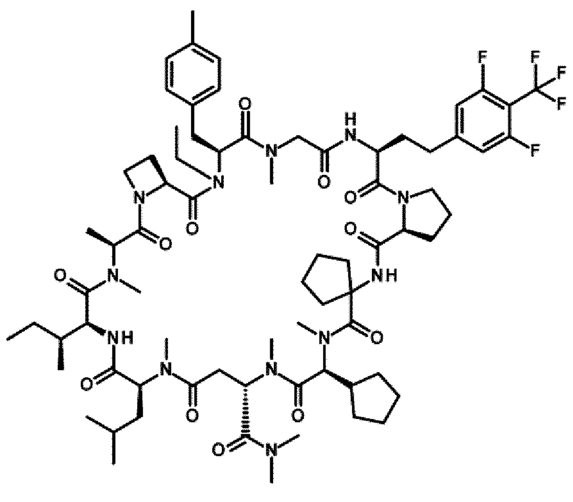

(1)

or, a salt thereof, or a solvate thereof; a liquid additive; and optionally an oily component.

Fig.1

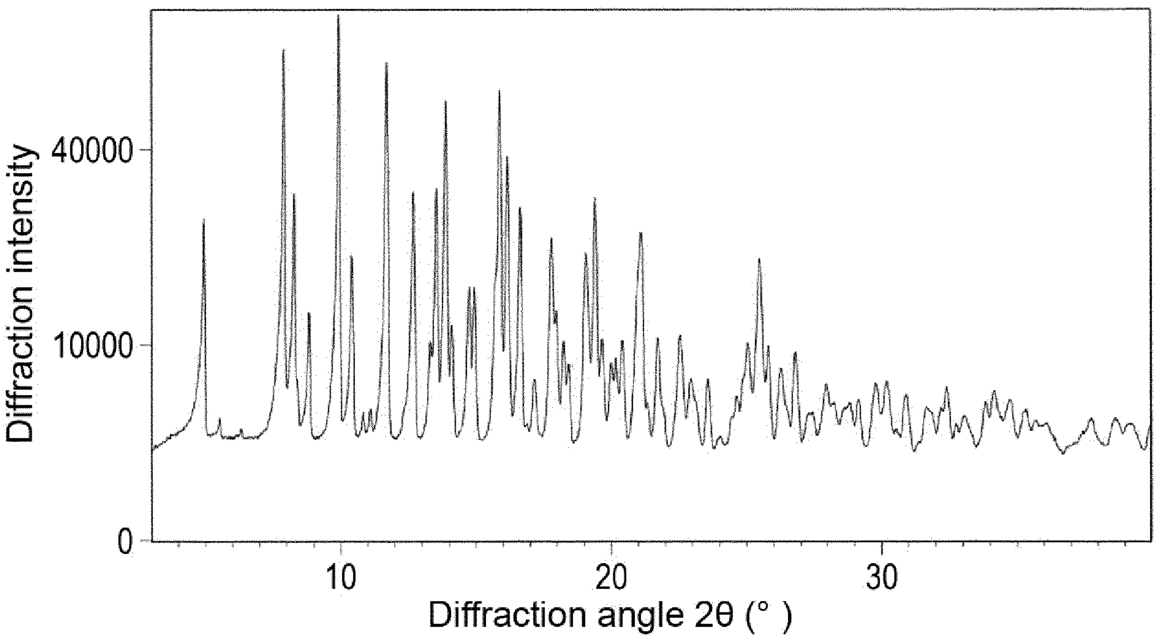
Diffraction intensity
40000
10000
0
10
20
30
Diffraction angle 2θ (° )

## Description

[Technical Field]

The present invention relates to a formulation containing a cyclic peptide compound having a KRAS inhibitory activity and a method for producing the same.

[Background Art]

Conventional compounds used as oral drugs have been considered to be desirable to have a molecular weight of 500 g/mol or less, as is known as the Lipinski rule (Non Patent Literature 1). In recent years, it has become known that compounds having a molecular weight of more than 500 g/mol may contribute to the inhibition of the interaction at the surface of a target protein, such as protein-protein interaction, which is difficult to interact by conventional small molecule compounds and has been referred to as tough targets. These molecules are called medium-molecular compounds (with a molecular weight of 500 to 2000 g/mol), which are neither small molecules with molecular weight of 500 g/mol or less that have been used as oral drugs, nor polymeric molecules with a molecular weight of more than 100000 g/mol, such as antibody pharmaceuticals, and have gained prominence as new modalities that can realize drug discovery against tough targets (Non Patent Literature 2).

Peptides composed of natural amino acids, such as insulin used in the treatment of hyperglycemia, have poor metabolic stability and have conventionally been difficult to be developed as oral drugs. However, it has been found that the metabolic stability and membrane permeability of the peptide is improved by cyclizing the peptide and using a non-natural amino acid such as N-methyl amino acid in the peptide (Non Patent Literatures 3 and 4).

Among cyclic peptides containing non-natural amino acids, it has become known that cyclic peptides particularly containing N-substituted amino acids may have metabolic stability or membrane permeability, i.e., may have drug-likeness (Patent Literature 1).

It has been suggested that library compounds of cyclic peptides containing non-natural amino acids are useful for the creation of inhibitors of protein-protein interactions (Non Patent Literature 5).

Conditions have also been clarified for cyclic peptides containing non-natural amino acids to be drug-like molecules having a high degree of membrane permeability and metabolic stability that can be used as pharmaceuticals, thus the attention of cyclic peptides as pharmaceutical modality has further increased (Patent Literatures 2 and 3).

Patent Literature 6 discloses a cyclic peptide exhibiting a pharmacological action. Patent Literature 6 also discloses the formulation technology of cyclosporine. Studies have also been conducted on the oral formulation of somatostatin (Non Patent Literature 6).

[Citation List]

[Patent Literature]

[Patent Literature 1] International Publication No. WO 2013/100132
[Patent Literature 2] International Publication No. WO 2018/225864
[Patent Literature 3] International Publication No. WO 2020/122182
[Patent Literature 4] International Publication No. WO 2012/122059
[Patent Literature 5] International Publication No. WO 2017/181061
[Patent Literature 6] International Publication No. WO 2018/031730
[Patent Literature 7] International Publication No. WO 2016/071515

[Non Patent Literature]

[Non Patent Literature 1] Adv. Drug Del. Rev. 1997, 23, 3-25.
[Non Patent Literature 2] Future Med. Chem., 2009, 1, 1289-1310.
[Non Patent Literature 3] Acc. Chem. Res., 2008, 41, 1331-1342.
[Non Patent Literature 4] Angew. Chem. Int. Ed., 2013, 52, 254-269.
[Non Patent Literature 5] Chem. Rev., 2019, 119, 10360-10391.
[Non Patent Literature 6] Ther. Deliv. (2017) 8(10), 867-878.

[Summary of Invention]

[Technical Problem]

An object of the present invention is to provide a formulation of a cyclic compound containing a non-natural amino acid. Another object of the present invention is to provide a method of producing a formulation of a cyclic compound having an efficient RAS inhibitory effect.

Patent Literature 4 describes inhibition of binding of RAS and SOS. Patent Literature 5 describes peptides that compete with compounds that bind RAS. However, these literatures do not indicate pharmacological effects, especially effects on tumor cells. Furthermore, these literatures do not describe drug-like peptides.

Patent Literatures 6 and 7 describe the formulation of cyclic peptides, but both describe the formulation of only specific compounds.

Non Patent Literature 2 describes a peptide that is used as a pharmaceutical, but does not describe a drug-like peptide or a peptide useful for RAS mutant cancer.

Non Patent Literatures 3 and 4 describe that peptides containing N-methylamino acids may be used as pharmaceuticals, but do not describe a peptide useful for RAS mutant cancer.

Non Patent Literature 5 describes that a cyclic peptide may be used as a pharmaceutical, but does not describe a peptide useful for RAS mutant cancer.

Non Patent Literature 6 illustrates somatostatin and describes the elements necessary for oral formulation, but describes only about somatostatin.

Thus, it is necessary to provide an excellent formulation as pharmaceuticals for cyclic compounds containing non-natural amino acids.

[Solution to Problem]

The present inventors have intensively studied to solve the above-described problems. As a result, it has been found that the addition of a specific additive to the active ingredient can achieve a formulation that can be used as a pharmaceutical. It has also been found that surfactants, preferably a combination of hydrophobic and hydrophilic surfactants, are effective as the specific additive. Furthermore, it has been found that the addition of an oily component can make them more effective as a formulation. It has been found that a mixture of the active ingredient and these additives can be formulated and used in a formulation having a specific dosage form.

The formulation according to the present invention has been found to have excellent stability of the active ingredient in the formulation and dissolvability from the formulation.

That is, the present invention relates to the following.

[1] A composition containing a compound represented by the following Formula (1):

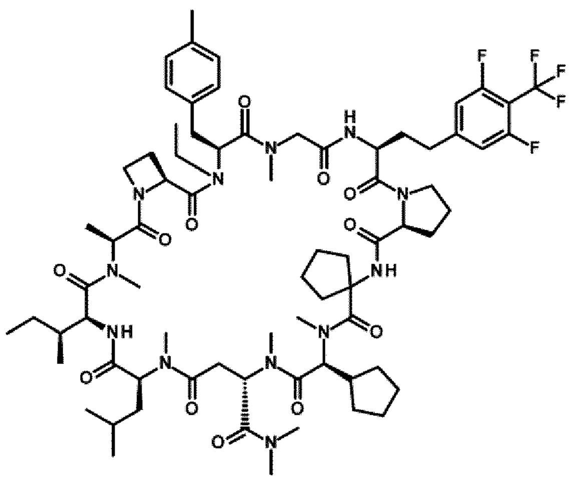

(1)

or a salt thereof, or a solvate thereof; a liquid additive; and optionally an oily component.

[2] The composition according to [1], wherein the liquid additive is a surfactant.

[3] The composition according to [2], wherein the surfactant is a combination of a hydrophobic surfactant and a hydrophilic surfactant.

[4] The composition accordingo [3], wherein an HLB value of the hydrophobic surfactant is 0 or more and less than 10, and an HLB value of the hydrophilic surfactant is 10 or more and 30 or less.

[5] The composition according to [3] or [4], wherein the hydrophobic surfactant is at least one selected from the group consisting of a propylene glycol fatty acid ester, a glyceryl fatty acid ester, a polyglyceryl fatty acid ester, a sorbitan fatty acid ester, and a hydrophobic polyoxyethylene hydrogenated castor oil.

[6] The composition according to any one of [3] to [5], wherein the hydrophobic surfactant is at least one selected from the group consisting of a propylene glycol fatty acid ester and a sorbitan fatty acid ester.

[7] The composition according to any one of [3] to [6], wherein the hydrophobic surfactant comprises a propylene glycol fatty acid ester.

[8] The composition according to [5], wherein the propylene glycol fatty acid ester is at least one selected from the group consisting of propylene glycol monocaproate, propylene glycol monocaprylate, propylene glycol monocaprate, propylene glycol monolaurate, propylene glycol monomyristate, propylene glycol monopalmitate, propylene glycol monostearate, and propylene glycol monooleate.

[9] The composition according to [5], wherein the glyceryl fatty acid ester is at least one selected from the group consisting of glyceryl monocaproate, glyceryl monocaprylate, glyceryl monocaprate, glyceryl monolaurate, glyceryl monomyristate, glyceryl monopalmitate, glyceryl monostearate, glyceryl monooleate, and glyceryl monolinoleate.

[10] The composition according to [5], wherein the polyglyceryl fatty acid ester is diglyceryl monooleate.

[11] The composition according to [5], wherein the sorbitan fatty acid ester is at least one selected from the group consisting of sorbitan monocaprylate, sorbitan monocaprate, sorbitan monolaurate, sorbitan monomyristate, sorbitan monopalmitate, sorbitan monostearate, sorbitan monooleate, sorbitan sesquioleate, and sorbitan trioleate.

[12] The composition according to [5], wherein the hydrophobic polyoxyethylene hydrogenated castor oil is at least one selected from the group consisting of polyoxyethylene hydrogenated castor oil 5 and polyoxyethylene hydrogenated castor oil 10.

[13] The composition according to any one of [3] to [7], wherein the hydrophobic surfactant is propylene glycol monocaprylate.

[14] The composition according to any one of [3] to [13], wherein the hydrophilic surfactant is at least one selected from the group consisting of a polyethylene glycol fatty acid ester, a polyoxyethylene castor oil, a hydrophilic polyoxyethylene hydrogenated castor oil, a polyoxyethylene sorbitan fatty acid ester, D-α-tocopheryl polyethylene glycol 1000 succinate, a caprylocaproyl polyoxyl-8 glyceride, and a combination thereof.

[15] The composition according to any one of [3] to [14], wherein the hydrophilic surfactant is at least one selected from the group consisting of a polyoxyethylene castor oil, a polyoxyethylene sorbitan fatty acid ester, D-α-tocopheryl polyethylene glycol 1000 succinate, caprylocaproyl polyoxyl-8 glyceride, and a combination thereof.

[16] The composition according to any one of [3] to [15], wherein the hydrophilic surfactant comprises a polyoxyethylene castor oil.

[17] The composition according to [14], wherein the polyethylene glycol fatty acid ester is at least one selected from the group consisting of polyoxyethylene hydroxystearate, polyethylene glycol monolaurate, polyethylene glycol monostearate, and polyoxyl 40 stearate.

[18] The composition according to [14], wherein the polyoxyethylene castor oil is at least one selected from the group consisting of polyoxyl 30 castor oil, polyoxyl 35 castor oil, and polyoxyl 40 castor oil.

[19] The composition according to [14], wherein the polyoxyethylene castor oil is polyoxyl 35 castor oil.

[20] The composition according to [14], wherein the hydrophilic polyoxyethylene castor oil is at least one selected from the group consisting of polyoxyethylene hydrogenated castor oil 20, polyoxyethylene hydrogenated castor oil 40, polyoxyethylene hydrogenated castor oil 50, and polyoxyethylene hydrogenated castor oil 60.

[21] The composition according to [14], wherein the polyoxyethylene sorbitan fatty acid ester is at least one selected from the group consisting of polysorbate 20, polysorbate 40, polysorbate 60, and polysorbate 80.

[22] The composition according to any one of [3] to [15], wherein the hydrophilic surfactant is at least one selected from the group consisting of polyoxyl 35 castor oil, D-α-tocopheryl polyethylene glycol 1000 succinate, caprylocaproyl polyoxyl-8 glyceride, polysorbate 80, and a combination thereof.

[23] The composition according to any one of [3] to [15], wherein the hydrophilic surfactant comprises polyoxyl 35 castor oil.

[24] The composition according to [3], wherein the hydrophobic surfactant is a propylene glycol fatty acid ester, and the hydrophilic surfactant comprises a polyoxyethylene castor oil.

[25] The composition according to [3], wherein the hydrophobic surfactant is propylene glycol monocaprylate, and the hydrophilic surfactant is at least one selected from the group consisting of polyoxyl 35 castor oil, D-α-tocopheryl polyethylene glycol 1000 succinate, caprylocaproyl polyoxyl-8 glyceride, polysorbate 80, and a combination thereof.

[26] The composition according to [3], wherein the hydrophobic surfactant is propylene glycol monocaprylate, and the hydrophilic surfactant comprises polyoxyl 35 castor oil.

[27] The composition according to any one of [1] to [26], wherein the composition contains an oily component.

[28] The composition according to any one of [1] to [27], wherein the oily component is at least one selected from the group consisting of a fatty acid, an acyl glycerol, a plant oil, and a combination thereof.
[29] The composition according to any one of [1] to [28], wherein the oily component is at least one selected from the group consisting of a fatty acid, an acyl glycerol, and a combination thereof.
[30] The composition according to any one of [1] to [29], wherein the oily component comprises a fatty acid.
[31] The composition according to [28], wherein the fatty acid is at least one selected from the group consisting of caproic acid, caprylic acid, capric acid, lauric acid, myristic acid, myristoleic acid, palmitic acid, palmitoleic acid, stearic acid, oleic acid, linoleic acid, and linolenic acid.
[32] The composition according to [28], wherein the fatty acid is at least one selected from the group consisting of oleic acid, linoleic acid, and linolenic acid.
[33] The composition according to [28], wherein the acyl glycerol is at least one selected from the group consisting of triacetin, tributylin, tricaproin, tricapriline, tricaprine, tripalmitine, tripalmitolein, glyceryl tristearate, triolein, trilinolein, trilinolenine, and a triglyceride of medium chain fatty acid.
[34] The composition according to [28], wherein the acyl glycerol is triacetin.
[35] The composition according to [28], wherein the plant oil is at least one selected from the group consisting of olive oil, almond oil, palm oil, cocoa butter, macadamia nut oil, avocado oil, safflower oil, soybean oil, linseed oil, rapeseed oil, castor oil, corn oil, and palm oil.
[36] The composition according to any one of [1] to [35], wherein the oily component is oleic acid or triacetin.
[37] The composition according to any one of [1] to [36], wherein the oily component is oleic acid.
[38] The composition according to [3], wherein the composition contains a propylene glycol fatty acid ester as a hydrophobic surfactant, a polyoxyethylene castor oil as a hydrophilic surfactant, and a fatty acid as an oily component.
[39] The composition according to [3], wherein the composition contains propylene glycol monocaprylate as a hydrophobic surfactant, polyoxyl 35 castor oil as a hydrophilic surfactant, and oleic acid as an oily component.
[40] The composition according to any one of [1] to [39], further containing an antioxidant.
[41] The composition according to [40], wherein the antioxidant is at least one selected from the group consisting of dl-$\alpha$-tocopherol, butylated hydroxytoluene, butylated hydroxyanisole, propyl gallate, gallic acid propyl ester, a pharmaceutically acceptable quinone, astaxanthin, and D-$\alpha$-tocopheryl polyethylene glycol 1000 succinate.
[42] The composition according to any one of [1] to [41], further containing a solubilizer.
[43] The composition according to [42], wherein the solubilizer is at least one selected from the group consisting of ethanol, propylene glycol, polyethylene glycol 300, polyethylene glycol 400, diethylene glycol monoethyl ether, and a combination thereof.
[44] The composition according to any one of [1] to [43], wherein the compound represented by Formula (1) or a salt thereof, or a solvate thereof, is a compound represented by Formula (1) or a hydrate thereof.
[45] The composition according to [44], wherein the hydrate is a hydrate of the compound represented by Formula (1).
[46] The composition according to [44], wherein the compound represented by Formula (1) or a salt thereof, or a solvate thereof is a compound represented by Formula (1).
[47] The composition according to any one of [1] to [46], wherein a content of the compound represented by Formula (1) or a salt thereof, or a solvate thereof, based on the total composition is 10% by weight or less.
[48] The composition according to any one of [1] to [47], wherein a content of the compound represented by Formula (1) or a salt thereof, or a solvate thereof, based on the total composition is 8% by weight or less.
[49] The composition according to any one of [1] to [48], wherein a content of the compound represented by Formula (1) or a salt thereof, or a solvate thereof, based on the total composition is 7% by weight or less.
[50] The composition according to any one of [1] to [49], wherein a content of the liquid additive based on the total composition is 50% by weight or more and 97% by weight or less.
[51] The composition according to any one of [1] to [50], wherein a content of the liquid additive based on the total composition is 55% by weight or more and 96% by weight or less.
[52] The composition according to any one of [1] to [51], wherein a content of the liquid additive based on the total composition is 70% by weight or more and 90% by weight or less.
[53] The composition according to any one of [3] to [52], wherein a content of the hydrophobic surfactant based on the total composition is 20% by weight or more and 70% by weight or less.
[54] The composition according to any one of [3] to [53], wherein a content of the hydrophobic surfactant based on the total composition is 25% by weight or more and 65% by weight or less.
[55] The composition according to any one of [3] to [54], wherein a content of the hydrophobic surfactant based on the total composition is 30% by weight or more and 55% by weight or less.
[56] The composition according to any one of [3] to [55], wherein a content of the hydrophilic surfactant based on the total composition is 20% by weight or more and 40% by weight or less.
[57] The composition according to any one of [3] to [56], wherein a content of the hydrophilic surfactant based on the total composition is 25% by weight or more and 35% by weight or less.

[58] The composition according to any one of [3] to [57], wherein a content of the hydrophilic surfactant based on the total composition is 28% by weight or more and 33% by weight or less.
[59] The composition according to any one of [1] to [58], wherein a content of the oily component based on the total composition is 0% by weight or more and 50% by weight or less.
[60] The composition according to any one of [1] to [59], wherein a content of the oily component based on the total composition is 1% by weight or more and 40% by weight or less.
[61] The composition according to any one of [1] to [60], wherein a content of the oily component to an entire of the composition is 10% by weight or more and 20% by weight or less.
[62] The composition according to any one of [1] to [61], wherein the oily component comprises a fatty acid.
[63] The composition according to any one of [3] to [62], wherein a weight ratio of the hydrophobic surfactant to the hydrophilic surfactant is 0.5 to 3.0.
[64] The composition according to any one of [3] to [63], wherein a weight ratio of the hydrophobic surfactant to the hydrophilic surfactant is 1.0 to 2.5.
[65] The composition according to any one of [3] to [64], wherein a weight ratio of the hydrophobic surfactant to the hydrophilic surfactant is 1.5 to 2.0.
[66] The composition according to any one of [1] to [65], wherein a weight ratio of the liquid additive to the compound represented by Formula (1) or a salt thereof, or a solvate thereof, is 5 or more.
[67] The composition according to any one of [1] to [66], wherein a weight ratio of the liquid additive to the compound represented by Formula (1) or a salt thereof, or a solvate thereof, is 10 or more.
[68] The composition according to any one of [1] to [66], wherein a weight ratio of the liquid additive to the compound represented by Formula (1) or a salt thereof, or a solvate thereof, is 5 to 2000.
[69] The composition according to any one of [1] to [68], wherein a weight ratio of a total of the liquid additive and the oily component to the compound represented by Formula (1) or a salt thereof, or a solvate thereof, is 10 or more.
[70] The composition according to any one of [1] to [69], wherein a weight ratio of a total of the liquid additive and the oily component to the compound represented by Formula (1) or a salt thereof, or a solvate thereof, is 10 to 2000.
[71] The composition according to any one of [1] to [70], wherein a weight ratio of a total of the liquid additive and the oily component to the compound represented by Formula (1) or a salt thereof, or a solvate thereof, is 10 to 1000.
[72] The composition according to any one of [1] to [71], wherein the composition is a liquid.
[73] The composition according to any one of [1] to [72], wherein an average particle size of droplets formed when the composition is dispersed in water is less than 200 nm.
[74] The composition according to [73], wherein the average particle size of droplets is 10 nm or more and less than 200 nm.
[75] The composition according to [74], wherein the average particle size of droplets is 50 nm or more and 200 nm or less.
[76] A pharmaceutical formulation containing the composition according to any one of [1] to [75].
[77] The pharmaceutical formulation according to [76], wherein a dosage form of the formulation is a capsule formulation.
[78] A method for producing the composition according to any one of [1] to [75], comprising the steps of:

(1) providing a compound represented by the following Formula (1):

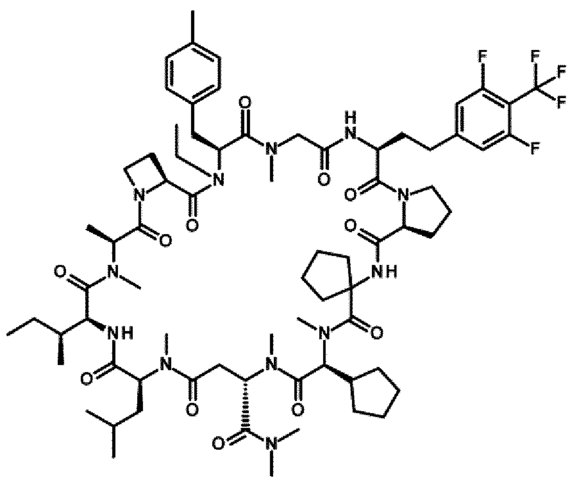

(1)

or a salt thereof or a solvate thereof, a liquid additive, and optionally an oily component;
(2) mixing the compound represented by Formula (1) or a salt thereof or a solvate thereof, the liquid additive, and optionally the oily component; and
(3) obtaining a composition in which the compound represented by Formula (1) or a salt thereof or, a solvate thereof, is dissolved.

[79] The method according to [78], wherein the compound represented by Formula (1) or a salt thereof, or a solvate thereof, provided in step (1) is a hydrate of the compound represented by Formula (1).
[80] The method according to [79], wherein the hydrate of the compound represented by Formula (1) is a crystal, and the crystal has, in powder X-ray diffraction, a powder X-ray diffraction pattern comprising at least one peak at diffraction angles 2Θ of 4.964°, 7.921°, 8.296°, 8.855°, 9.956°, 10.435°, 11.729°, 12.704°, 13.552°, 13.901°, 15.895°, 16.643°, and 17.813° (± 0.2°).

[80-1] The method according to [79], wherein the hydrate of the compound represented by Formula (1) is a crystal, and the crystal has, in powder X-ray diffraction, a powder X-ray diffraction pattern comprising at least two peaks at diffraction angles 2Θ of 4.964°, 7.921°, 8.296°, 8.855°, 9.956°, 10.435°, 11.729°, 12.704°, 13.552°, 13.901°, 15.895°, 16.643°, and 17.813° (± 0.2°).
[80-2] The method according to [79], wherein the hydrate of the compound represented by Formula (1) is a crystal, and the crystal has, in powder X-ray diffraction, a powder X-ray diffraction pattern comprising at least three peaks at diffraction angles 2Θ of 4.964°, 7.921°, 8.296°, 8.855°, 9.956°, 10.435°, 11.729°, 12.704°, 13.552°, 13.901°, 15.895°, 16.643°, and 17.813° (± 0.2°).
[80-3] The method according to [79], wherein the hydrate of the compound represented by Formula (1) is a crystal, and the crystal has, in powder X-ray diffraction, a powder X-ray diffraction pattern comprising at least four peaks at diffraction angles 2Θ of 4.964°, 7.921°, 8.296°, 8.855°, 9.956°, 10.435°, 11.729°, 12.704°, 13.552°, 13.901°, 15.895°, 16.643°, and 17.813° (± 0.2°).
[80-4] The method according to [79], wherein the hydrate of the compound represented by Formula (1) is a crystal, and the crystal has, in powder X-ray diffraction, a powder X-ray diffraction pattern comprising at least five peaks at diffraction angles 2Θ of 4.964°, 7.921°, 8.296°, 8.855°, 9.956°, 10.435°, 11.729°, 12.704°, 13.552°, 13.901°, 15.895°, 16.643°, and 17.813° (± 0.2°).
[80-5] The method according to [79], wherein the hydrate of the compound represented by Formula (1) is a crystal, and the crystal has, in powder X-ray diffraction, a powder X-ray diffraction pattern comprising at least six peaks at diffraction angles 2Θ of 4.964°, 7.921°, 8.296°, 8.855°, 9.956°, 10.435°, 11.729°, 12.704°, 13.552°, 13.901°, 15.895°, 16.643°, and 17.813° (± 0.2°).
[80-6] The method according to [79], wherein the hydrate of the compound represented by Formula (1) is a crystal, and the crystal has, in powder X-ray diffraction, a powder X-ray diffraction pattern comprising at least seven peaks at diffraction angles 2Θ of 4.964°, 7.921°, 8.296°, 8.855°, 9.956°, 10.435°, 11.729°, 12.704°, 13.552°, 13.901°, 15.895°, 16.643°, and 17.813° (± 0.2°).
[80-7] The method according to [79], wherein the hydrate of the compound represented by Formula (1) is a crystal, and the crystal has, in powder X-ray diffraction, a powder X-ray diffraction pattern comprising at least eight peaks at diffraction angles 2Θ of 4.964°, 7.921°, 8.296°, 8.855°, 9.956°, 10.435°, 11.729°, 12.704°, 13.552°, 13.901°, 15.895°, 16.643°, and 17.813° (± 0.2°).
[80-8] The method according to [79], wherein the hydrate of the compound represented by Formula (1) is a crystal, and the crystal has, in powder X-ray diffraction, a powder X-ray diffraction pattern comprising at least nine peaks at diffraction angles 2Θ of 4.964°, 7.921°, 8.296°, 8.855°, 9.956°, 10.435°, 11.729°, 12.704°, 13.552°, 13.901°, 15.895°, 16.643°, and 17.813° (± 0.2°).
[80-9] The method according to [79], wherein the hydrate of the compound represented by Formula (1) is a crystal, and the crystal has, in powder X-ray diffraction, a powder X-ray diffraction pattern comprising at least ten peaks at diffraction angles 2Θ of 4.964°, 7.921°, 8.296°, 8.855°, 9.956°, 10.435°, 11.729°, 12.704°, 13.552°, 13.901°, 15.895°, 16.643°, and 17.813° (± 0.2°).
[80-10] The method according to [79], wherein the hydrate of the compound represented by Formula (1) is a crystal, and the crystal has, in powder X-ray diffraction, a powder X-ray diffraction pattern comprising at least 11 peaks at diffraction angles 2Θ of 4.964°, 7.921°, 8.296°, 8.855°, 9.956°, 10.435°, 11.729°, 12.704°, 13.552°, 13.901°, 15.895°, 16.643°, and 17.813° (± 0.2°).
[80-11] The method according to [79], wherein the hydrate of the compound represented by Formula (1) is a crystal, and the crystal has, in powder X-ray diffraction, a powder X-ray diffraction pattern comprising at least 12 peaks at diffraction angles 2Θ of 4.964°, 7.921°, 8.296°, 8.855°, 9.956°, 10.435°, 11.729°, 12.704°, 13.552°, 13.901°, 15.895°, 16.643°, and 17.813° (± 0.2°).
[80-12] The method according to [79], wherein the hydrate of the compound represented by Formula (1) is a

crystal, and the crystal has, in powder X-ray diffraction, a powder X-ray diffraction pattern comprising peaks at diffraction angles 2Θ of 4.964°, 7.921°, 8.296°, 8.855°, 9.956°, 10.435°, 11.729°, 12.704°, 13.552°, 13.901°, 15.895°, 16.643°, and 17.813° (± 0.2°).

[81] The method according to [79], wherein the hydrate of the compound represented by Formula (1) is a crystal, and the crystal has, in powder X-ray diffraction, a powder X-ray diffraction pattern comprising at least one peak at diffraction angles 2Θ of 7.921°, 9.956°, 10.435° 11.729°, 12.704°, 15.895°, and 16.643° (± 0.2°).

[81-1] The method according to [79], wherein the hydrate of the compound represented by Formula (1) is a crystal, and the crystal has, in powder X-ray diffraction, a powder X-ray diffraction pattern comprising at least two peaks at diffraction angles 2Θ of 7.921°, 9.956°, 10.435° 11.729°, 12.704°, 15.895°, and 16.643° (± 0.2°).
[81-2] The method according to [79], wherein the hydrate of the compound represented by Formula (1) is a crystal, and the crystal has, in powder X-ray diffraction, a powder X-ray diffraction pattern comprising at least three peaks at diffraction angles 2Θ of 7.921°, 9.956°, 10.435° 11.729°, 12.704°, 15.895°, and 16.643° (± 0.2°).
[81-3] The method according to [79], wherein the hydrate of the compound represented by Formula (1) is a crystal, and the crystal has, in powder X-ray diffraction, a powder X-ray diffraction pattern comprising at least four peaks at diffraction angles 2Θ of 7.921°, 9.956°, 10.435° 11.729°, 12.704°, 15.895°, and 16.643° (± 0.2°).
[81-4] The method according to [79], wherein the hydrate of the compound represented by Formula (1) is a crystal, and the crystal has, in powder X-ray diffraction, a powder X-ray diffraction pattern comprising at least five peaks at diffraction angles 2Θ of 7.921°, 9.956°, 10.435° 11.729°, 12.704°, 15.895°, and 16.643° (± 0.2°).
[81-5] The method according to [79], wherein the hydrate of the compound represented by Formula (1) is a crystal, and the crystal has, in powder X-ray diffraction, a powder X-ray diffraction pattern comprising at least six peaks at diffraction angles 2Θ of 7.921°, 9.956°, 10.435° 11.729°, 12.704°, 15.895°, and 16.643° (± 0.2°).
[81-6] The method according to [79], wherein the hydrate of the compound represented by Formula (1) is a crystal, and the crystal has, in powder X-ray diffraction, a powder X-ray diffraction pattern comprising peaks at diffraction angles 2Θ of 7.921°, 9.956°, 10.435° 11.729°, 12.704°, 15.895°, and 16.643° (± 0.2°).
[81-7] The method according to [79], wherein the hydrate of the compound represented by Formula (1) is a crystal, and the crystal has, in powder X-ray diffraction, a powder X-ray diffraction pattern comprising at least seven peaks at diffraction angles 2Θ of 4.964°, 7.921°, 8.296°, 8.855°, 9.956°, 10.435°, 11.729°, 12.704°, 13.552°, 13.901°, 14.752°, 14.968°, 15.895°, 16.190°, 16.643°, 17.813°, and 19.424° (± 0.2°).
[81-8] The method according to [79], wherein the hydrate of the compound represented by Formula (1) is a crystal, and the crystal has, in powder X-ray diffraction, a powder X-ray diffraction pattern comprising at least ten peaks at diffraction angles 2Θ of 4.964°, 7.921°, 8.296°, 8.855°, 9.956°, 10.435°, 11.729°, 12.704°, 13.552°, 13.901°, 14.752°, 14.968°, 15.895°, 16.190°, 16.643°, 17.813°, and 19.424° (± 0.2°).
[81-9] The method according to [79], wherein the hydrate of the compound represented by Formula (1) is a crystal, and the crystal has, in powder X-ray diffraction, a powder X-ray diffraction pattern comprising at least 13 peaks at diffraction angles 2Θ of 4.964°, 7.921°, 8.296°, 8.855°, 9.956°, 10.435°, 11.729°, 12.704°, 13.552°, 13.901°, 14.752°, 14.968°, 15.895°, 16.190°, 16.643°, 17.813°, and 19.424° (± 0.2°).
[81-10] The method according to [79], wherein the hydrate of the compound represented by Formula (1) is a crystal, and the crystal has, in powder X-ray diffraction, a powder X-ray diffraction pattern comprising at least 15 peaks at diffraction angles 2Θ of 4.964°, 7.921°, 8.296°, 8.855°, 9.956°, 10.435°, 11.729°, 12.704°, 13.552°, 13.901°, 14.752°, 14.968°, 15.895°, 16.190°, 16.643°, 17.813°, and 19.424° (± 0.2°).
[81-10] The method according to [79], wherein the hydrate of the compound represented by Formula (1) is a crystal, and the crystal has, in powder X-ray diffraction, a powder X-ray diffraction pattern comprising peaks at diffraction angles 2Θ of 4.964°, 7.921°, 8.296°, 8.855°, 9.956°, 10.435°, 11.729°, 12.704°, 13.552°, 13.901°, 14.752°, 14.968°, 15.895°, 16.190°, 16.643°, 17.813°, and 19.424° (± 0.2°).

[82] A method for producing a pharmaceutical formulation, comprising a step of providing the composition according to any one of [1] to [75], and a step of formulating the composition to provide a pharmaceutical formulation.
[83] A method for producing a capsule formulation, comprising a step of providing the composition according to any one of [1] to [75], and a step of filling the composition into a capsule to provide a capsule formulation.

[Advantageous Effects of Invention]

The composition according to the present invention is excellent in various properties required as a formulation. For example, the composition according to the present invention has desirable particle properties when a liquid composition is emulsified to form droplets, and has excellent stability and dispersity, as well as excellent absorption into the body.

[Brief Description of Drawings]

[Figure 1] Figure 1 is a graph showing the results of powder X-ray diffraction measurement of the hydrate crystal (Form C) of Compound 1 obtained in Preparation Example 3. The ordinate represents diffraction intensity, and the abscissa represents diffraction angle 2θ (°).

[Figure 2] Figure 2 shows the results of thermal gravimetric and differential thermal analysis of the hydrate crystal (Form C) of Compound 1. The abscissa represents temperature (°C) and measurement time (min), and the right ordinate represents the weight change (mg) of the sample in thermal gravimetric analysis. The left ordinate represents the heat flow (mW) observed in differential thermal analysis.

[Figure 3] Figure 3 shows a crystal structure by single crystal X-ray structural analysis of the hydrate crystal (Form C) of Compound 1.

[Figure 4] Figure 4 shows the results of dynamic moisture vapor adsorption measurement of the hydrate crystal (Form C) of Compound 1. The ordinate represents weight change (%) and the abscissa represents relative humidity (%). In Figure 4, "Cycle 1 Sorp" (black diamond mark) indicates adsorption in cycle 1, "Cycle 1 Desorp" (black square mark) indicates desorption in cycle 1, "Cycle 2 Sorp" (black triangle mark) indicates adsorption in cycle 2, and "Cycle 2 Desorp" (black square mark) indicates desorption in cycle 2.

[Figure 5] Figure 5 represents a graph showing the temporal changes in the dispersity of the formulations according to the present invention (formulation C, formulation F) and Comparative Example (formulation AJ) into the fasted state simulated intestinal fluid (FaSSIF).

[Description of Embodiments]

The composition of the present invention contains a compound represented by the following Formula (1):

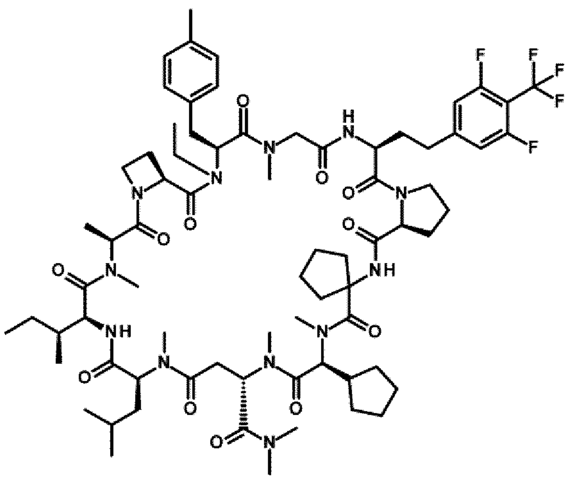

(1)

("(5S,8S,11S,15S,18S,23aS,29S,35S,37aS)-8-((S)-sec-butyl)-18-cyclopentyl-29-(3,5-difluoro-4-(trifluoromethyl)phenethyl)-36-ethyl-11-isobutyl-N,N,5,6,12,16,19,33-octamethyl-35-(4-methylbenzyl)-4,7,10,13,17,20,23,28,31,34,37-undecaoxotetratriacontahydro-2H,4H-spiro[azeto[2, 1-u]pyrrolo[2,1-i][1,4,7,10,13,16,19,22,25,28,31]undecaazacyclotetratriacontin-21,1'-cyclopentane]-15-carboxamide", hereinafter, sometimes referred to as "Compound 1") or a salt thereof, or a solvate thereof, and a liquid additive.

Furthermore, the composition of the present invention optionally contains an oily component, and preferably contains an oily component.

<u>Compound (active ingredient)</u>

The compounds that can be used in the present invention are the compound represented by Formula (1) or a salt thereof, or a solvate thereof. The salt of Compound 1 can be preferably a chemically or pharmaceutically acceptable salt. Compound 1 or a salt thereof that can be used in the present invention can be a solvate thereof, preferably a chemically or pharmaceutically acceptable solvate thereof. The compound used in the present invention may preferably be in the free form wholly in the composition, but depending on the aspect of the composition, the composition may

contain the compound in a salt or solvate form.

Examples of the salt of Compound 1 in the present invention include: hydrochloride; hydrobromide; hydroiodide; phosphate; phosphonate; sulfate; sulfonate such as methanesulfonate and p-toluenesulfonate; carboxylate such as acetate, citrate, malate, tartrate, succinate, and salicylate; alkali metal salts such as sodium salt, and potassium salt; alkaline earth metal salts such as magnesium salt and calcium salt; and ammonium salts such as ammonium salt, alkylammonium salt, dialkylammonium salt, trialkylammonium salt, and tetraalkylammonium salt. These salts are produced by, for example, bringing Compound 1 into contact with an acid or a base that can be used in the manufacture of pharmaceuticals.

In the present invention, a solvate refers to one in which a compound and a solvent together forms a single molecular population, and is not particularly limited as long as the solvate is formed with a solvent that is acceptable for ingestion along with the administration of a pharmaceutical. Examples of the solvate include hydrates, alcohol solvates (such as ethanol solvates, methanol solvates, 1-propanol solvates, 2-propanol solvates), and not only solvates formed with a single solvent such as dimethyl sulfoxide, but also solvates formed with a plurality of solvents per one molecule of compound, or solvates formed with a plurality of types of solvents per one molecule of compound. When the solvent is water, the solvates are called hydrates. The solvate of the compound of the present invention is preferably a hydrate. Specific examples of such hydrate include a mono- to deca-hydrate, preferably a mono- to pentahydrate, further preferably a mono- to tri-hydrate. The hydrate of Compound 1 used in the present invention may change the number of water molecules by desorption of the water molecule that attaches to Compound 1, depending on the ambient environment such as temperature or humidity.

Compound 1 or a salt thereof, or a solvate thereof that may be used in the present invention may be provided in the form of crystal, amorphous, or a mixture thereof, and preferably Compound 1 or a salt thereof, or a solvate thereof may be provided in the form of crystal. The crystal of Compound 1 or a salt thereof, or a solvate thereof which may be used in the present invention, preferably includes a hydrate crystal (also referred to as Form C) of Compound 1.

Crystals of Compound 1 or a salt thereof, or a solvate thereof can be characterized by techniques known in the art, such as powder X-ray diffraction (XRPD), measurement of moisture content (e.g., Karl Fischer method), scanning electron microscopy (SEM) analysis, solid-state NMR, or thermal techniques such as differential scanning calorimetry (DSC), or any other standard quantitative measurement method.

The diffraction angle 2θ in powder X-ray diffraction is preferably a diffraction peak measured with CuKα radiation.

For example, the hydrate crystal of Compound 1 that may be used in the present invention has, in the powder X-ray diffraction, a powder X-ray diffraction pattern comprising at least one of the following peaks at diffraction angles 2θ: 4.964°, 7.921°, 8.296°, 8.855°, 9.956°, 10.435°, 11.729°, 12.704°, 13.552°, 13.901°, 15.895°, 16.643°, 17.813° (± 0.2°).

The term "(± 0.2°)" described at the end of listed diffraction angles 2θ in the diffraction angles 2θ description means that a range of ± 0.2° for each described value is acceptable for all of the listed diffraction angles 2θ.

Compound 1 used in the present invention includes all isotopes of Compound 1. The isotope of Compound 1 is one in which at least one atom is substituted with an atom having the same atomic number (the number of protons) and a different mass number (the sum number of protons and neutrons) in an abundance ratio different from the natural abundance ratio. Examples of the isotope contained in Compound 1 include a hydrogen atom, a carbon atom, a nitrogen atom, an oxygen atom, and a fluorine atom, and they include $^{2}H$, $^{3}H$; $^{13}C$, $^{14}C$; $^{15}N$; $^{17}O$, $^{18}O$; $^{18}F$; and the like, respectively. In particular, radioisotopes, such as $^{3}H$ or $^{14}C$, that release radiation as it break down, are useful in the test of tissue distribution in vivo of pharmaceuticals or compounds. The stable isotope can be used safely because it does not break down, changes little in abundance over time, and has no radioactivity. The isotopes of Compound 1 can be converted according to conventional methods by replacing the reagent used in the synthesis with a reagent containing the corresponding isotope. The acid or base used to form a salt of Compound 1, and the solvent used to form a solvate of Compound 1 can also include all isotopes.

## Liquid additive

The "liquid additive" that can be used in the composition according to the present invention is a pharmaceutically acceptable additive that can dissolve the compound used in the present invention. In the present invention, the term "liquid additive" means the additive that is in a dissolved state in the composition. A liquid additive that is not dissolved at the stage provided as a raw material in the production of the composition may be dissolved by mixing with other ingredients, heating, or the like in the production process to be used as an ingredient of the composition according to the present invention. As the liquid additive, an additive that is liquid at room temperature can be preferably used.

As used herein, the term "room temperature" is used in the ordinary sense in the art, and is not particularly limited, but unless otherwise stated, the room temperature is, for example, preferably about 1 to 30°C, more preferably about 15 to 28°C.

Examples of the liquid additive that can be used in the present invention preferably include surfactants, and examples of the surfactants preferably include hydrophobic surfactants and hydrophilic surfactants. In the present invention, it is desirable to use a combination of a hydrophobic surfactant and a hydrophilic surfactant.

In the hydrophobic surfactant and hydrophilic surfactant used in the present invention, the hydrophilic-lipophilic balance value (HLB value) of the hydrophobic surfactant is preferably less than 10, more preferably 0 or more and less than 10, and the HLB value of the hydrophilic surfactant is preferably 10 or more, more preferably 10 or more and 30 or less, further preferably 10 or more and 20 or less. The HLB value is a value indicating the degree of affinity of the surfactant for water and oil (an organic compound that is insoluble in water), and is known by those skilled in the art. For example, a value based on known methods such as the Griffin method, the Atlas method, the Davis method, or the like can be employed as the HLB value.

Examples of the hydrophobic surfactant preferably include a propylene glycol fatty acid ester, a glyceryl fatty acid ester, a polyglyceryl fatty acid ester, a sorbitan fatty acid ester, and a hydrophobic polyoxyethylene hydrogenated castor oil, and a combination thereof can also be used. As the hydrophobic surfactant, a propylene glycol fatty acid ester or a sorbitan fatty acid ester can be more preferably used, and propylene glycol fatty acid esters can be further preferably used.

Examples of the propylene glycol fatty acid ester preferably include propylene glycol monocaproate, propylene glycol monocaprylate, propylene glycol monocaprate, propylene glycol monolaurate, propylene glycol monomyristate, propylene glycol monopalmitate, propylene glycol monostearate, and propylene glycol monooleate. Of these, propylene glycol monocaprylate can be more preferably used.

Examples of the glyceryl fatty acid ester preferably include glyceryl monocaproate, glyceryl monocaprylate, glyceryl monocaprate, glyceryl monolaurate, glyceryl monomyristate, glyceryl monopalmitate, glyceryl monostearate, glyceryl monooleate, and glyceryl monolinoleate.

Examples of the polyglyceryl fatty acid ester preferably include diglyceryl monooleate.

Examples of the sorbitan fatty acid ester preferably include sorbitan monocaprylate, sorbitan monocaprate, sorbitan monolaurate, sorbitan monomyristate, sorbitan monopalmitate, sorbitan monostearate, sorbitan monooleate, sorbitan sesquioleate, and sorbitan trioleate.

As used herein, the hydrophobic polyoxyethylene hydrogenated castor oil is a polyoxyethylene hydrogenated castor oil having hydrophobicity, and examples thereof include polyoxyethylene hydrogenated castor oil having an HLB value of preferably less than 10, more preferably 0 or more and less than 10. Examples of the hydrophobic polyoxyethylene hydrogenated castor oil preferably include polyoxyethylene hydrogenated castor oil 5 and polyoxyethylene hydrogenated castor oil 10.

Examples of the hydrophilic surfactant preferably include a polyethylene glycol fatty acid ester, a polyoxyethylene castor oil, a hydrophilic polyoxyethylene hydrogenated castor oil, a polyoxyethylene sorbitan fatty acid ester, D-$\alpha$-tocopheryl polyethylene glycol 1000 succinate, and a caprylocaproyl polyoxyl-8 glyceride, and a combination thereof can also be used. As the hydrophilic surfactant, a polyoxyethylene castor oil, a polyoxyethylene sorbitan fatty acid ester, D-$\alpha$-tocopheryl polyethylene glycol 1000 succinate, a caprylocaproyl polyoxyl-8 glyceride, and a combination thereof can be more preferably used, and a polyoxyethylene castor oil can be further preferably used.

Examples of the polyethylene glycol fatty acid ester preferably include polyoxyethylene hydroxystearate, polyethylene glycol monolaurate, polyethylene glycol monostearate, and polyoxyl 40 stearate.

Examples of the polyoxyethylene castor oil preferably include polyoxyl 30 castor oil, polyoxyl 35 castor oil, and polyoxyl 40 castor oil, and more preferably polyoxyl 35 castor oil.

As used herein, the hydrophilic polyoxyethylene hydrogenated castor oil is a polyoxyethylene hydrogenated castor oil having hydrophilicity, and examples thereof include polyoxyethylene hydrogenated castor oil having an HLB value of preferably 10 or more, more preferably 10 or more and 30 or less, and further preferably 10 or more and 20 or less. Examples of the hydrophilic polyoxyethylene castor oil preferably include polyoxyethylene hydrogenated castor oil 20, polyoxyethylene hydrogenated castor oil 40, polyoxyethylene hydrogenated castor oil 50, and polyoxyethylene hydrogenated castor oil 60.

Examples of the polyoxyethylene sorbitan fatty acid ester preferably include polysorbate 20, polysorbate 40, polysorbate 60, and polysorbate 80.

As the hydrophilic surfactant, further preferably, polyoxyl 35 castor oil, D-$\alpha$-tocopheryl polyethylene glycol 1000 succinate, caprylocaproyl polyoxyl-8 glyceride, polysorbate 80, or a combination thereof can be desirably used.

For the surfactant, propylene glycol monocaprylate can be preferably used as the hydrophobic surfactant, and polyoxyl 35 castor oil, D-$\alpha$-tocopheryl polyethylene glycol 1000 succinate, caprylocaproyl polyoxyl-8 glyceride, polysorbate 80, or a combination thereof can be preferably used as the hydrophilic surfactant.

For the surfactant, preferably, it is desirable that propylene glycol fatty acid ester is used as the hydrophobic surfactant, and it is desirable that at least polyoxyethylene castor oil is contained as the hydrophilic surfactant, and particularly preferably, it is desirable that propylene glycol monocaprylate is used as the hydrophobic surfactant and at least polyoxyl 35 castor oil is contained as the hydrophilic surfactant.

Oily component

In the composition according to the present invention, an oily component can be contained. Examples of the oily component preferably include a fatty acid, an acyl glycerol, a plant oil, and a combination thereof. As the oily component, a fatty acid or an acyl glycerol can be further preferably used, and a fatty acid can be more preferably used.
Examples of the fatty acid preferably include caproic acid, caprylic acid, capric acid, lauric acid, myristic acid, myristoleic acid, palmitic acid, palmitoleic acid, stearic acid, oleic acid, linoleic acid, and linolenic acid. Among these, more preferable examples include oleic acid, linoleic acid, and linolenic acid, and further preferable examples include oleic acid.
Examples of the acyl glycerol preferably include triacetin, tributylin, tricaproin, tricapriline, tricaprine, tripalmitine, tripalmitolein, glyceryl tristearate, triolein, trilinolein, trilinolenine, and a triglyceride of medium chain fatty acid. Among these, more preferable examples include triacetin.
Examples of the plant oil include olive oil, almond oil, palm oil, cocoa butter, macadamia nut oil, avocado oil, safflower oil, soybean oil, linseed oil, rapeseed oil, castor oil, corn oil, and palm oil.
As the oily component, oleic acid, triacetin, or a combination thereof can be preferably used, and further preferably it is desirable that oleic acid is used.
In the compositions of the present invention, for the surfactant, preferably, it is desirable that propylene glycol fatty acid ester is used as the hydrophobic surfactant, at least polyoxyethylene castor oil is contained as the hydrophilic surfactant, and a fatty acid is used as the oily component, and more preferably, it is desirable that propylene glycol monocaprylate is used as the hydrophobic surfactant, at least polyoxyl 35 castor oil is contained as the hydrophilic surfactant and oleic acid is used as the oily component.
The composition according to the present invention can further contain an antioxidant. Examples of the antioxidant preferably include dl-$\alpha$-tocopherol, butylated hydroxytoluene, butylated hydroxyanisole, propyl gallate, gallic acid propyl ester, a pharmaceutically acceptable quinone, astaxanthin, and D-$\alpha$-tocopheryl polyethylene glycol 1000 succinate and a combination thereof. More preferable examples of the antioxidant include dl-$\alpha$-tocopherol.
The composition according to the present invention can further contain a solubilizer. Examples of the solubilizer preferably include ethanol, propylene glycol, polyethylene glycol 300, polyethylene glycol 400, diethylene glycol monoethyl ether, and a combination thereof. As the solubilizer, ethanol or propylene glycol can be further preferably used, and propylene glycol can be more preferably used.
The content of Compound 1 or a salt thereof, or a solvate thereof in the composition according to the present invention is not particularly limited as long as it is a concentration at which Compound 1 or a salt thereof, or a solvate thereof can be dissolved in the liquid additive and the optionally used oily component and exert a certain efficacy. However, the content of Compound 1 or a salt thereof, or a solvate thereof based on the total composition may preferably have the upper limit of 10% by weight or less, 9% by weight or less, 8% by weight or less, 7% by weight or less, 6% by weight or less, 5% by weight or less, 4% by weight or less, 3% by weight or less, or 2% by weight or less, more preferably 10% by weight or less, 8% by weight or less, or 7% by weight or less. The lower limit of the content is not particularly limited, but may be preferably more than 0%, 0.1% by weight or more, 0.2% by weight or more, 0.3% by weight or more, 0.4% by weight or more, 0.5% by weight or more, 0.6% by weight or more, 0.7% by weight or more, 0.8% by weight or more, 0.9% by weight or more, or 1% by weight or more, and more preferably 0.1% by weight or more, 0.2% by weight or more, 0.3% by weight or more, 0.4% by weight or more, or 0.5% by weight or more. Furthermore, the content may be in the range of any combination of the lower limit and the upper limit described above. For example, the range of the content may be preferably 0.1% by weight or more and 10% by weight or less, 0.2% by weight or more and 10% by weight or less, 0.3% by weight or more and 10% by weight or less, 0.4% by weight or more and 10% by weight or less, or 0.5% by weight or more and 10% by weight or less.
In the composition according to the present invention, the content of the liquid additive, preferably a surfactant, is not particularly limited as long as the liquid additive is contained so that Compound 1 or a salt thereof, or a solvate thereof can be dissolved in the liquid additive. However, the content of the liquid additive based on the total composition is preferably 50% by weight or more and 97% by weight or less, more preferably 55% by weight or more and 96% by weight or less, further preferably 70% by weight or more and 90% by weight or less.
In the composition according to the present invention, the content of the hydrophobic surfactant based on the total composition is preferably 20% by weight or more and 70% by weight or less, more preferably 25% by weight or more and 65% by weight or less, further preferably 30% by weight or more and 55% by weight or less.
In the composition according to the present invention, the content of the hydrophilic surfactant based on the total composition is preferably 20% by weight or more and 40% by weight or more, more preferably 25% by weight or more and 35% by weight or more, further preferably 28% by weight or more and 33% by weight or less.
In the composition according to the present invention, the weight ratio of the hydrophobic surfactant to the hydrophilic surfactant (hydrophobic surfactant/hydrophilic surfactant) is preferably 0.5 to 3.0, more preferably 1.0 to 2.5, further preferably 1.5 to 2.0.

In the composition according to the present invention, the content of the oily component based on the total composition is preferably 0% by weight or more and 50% by weight or less, more preferably 1% by weight or more and 40% by weight or less, further preferably 10% by weight or more and 20% by weight or less.

When the composition according to the present invention contains an antioxidant, the content of the antioxidant based on the total composition is not particularly limited to the extent that it does not adversely affect the pharmaceutical properties of the formulation, but the content is preferably about 0.01% by weight or more, more preferably about 0.01% by weight or more and 5% by weight or less, further preferably about 0.1% by weight or more and 5% by weight or less, further preferably about 0.1% by weight or more and 2% by weight or less.

When the composition according to the present invention contains a solubilizer, the content of the solubilizer based on the total composition is not particularly limited to the extent that it does not adversely affect the pharmaceutical properties of the formulation, but the content is preferably about 1% by weight or more and 20% by weight or less, more preferably about 2% by weight or more and 15% by weight or less.

In the composition according to the present invention, the weight ratio of the liquid additive to the compound represented by Formula (1) or a salt thereof, or a solvate thereof (liquid additive/Compound 1 or a salt thereof, or a solvate thereof) is not particularly limited as long as Compound 1 or a salt thereof, or a solvate thereof is dissolved in the liquid additive, but preferably 5 or more, more preferably 10 or more. The upper limit of the weight ratio is not particularly limited, but for example, can be preferably 2000 or less, further preferably 1000 or less. The weight ratio may be in the range of any combination of the lower limit and the upper limit, and for example, can be preferably from 5 to 2000, more preferably from 10 to 2000, still more preferably from 10 to 1000. The liquid additive is preferably a surfactant.

In the composition according to the present invention, the weight ratio of the total of the liquid additive and the oily component to the compound represented by Formula (1) or a salt thereof, or a solvate thereof (liquid additive and oily component/Compound 1 or a salt thereof, or a solvate thereof) is not particularly limited as long as Compound 1 or a salt thereof, or a solvate thereof can be dissolved in the liquid additive, but preferably 10 or more. The upper limit of the weight ratio is not particularly limited, but for example, can be preferably 2000 or less, further preferably 1000 or less. The weight ratio may be in the range of any combination of the lower limit and the upper limit, for example, the weight ratio can be preferably from 10 to 2000, more preferably from 10 to 2000. The liquid additive is preferably a surfactant.

The form of the composition of the present invention may be liquid, gel-like, or semisolid, and is preferably liquid.

The composition according to the present invention is characterized in that the average particle size of the droplets formed when the composition is dispersed in a liquid such as water is small, and the particle size distribution is narrow.

For example, for a mixed solution in which the composition of the present invention and water are mixed, the average particle size of the droplets formed when the mixed solution is agitated under a certain dilution ratio (e.g., 0.01 to 1% by volume of the composition with respect to the entire of the mixed solution) at a temperature suitable for handling such as stirring or mixing of the composition and/or at a temperature that does not affect the stability of the substance contained in the composition, e.g., near room temperature (about 25°C), and the composition of the present invention is dispersed in the solution, is preferably less than 200 nm, more preferably 10 nm or more and less than 200 nm, further preferably 50 nm or more and less than 200 nm. Further, the composition of the present invention has a polydispersity index (PDI), which is an index expressing a particle size distribution, of preferably less than 0.5, more preferably 0.4 or less, more preferably 0.3 or less. The average particle size and polydispersity index of the compound in the composition can be determined using known methods such as Dynamic Light Scattering (DLS).

Furthermore, for example, when a solution of a simulated environment (for example, Japanese Pharmacopeia 1st Fluid for dissolution test, pH 1.2, simulated in vivo temperature (e.g., 36 to 37°C), which is used in the disintegration test of an enteric formulation) is used for a mixed solution, the particle size distribution of the droplets formed when the composition of the present invention is stirred at a constant dilution ratio (e.g., 0.01 to 1% by volume of the composition based on the entire mixed solution) to disperse the composition in the solution may similarly be characterized by a small average particle size of the droplets and a narrow width of the particle size distribution. That is, droplets with a small average particle size and a narrow particle size distribution can be formed in the gastrointestinal tract.

The composition of the present invention may have excellent dispersity in a liquid and also have excellent absorption into the body.

<Pharmaceutical formulation>

The pharmaceutical formulation according to the present invention is a pharmaceutical formulation containing a composition containing: Compound 1 or a salt thereof, or a solvate thereof of the present invention; a liquid additive; and optionally an oily component.

The pharmaceutical formulation according to the present invention can be produced by introducing a pharmaceutically acceptable carrier in addition to Compound 1 or a salt thereof, or a solvate thereof of the present invention, a liquid additive, and optionally an oily component, and optionally an antioxidant, a solubilizer, or the like. The pharmaceutical formulation according to the present invention can be formulated with conventional excipients, binders, lubricants, colorants, or flavor modifiers, and if necessary, stabilizers, emulsifiers, absorption enhancers, pH adjusters, preservatives, or the like, and can be formulated by conventional methods by mixing ingredients generally used as raw materials for pharmaceutical formulations.

The formulations according to the present invention may be administered orally or parenterally. Preferably, it is administered orally, but the method of administration is not limited to oral administration.

When producing an oral formulation, for example, Compound 1 or a salt thereof, or a solvate thereof of the present invention, the liquid additive, the optional oily component, and the like, and further, if necessary, a binder, a disintegrant, a lubricant, a colorant, a flavor modifier, or the like are mixed, and then formulated by the conventional method to give a liquid formulation, a capsule formulation, or the like. The composition of the present invention is preferably a liquid, thus it can be formulated into a liquid formulation such as an injection formulation or a capsule formulation.

When the composition of the present invention is formulated into a capsule formulation, the capsule can be a capsule that is commonly used in capsule formulations.

The type of capsule is not particularly limited, and those commonly used in the art can be used. Examples of the capsule include a hard capsule and a soft capsule. The hard capsule typically consists of a cap and a body, and can be produced by covering a body into which the formulation is filled by a cap. Examples of the raw material of the hard capsule include gelatin, hydroxypropyl methylcellulose, pullulan, or a mixture thereof. The size of the capsule may be specified by standards such as No. 9, No. 5, No. 4, No. 3, No. 2, No. 1, No. 0, No. 00, and No. 000. The soft capsule can be produced, for example, by wrapping the formulation with a base agent such as gelatin. Examples of the raw material of the soft capsule include gelatin, starch, carrageenan, agar, glycerol, sorbitol, or a mixture thereof.

Examples of the excipient include lactose, corn starch, white sugar, glucose, mannitol, sorbit, crystalline cellulose, and silicon dioxide.

Examples of the binder include polyvinyl alcohol, polyvinyl ether, methylcellulose, ethylcellulose, gum arabic, tragacanth, gelatin, shellac, hydroxypropyl methylcellulose, hydroxypropylcellulose, polyvinylpyrrolidone, polypropylene glycol, polyoxyethylene block polymer, and meglumine.

Examples of the disintegrant include starch, agar, gelatin powder, crystalline cellulose, calcium carbonate, sodium hydrogen carbonate, calcium citrate, dextrin, pectin, and carboxymethyl cellulose calcium.

Examples of the lubricant include magnesium stearate, talc, polyethylene glycol, silica, and hydrogenated plant oil.

Examples of the colorant include those that are permitted to be added to pharmaceuticals. Examples of the flavor modifier include cocoa powder, menthol, aromatic powder, mint oil, borneol, and cinnamon powder.

The liquid formulation such as a syrup or injectable formulation can be produced by adding a pH adjusting agent, a solubilizer, an isotonic agent, or the like, and further, if necessary, a dissolution aid, a stabilizer, or the like, to Compound 1 or a salt thereof, or a solvate thereof, used in the present invention, and formulating the mixture by a conventional method.

Specific examples of the parenteral administration include an injection dosage form, a nasal dosage form, a pulmonary dosage form, and a transdermal dosage form. The injection dosage form can be administered systemically or topically, for example, by intravenous injection, intramuscular injection, intraperitoneal injection, subcutaneous injection, or the like. The nasal dosage form can be administered systemically or topically, for example, by having the active ingredient absorbed in the formulation from the nasal mucosa or administering the formulation through the nasal cavity. The pulmonary dosage form can be administered systemically or topically, for example, by administering the formulation through the trachea to the lung. The transdermal dosage form can be administered systemically or topically, for example, by plastering the formulation to the skin.

The administration method can be appropriately selected according to the age and symptoms of a patient. The dosage of a pharmaceutical formulation containing Compound 1 or a salt thereof, or a solvate thereof used in the present invention can be selected, for example, from the range of 0.001 mg to 100 mg per kilogram of body weight per dose. Alternatively, the dosage can be selected, for example, from the range of 0.1 to 1000 mg/body per patient. However, the dosage is not necessarily limited to these numerical values. The dosage and method of administration vary depending on the patient's weight, age, symptoms, and the like, but can be appropriately selected by those skilled in the art.

<Production method of composition and pharmaceutical formulation>

The production method of the composition according to present invention includes:

(1) providing a compound represented by the following Formula (1):

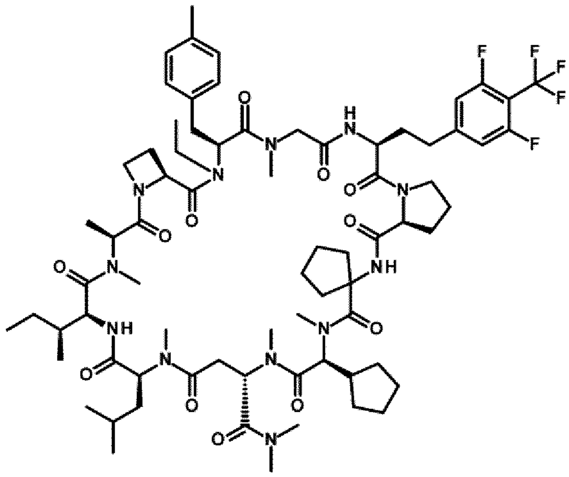

(1)

or a salt thereof, or a solvate thereof, a liquid additive, and optionally an oily component (step 1);

(2) mixing the compound represented by Formula (1) or a salt thereof, or a solvate thereof, the liquid additive, and optionally the oily component (step 2); and

(3) obtaining a composition in which the compound represented by Formula (1) or a salt thereof, or a solvate thereof is dissolved (step 3).

In the method for producing the composition of the present invention, Compound 1 or a salt thereof, or a solvate thereof provided in step (1) is preferably a hydrate of the compound represented by Formula (1). More preferably, it is desirable that the hydrate includes one or more water molecules per compound represented by Formula (1). The number of water molecules contained in the hydrate of the compound represented by Formula (1) may be changed by desorption depending on the surrounding environment such as temperature and humidity.

Furthermore, in the method for producing the composition of the present invention, Compound 1 or a salt thereof, or a solvate thereof, provided in step (1) is preferably a crystal of Compound 1 or a salt thereof or solvate thereof, more preferably a crystal of a hydrate of the compound represented by Formula (1). It is desirable that the water molecule in the crystal of the hydrate of the compound represented by Formula (1) contains one or more water molecules, but the number of water molecules can be changed by desorption depending on the surrounding environment such as temperature and humidity, as described above.

The crystal of the hydrate of the compound represented by Formula (1) can have, in powder X-ray diffraction, a powder X-ray diffraction pattern comprising at least one peak at diffraction angles 2θ of 4.964°, 7.921°, 8.296°, 8.855°, 9.956°, 10.435°, 11.729°, 12.704°, 13.552°, 13.901°, 14.752°, 14.968°, 15.895°, 16.190°, 16.643°, 17.813°, and 19.424° (± 0.2°).

The crystal of the hydrate of the compound represented by Formula (1) can have, in powder X-ray diffraction, a powder X-ray diffraction pattern comprising at least one peak at diffraction angles 2θ of 4.964°, 7.921°, 8.296°, 8.855°, 9.956°, 10.435°, 11.729°, 12.704°, 13.552°, 13.901°, 15.895°, 16.643°, and 17.813° (± 0.2°).

The crystal of the hydrate of the compound represented by Formula (1) can have, in powder X-ray diffraction, a powder X-ray diffraction pattern comprising at least one peak at diffraction angles 2θ of 7.921°, 9.956°, 10.435° 11.729°, 12.704°, 15.895°, and 16.643° (± 0.2°).

The crystal of the hydrate of the compound represented by Formula (1) can have, in powder X-ray diffraction, a powder X-ray diffraction pattern comprising at least two peaks at diffraction angles 2θ of 7.921°, 9.956°, 10.435° 11.729°, 12.704°, 15.895°, and 16.643° (± 0.2°).

The crystal of the hydrate of the compound represented by Formula (1) can have, in powder X-ray diffraction, a powder X-ray diffraction pattern comprising at least three peaks at diffraction angles 2θ of 7.921°, 9.956°, 10.435° 11.729°, 12.704°, 15.895°, and 16.643° (± 0.2°).

The crystal of the hydrate of the compound represented by Formula (1) can have, in powder X-ray diffraction, a powder X-ray diffraction pattern comprising at least four peaks at diffraction angles 2θ of 7.921°, 9.956°, 10.435° 11.729°, 12.704°, 15.895°, and 16.643° (± 0.2°).

The crystal of the hydrate of the compound represented by Formula (1) can have, in powder X-ray diffraction, a powder X-ray diffraction pattern comprising at least five peaks at diffraction angles 2θ of 7.921°, 9.956°, 10.435° 11.729°, 12.704°, 15.895°, and 16.643° (± 0.2°).

The crystal of the hydrate of the compound represented by Formula (1) can have, in powder X-ray diffraction, a powder X-ray diffraction pattern comprising at least six peaks at diffraction angles 2θ of 7.921°, 9.956°, 10.435° 11.729°,

12.704°, 15.895°, and 16.643° (± 0.2°).

It is preferable that the crystal of the hydrate of the compound represented by Formula (1) has, in powder X-ray diffraction, a powder X-ray diffraction pattern comprising peaks at diffraction angles 2θ of 7.921°, 9.956°, 10.435° 11.729°, 12.704°, 15.895°, and 16.643° (± 0.2°).

It is more preferable that the crystal of the hydrate of the compound represented by Formula (1) has, in powder X-ray diffraction, a powder X-ray diffraction pattern comprising peaks at diffraction angles 2θ of 4.964°, 7.921°, 8.296°, 8.855°, 9.956°, 10.435°, 11.729°, 12.704°, 13.552°, 13.901°, 15.895°, 16.643°, and 17.813° (± 0.2°).

It is further preferable that the crystal of the hydrate of the compound represented by Formula (1) has, in powder X-ray diffraction, a powder X-ray diffraction pattern comprising peaks at diffraction angles 2θ of 4.964°, 7.921°, 8.296°, 8.855°, 9.956°, 10.435°, 11.729°, 12.704°, 13.552°, 13.901°, 14.752°, 14.968°, 15.895°, 16.190°, 16.643°, 17.813°, and 19.424° (± 0.2°).

The analysis by powder X-ray diffraction of the present invention can be performed, for example, in accordance with a conventional method such as "Powder X-ray Diffractometry" described in Japanese Pharmacopeia (the 15th edition). In addition, according to the Japanese Pharmacopeia, the diffraction angle 2θ in the same crystal form usually matches within a range of ± 0.2°. Thus, not only crystals whose peak diffraction angles in powder X-ray diffraction match perfectly, but also crystals whose peak diffraction angles match within an error of about ± 0.2° are included in the present invention.

The percentage of each ingredient used, such as Compound 1 or a salt thereof, or a solvate thereof, the liquid additive, and the oily component, is as described above.

In the step of mixing each ingredient, a compound represented by Formula (1) or a salt thereof, or a solvate thereof, the liquid additive, and optionally the oily component, and, if necessary, ingredients commonly used as excipients of pharmaceuticals or the like, are input to a known stirring and mixing device or the like, and mixed.

The mixing temperature and mixing time of each ingredient are not particularly limited as long as they do not affect the ingredients. For example, the mixing temperature is preferably 0 to 50°C, more preferably 10 to 30°C, and the mixing time is preferably about 5 minutes to 60 minutes.

The method for producing the pharmaceutical formulation according to the present invention can include a step of providing the composition of the present invention, and a step of formulating the composition to provide a pharmaceutical formulation.

The method for producing the pharmaceutical formulation according to the present invention can include a step of providing the composition of the present invention, and a step of filling the composition into a capsule to prepare a capsule formulation.

The method for producing the pharmaceutical formulation according to the present invention can include a step of filling the composition obtained from the method for producing a composition into a capsule to provide a capsule formulation. That is, the composition obtained by steps (1) to (3) of the method for producing the composition can be filled into a capsule to provide a capsule formulation.

In the present invention, the term "and/or" is meant to include every combination of the terms "and" and "or" appropriately combined. Specifically, for example, the term "A, B, and/or C" includes the following seven variations; (i) A, (ii) B, (iii) C, (iv) A and B, (v) A and C, (vi) B and C, and (vii) A, B, and C.

As used herein, the term "about", when used in combination with a numeric value, means the value range of + 10% and - 10% of the numeric value.

As used herein, the term "to" that indicates a range includes values of both ends thereof. For example, "A to B" means the range of A or more and B or less.

The unit of molecular weight herein is "g/mol". The unit of molecular weight is sometimes omitted herein.

The use of the articles "one (a)", "one (an)", and "the" in both the specification and claims is to be construed to cover both the singular and the plural, unless specifically indicated herein or expressly denied by context.

All references cited herein are incorporated herein by reference.

[Examples]

The present invention will be described in further detail by way of Examples and Reference Examples, but the present invention is not limited to those. All starting materials and reagents were obtained from commercial suppliers, or synthesized using known methods. The analysis conditions of LC/MS are shown in Table 1.

[Table 1]

| Analysis conditions | Device | Column (I.D. × Length) (mm) | Mobile phase | Gradient (A/B) | Flow rate (mL/min) | Column temperature (°C) | Wavelength | Remarks |
|---|---|---|---|---|---|---|---|---|
| SMDAM05 | Hexer a/2020 | Ascent is Express RP-amide (2.1x50) | A) 0.1% FA, water<br>B) 0.1% FA. acetonitrile | 95/5 (initial) => 0/100 (1.5 minutes) => 0/100 (0.5 minutes) | 1.0 | 35 | 210-400nm<br>PDA total | |
| SMDFA05 | Nexera/2020 | Ascent is Express C18 (2.1×50) | A) 0.1% FA, water<br>B) 0.1% FA, acetonitrile | 95/5 (initial) => 0/100 (1.5 minutes) => 0/100 (0.5 minutes) | 1.0 | 35 | 210-400nm<br>PDA total | |
| SODAA50 | Acquity UPLC/SQD or Acquity UPLC/SQD2 | Ascent is Express C18 (2.1×50) | A) 10mM $AcONH_4$, water<br>B) methanol | 50/50 (initial) => 0/100 (0.7 minutes) => 0/100 (0.7 minutes) | 1.0 | 35 | 210-400nm<br>PDA total | |
| SSC-A-AF-01 | Nexera UC/2020 | Ascentis Express C18 (2.1×50) | A) 10mM $NH_4HCO_2$, water<br>B) methanol | 70/30 (initial) => 0/100 (8.75 minutes) => 0/100 (1.25 minutes) | 0.5 | 50 | 210-400nm<br>PDA total | Loop injection |

(continued)

| Analysis conditions | Device | Column (I.D. × Length) (mm) | Mobile phase | Gradient (A/B) | Flow rate (mL/min) | Column temperature (°C) | Wavelength | Remarks |
|---|---|---|---|---|---|---|---|---|
| SQDFA05 | Acquity UPLC/SQD or Acquity UPLC/SOD2 | Ascentis Express C18 (2.1x50) | A) 0.1% FA, water<br>B) 0.1% FA, acetonitrile | 95/5 (initial) => 0/100 (1.0 minutes) => 0/100 (0.4 minutes) | 1.0 | 35 | 210-400nm<br>PDA total | |
| SMDmethod_05 | Shimadzu<br>LCMS-2020 | Shim-Pack XR-ODS (3.0×50) | A) 0.05% TFA, water<br>B) 0.05% TFA, acetonitrile | 95/5 (initial) => 5/95 (2.0 minutes) => 5/95 (0.7 minutes) | 1.2 | 40 | 190-400nm<br>PDA total | |
| SMDmethod_14 | Shimadzu<br>LCMS-2020 | Shim-Pack XR-0DS (3.0×50) | A) 0.05% TFA, water<br>B) 0.05% TFA, acetonitrile | 70/30 (initial) => 20/80 (3.8 minutes) => 0/100 (0.3 minutes) => 0/100 (0.5 minutes) | 1.2 | 40 | 190-400nm<br>PDA total | |
| SMDmethod_15 | Shimadzu<br>LCMS-2020 | Shim-Pack XR-0DS (3.0×50) | A) 0.05% TFA, water<br>B) 0.05% TFA, acetonitrile | 95/5 (initial) => 0/100 (1.1 minutes) => 0/100 (0.6 minutes) | 1 2 | 40 | 190-400nm<br>PDA total | |

(continued)

| Analysis conditions | Device | Column (I.D. × Length) (mm) | Mobile phase | Gradient (A/B) | Flow rate (mL/min) | Column temperature (°C) | Wavelength | Remarks |
|---|---|---|---|---|---|---|---|---|
| SMDmethod_16 | Shimadzu LCMS-2020 | Accucore C18 (2.1×50) | A) 0.1% FA, water B) 0.1% FA. acetonitri le | 90/10 (initial) => 0/100 (1.1 minutes) => 0/100 (0.5 minutes) | 1 0 | 40 | 190-400nm PDA total | |
| SMDmethod 20 | Sh i madzu LCMS-2020 | Ascentis Express C18 (3.0×50) | A) 0.05% TFA, water B) 0.05% TFA, acetonitrile | 95/5 (initial) => 5/95 (1.1 minutes) => 5/95 (0.5 minutes) | 1.0 | 40 | 190-400nm PDA total | |

Except as otherwise indicated herein, the meanings of the English notations and abbreviations herein are as follows:

Triacetin: triacetin
Oleic acid: oleic acid
Propylene glycol (PG) monocaprylate: propylene glycol monocaprylate
Glycerol monooleate: glycerol monooleate
Sorbitan trioleate: sorbitan trioleate
Polyoxyl 35 castor oil: polyoxyl 35 castor oil
Polyoxyethylene (15) hydroxystearate: polyoxyethylene hydroxystearate
PEG-8 Caprylic/Capric Glycerides: caprylocaproyl polyoxyl-8 glycerides
Polysorbate 80: polysorbate 80
Propylene glycol (PG): propylene glycol
EtOH: ethanol
PEG400: polyethylene glycol 400
D-α-Tocopherol polyethylene glycol 1000 succinate: D-α-tocopherol polyethylene glycol 1000 succinate
dl-α-tocopherol: dl-α-tocopherol
Boc: tert-butoxycarbonyl
t-Bu: tert-butyl
CSA: (+)-10-camphorsulfonic acid
DBU: 1,8-diazabicyclo[5.4.0]-7-undecene
DCM: dichloromethane
DCE: 1,2-dichloroethane
DMA: dimethylacetamide
DMF: N,N-dimethylformamide
DIC: N,N'-diisopropylcarbodiimide
DIPEA: N,N'-diisopropylethylamine
DMAP: N,N-dimethyl-4-aminopyridine
DMSO: dimethyl sulfoxide
EDTA: ethylenediaminetetraacetic acid
Fmoc: 9-fluorenylmethyloxycarbonyl
Fmoc-OSu: N-succinimidyl 9-fluorenylmethyl carbonate
HOAt: 1-hydroxy-7-azabenzotriazole
HOBt: 1-hydroxybenzotriazole
HOOBt: 3,4-dihydro-3-hydroxy-4-oxo-1,2,3-benzotriazine
MTBE: methyl tert-butyl ether
NMP: N-methyl-2-pyrrolidone
oxyma: ethyl cyano(hydroxyimino)acetate
TES: triethylsilane
TFA: trifluoroacetylacetic acid
TFE: 2,2,2-trifluoroethanol
THF: tetrahydrofuran
TfOH: trifluoromethanesulfonic acid
TsOH: p-toluenesulfonic acid
WSCI·HCl: 1-ethyl-3-(3-dimethylaminopropyl)carbodiimide hydrochloride

Reagents such as a liquid additive, a surfactant, an oily component, a solubilizer, an antioxidant, or a solvent used in the implementation of the present invention were used as it was provided by commercial suppliers without purification other than those specifically described.

The mixing of the additives and the mixing of the compound represented by Formula (1) or a salt thereof, or a solvate thereof with the additives or formulations were carried out in a methods well known to those skilled in the art, such as shaking or stirring, so that the mixture became uniform, using normal laboratory equipment.

The measurement of the solubility of the compound represented by Formula (1) or a salt thereof, or a solvate thereof was performed by adding the additive or the formulation to the compound, then subjecting the mixture to manipulations such as shaking or stirring, and confirming the dissolution visually, and calculating the solubility from the amount of the liquid added. The solubility can also be measured with devices such as high performance liquid chromatograph.

Test method 1: Particle size distribution measurement by dynamic light scattering (DLS)

Particle size distribution by dynamic scattering (DLS) is determined using ZETASIZER Nano-ZS (Malvern Panalytical Ltd.). The samples are placed in disposable cells (cuvettes for small amounts) and set in ZETASIZER Nano-ZS (Malvern Panalytical Ltd.) to determine the particle size distribution by dynamic light scattering (DLS). The average particle size (Z-average size) and polydispersity index (PDI) are used as indicators for the particle size distribution. Z-average is the average particle size based on the scattering intensity by the method of cumulants. PDI is an indicator representing the width of the particle size distribution, and indicated in the range of 0 to 1. PDI = 0 represents a suspension with no distribution of particle size. A dispersion having PDI of 0.1 or less is considered as having monodispersity, and a dispersion having PDI between 0.1 and 0.5 is considered to have a narrow distribution. While, a dispersion having PDI of greater than 0.5 is considered as having polydispersity.

Test method 2: Dispersity assessment

Dispersion profiles are analyzed using μDISS Profiler (Pion Inc.) to estimate the dispersion performance of the formulation into the fasted state simulated intestinal fluid (FaSSIF). 100 μL of each formulation was added to 10 mL of FaSSIF surface warmed at 37°C and the mixture was stirred at a rate of 200 rpm. At the timepoints of 5, 10, 15, 20, 25, 30, and 60 minutes, 50 μL of the solution is aliquoted from the center of the container through the guide plastic tube, and the concentration of the compound of interest is determined by UPLC. The dispersity (%) is determined by the following equation.

Dispersity (%) = (concentration of compound in aliquoted solution/concentration of compound when entire formulation is uniformly dispersed) X 100

Test method 3: Calculation of pharmacokinetic parameters

Plasma is separated from the collected blood by centrifugation, and after the deproteinization with acetonitrile, the plasma concentration is measured with an LC-MS/MS device. From the obtained changes of the plasma concentration, the area under the plasma drug concentration vs time curve (AUC) and the maximum plasma concentration (Cmax) are calculated by non-compartmental analysis using a pharmacokinetic analysis software Phoenix WinNonlin 8.2 (Certara, L. P.).

Preparation Example 1:

Preparation of Compound 1

Compound 1 ("(5S,8S,11S,15S,18S,23aS,29S,35S,37aS)-8-((S)-sec-butyl)-18-cyclopentyl-29-(3,5-difluoro-4-(trifluoromethyl)phenethyl)-36-ethyl-11-isobutyl-N,N,5,6,12,16,19,33-octamethyl-35-(4-methylbenzyl)-4,7,10,13,17,20,23,28,31,34,37-undecaoxotetratriacontahydro-2H,4H-spiro[azeto[2,1-u]pyrrolo[2,1-i][1,4,7,10,13,16,19,22,25,28,31]undecaazacyclotetratriacontin-21,1'-cyclopentane]-15-carboxamide") having the following structure was synthesized according to Scheme 1 below:

Compound 1

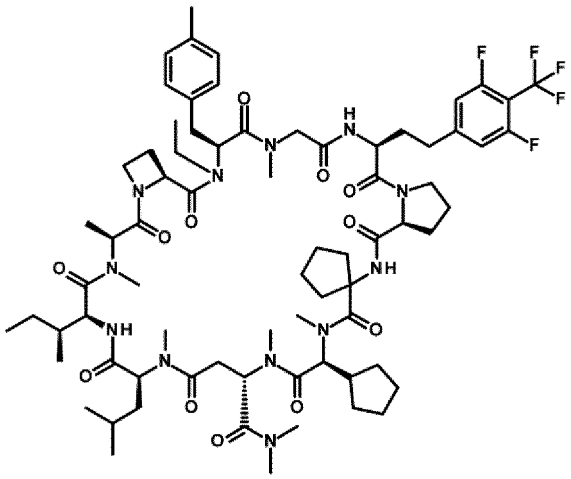

Scheme 1

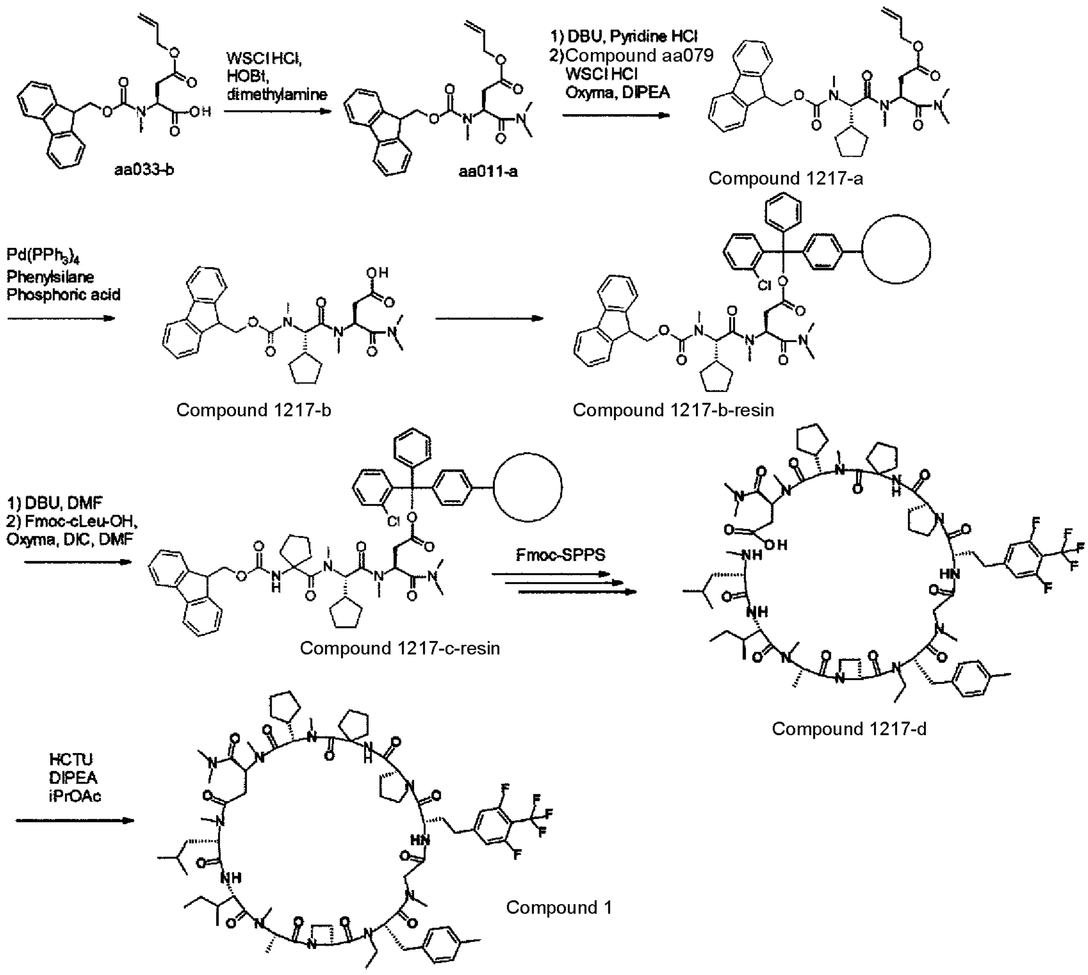


aa033-b

aa011-a

Compound 1217-a

Compound 1217-b

Compound 1217-b-resin

Compound 1217-c-resin

Compound 1217-d

Compound 1

Synthesis of compound aa033-b ((2S)-2-[9H-fluoren-9-ylmethoxycarbonyl(methyl)amino]-4-oxo-4-prop-2-enoxybutanoic acid)

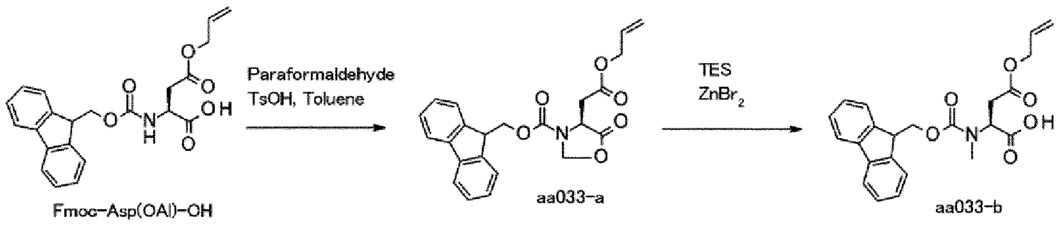

Fmoc-Asp(OAl)-OH
CAS 146982-24-3
aa033-a
aa033-b

Fmoc-Asp(OAl)-OH ((2S)-2-(9H-fluoren-9-ylmethoxycarbonylamino)-4-oxo-4-prop-2-enoxybutanoic acid, CAS No. 146982-24-3)(200 g, 506 mmol), p-toluenesulfonic acid (5.7 g, 0.05 equivalent), paraformaldehyde (45.6 g, 3 equivalent) were mixed into toluene, and the mixture was stirred at 110°C for 16 hours. The solvent of the reaction solution was distilled off under reduced pressure, and the residue was dissolved in ethyl acetate and washed twice with an aqueous sodium hydrogen carbonate solution. The organic layer was dried over anhydrous sodium sulfate, and then the solvent was distilled off under reduced pressure. The residue was purified by silica gel column chromatography (ethyl acetate/petroleum ether, 0/100 to 30/70) to obtain compound aa033-a (9H-fluoren-9-ylmethyl(4S)-5-oxo-4-(2-oxo-2-prop-2-enoxyethyl)-1,3-oxazolidine-3-carboxylate (175 g, 85%). Another batch that was similarly synthesized was mixed and used in the next reaction.

LCMS(ESI) m/z = 408(M+H)$^+$
Retention time: 1.407 minutes (analysis condition SMD method_20)

A mixed solution of compound aa033-a (100 g, 245 mmol), zinc bromide ($ZnBr_2$) (110 g, 496 mmol), triethylsilane (TES) (56 g, 481.6 mmol) in dichloromethane (DCM) (1 L) was stirred under a nitrogen atmosphere at room temperature for 48 hours. Four batches of reaction solution at the same scale were mixed and the solvent was distilled off under reduced pressure. The residue was dissolved in MTBE and extracted 10 times with 0.5 M phosphate buffer (pH = about 7.5). The aqueous layer was mixed, and the pH was adjusted to 2 with 5 M aqueous hydrochloric acid, and the mixture was extracted twice with isopropyl acetate (IPAC). The organic layers were combined, dried over anhydrous sodium sulfate, and the solvent was distilled off under reduced pressure. In order to remove IPAC, MTBE was added to the resulting residue and the solvent was distilled off under reduced pressure. This procedure was repeated six times to obtain compound aa033-b ((2S)-2-[9H-fluoren-9-ylmethoxycarbonyl (methyl)amino]-4-oxo-4-prop-2-enoxybutanoic acid). (270 g, 54%)

LCMS(ESI) m/z = 410(M+H)$^+$
Retention time: 1.956 minutes (analysis condition SMD method_05)

Synthesis of compound aa011-a

HOBt (17.72 g, 131 mmol) was added to a solution of WSCI·HCl (27.4 g, 143 mmol) in DMF (217 mL) with ice cooling under a nitrogen atmosphere. Further, compound aa033b (48.8 g, 119 mmol) was added as a mixed solution in DCM (90 mL) and DMF (90 mL), and the mixture was stirred at 0°C for 30 minutes. A THF solution of dimethylamine (2 mol/l, 65.6 mL, 131 mmol) was added dropwise thereto, and the mixture was stirred at 0°C for 30 minutes. The reaction solution was diluted with ethyl acetate (488 mL), and the organic phase was washed twice with hydrochloric acid (1 mol/L, 390 mL), followed by washing with water, twice with a mixed solution of saturated aqueous sodium hydrogen carbonate solution and water (1 : 1, 488 mL), and once with a mixed solution of saturated brine and water (1 : 1, 488 mL). Then, the obtained organic phase was dried over anhydrous sodium sulfate and the solvent was distilled off under reduced pressure to obtain compound aa011-a. (51.16 g, yield 98%).

LCMS(ESI) m/z = 437.0(M+H)$^+$
Retention time: 1.262 minutes (analysis condition SMDFA05)

Synthesis of compound aa079, (2S)-2-cyclopentyl-2-[9H-fluoren-9-ylmethoxycarbonyl(methyl)amino]acetic acid (Fmoc-MeGly(cPent)-OH)

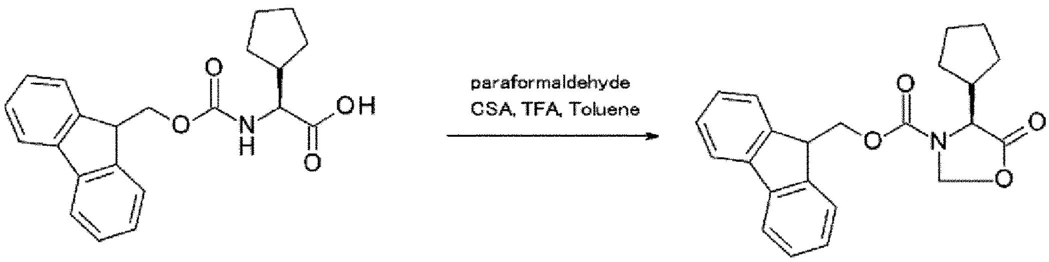


aa079-a aa079-b

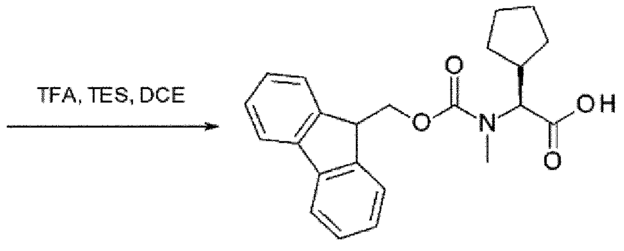


aa079

To a mixed solution of compound aa079-a ((2S)-2-cyclopentyl-2-[9H-fluoren-9-ylmethoxycarbonylamino]acetic acid, Fmoc-Gly (cPent)-OH (CAS number: 220497-61-0) (30.0 g, 82 mmol), paraformaldehyde (7.39 g, 246 mmol), and CSA (0.954 g, 4.10 mmol) in toluene (160 mL), trifluoroacetic acid (TFA) (9.0 mL) was added, and the mixture was stirred at 60°C for 4 hours. After cooling the reaction solution to room temperature, the solid was removed by filtration. The filtrate was concentrated under reduced pressure, diluted with ethyl acetate (220 mL), and then washed sequentially with saturated aqueous sodium hydrogen carbonate solution and saturated brine. The organic layer was dried over anhydrous sodium sulfate, filtered, and concentrated under reduced pressure to obtain compound aa079-b as a crude product. The compound was used for the next reaction without further purification.

LCMS(ESI) m/z = 378(M+H)$^+$
Retention time: 1.01 minutes (analysis condition SQDFA05)

To a mixed solution of triethylsilane (TES) (65.5 mL, 410 mmol) and aa079-b in dichloroethane (DCE) (90 mL), using the total amount of compound aa079-b obtained above, trifluoroacetic acid (TFA) (76 mL, 984 mmol) was added, and the mixture was stirred at 60°C for 16 hours. After the reaction solution was cooled to room temperature and then concentrated under reduced pressure, the obtained solid was washed with n-hexane/ethyl acetate (95/5) and dried under reduced pressure to obtain compound 2279 ((2S)-2-cyclopentyl-2-[9H-fluoren-9-ylmethoxycarbonyl (methyl)amino]acetic acid, Fmoc-MeGly(cPent)-OH) (29.1 g, 93% in 2 steps).

LCMS(ESI) m/z = 380(M+H)$^+$
Retention time: 0.92 minutes (analysis condition SQDFA05)

Synthesis of Compound 1217-a

To a solution of compound aa079 (42.2 g, 111 mmol) and Oxyma (19.99 g, 141 mmol) in DMF (391 mL), WSCI·HCl (31.5 g, 164 mmol) was added at room temperature, and the mixture was stirred for 30 minutes to obtain solution A.

To a solution of compound aa011-a (51.16 g, 117 mmol) in DMF (391 mL) under the nitrogen atmosphere, DBU (17.49 mL, 117 mmol) was added dropwise at room temperature, and the mixture was stirred for 5 minutes. Pyridine hydrochloride (14.9 g, 129 mmol) was added thereto, and the mixture was stirred for 10 minutes. To the obtained reaction solution, solution A and DIPEA (22.46 mL, 129 mmol) were added, and the mixture was stirred under the nitrogen atmosphere at room temperature for 7 hours. The reaction solution was diluted with ethyl acetate (422 mL), washed twice with hydrochloric acid (1 mol/L, 422 mL), and the obtained aqueous phase was extracted twice with ethyl acetate (422 mL). All organic phases were mixed, and washed sequentially with water (422 mL), with a mixed solution of saturated aqueous sodium hydrogen carbonate solution and water (1 : 1, 422 mL), and with a mixed solution of saturated brine and water (1 : 1, 422 mL). Then, the obtained organic phase was dried over sodium sulfate and the solvent was distilled off under reduced pressure. To the resulting residue, DCM (512 mL) was added, and the mixture was stirred for 0.5

hours. Magnesium sulfate (30 g) was added thereto, and the mixture was stirred for 30 minutes and then filtered to remove solids. The resulting solution was purified by silica gel column chromatography (hexane/ethyl acetate) to yield Compound 1217-a. (55.55 g, yield 87%).

LCMS(ESI) m/z = 598.2$(M+Na)^+$
Retention time: 1.320 minutes (analysis condition SMDAM05)

Synthesis of Compound 1217-b

To a solution of Compound 1217-a (55.55 g, 96 mmol) in DCM (193 mL) under the nitrogen atmosphere, tetrakis(triphenylphosphine) palladium(0) (1.115 g, 0.965 mmol) was added at room temperature, and phenylsilane (8.31 mL, 67.5 mmol) was added dropwise, and the mixture was stirred for 30 minutes. The reaction solution was diluted with MTBE (556 ml), and extracted with a mixed solution of saturated aqueous sodium hydrogen carbonate solution and water (1 : 1, 556 ml). The obtained organic phase was extracted with water (278 ml). The aqueous phase was mixed, and DCM (556 ml) was added. Phosphoric acid (56.7 g, 579 mmol) was added dropwise thereto, and the pH was adjusted to 2 to 3. The organic phase was separated, and the aqueous phase was extracted with DCM (556 ml). The obtained organic phases were mixed, washed with a mixed solution of saturated brine and water (1 : 1, 556 mL), and then dried over sodium sulfate, and the solvent was distilled off under reduced pressure to obtain Compound 1217-b. (48.87 g, yield 95%).

LCMS(ESI) m/z = 536$(M+H)^+$
Retention time: 1.138 minutes (analysis condition SMDAM05)

Synthesis of Compound 1217-b-resin

A reaction vessel with a filter was charged with 2-chlorotrityl chloride resin (purchased from Sunresin New Materials Co. Ltd., 1.36 mmol/g, 114 g, 155 mmol), and DCM (1140 mL) was added. The mixture was stirred at 25°C for 45 minutes, and the solvent was then discharged from the filter. A solution of Compound 1217-b (48.87 g, 91 mmol), methanol (29.6 mL, 730 mmol) and DIPEA (76 mL, 438 mmol) in DCM (798 mL) was added to the reaction vessel. The mixture was stirred at 25°C for 60 minutes, and the solution was discharged from the filter. Subsequently, a solution of methanol (111 mL, 2737 mmol) and DIPEA (76 mL, 438 mmol) in DCM (684 mL) was added to the reaction vessel. The mixture was stirred at 25°C for 90 minutes, and the solution was then discharged from the filter. To the reaction vessel, DCM (570 mL) was added, and the mixture was stirred for 5 minutes, and the solution was discharged from the filter. This washing procedure of resin was repeated four more times, and the obtained resin was dried under reduced pressure to obtain Compound 1217-b-resin (140.5 g). The supporting amount was calculated to be 0.482 mmol/g by the quantitative method of resin described in this Example.

Synthesis of Compound 1217-c-resin

The resin obtained above (0.482 mmol/g, 60 g, 28.92 mmol) was set in a plastic solid-phase reaction vessel. DCM (600 mL) was added to this solid-phase reaction vessel at room temperature, and after shaking for 5 minutes, the solvent was discharged from the frit. DMF (420 mL) was added to this solid-phase reaction vessel, and after shaking for 5 minutes, the solvent was discharged from the frit. This washing step of resin was repeated one more time. A solution of diazabicycloundecene (DBU) in DMF (2 v/v%, 420 mL) was added to this solid-phase reaction vessel, and deprotection of a Fmoc group was performed. After shaking for 10 minutes, the solution was discharged from the frit. DMF (420 mL) was added to this solid-phase reaction vessel, and after shaking for 5 minutes, the solution was discharged from the frit. A solution of triethylamine hydrochloride (7.96 g, 57.8 mmol) in DCM (420 mL) was added to this solid-phase reaction vessel, and after shaking for 5 minutes, the solution was discharged from the frit. DCM (420 mL) was added to this solid-phase reaction vessel, and after shaking for 5 minutes, the solution was discharged from the frit. DMF (420 mL) was added to this solid-phase reaction vessel, and after shaking for 5 minutes, the solution was discharged from the frit. This washing step of resin with DMF was repeated one more time.

A solution of Fmoc-cLeu-OH (40.7 g, 116 mmol) (CAS number: 117322-30-2) and Oxyma (10.3 g, 72.3 mmol) in DMF (180 mL) and a solution of N,N'-diisopropylcarbodiimide (DIC) in DMF (10%, 216 mL) were mixed, and after 2 minutes, the mixture was added to the solid-phase reaction vessel obtained above. This solid-phase reaction vessel was shaken at 50°C for 24 hours, and the solution was then discharged from the frit. DMF (420 mL) was added to this solid-phase reaction vessel, and after shaking at room temperature for 5 minutes, the solution was discharged from the frit. This washing step of resin with DMF was repeated four more times. DCM (420 mL) was added to this solid-phase reaction vessel, and after shaking at room temperature for 5 minutes, the solution was discharged from the frit. This

washing step of resin with DCM was repeated five more times. The resulting resin was dried under reduced pressure to obtain Compound 1217-c-resin (62.5 g).

Synthesis of compound aa134

Synthesis of compound aa134, (2S)-4-[3,5-difluoro-4-(trifluoromethyl)phenyll-2-(9H-fluoren-9-ylmethoxycarbonylamino)butanoic acid, Fmoc-Hph (4-CF3-35-F2)-OH

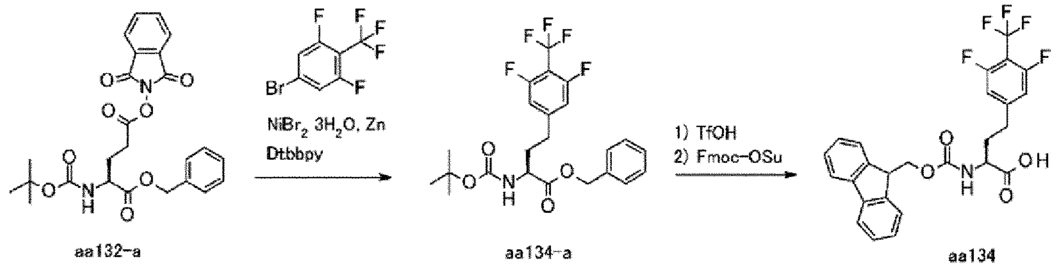


Synthesis of compound aa132-a, (1-O-benzyl-5-O-(1,3-dioxoisoindol-2-yl)(2S)-2[(2-methylpropan-2-yl)oxycarbonylamino]pentanedioate

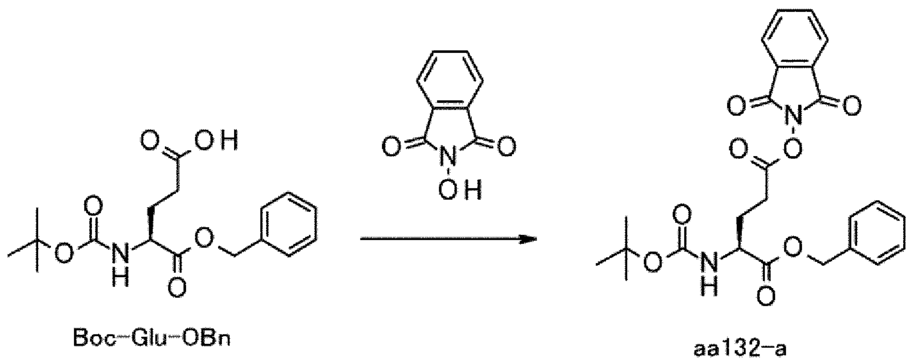


To a solution of (4S)-4-[(2-methylpropan-2-yl)oxycarbonylamino]-5-oxo-5-phenylmethoxypentanoic acid (Boc-Glu-OBn, CAS No. 30924-93-7) (200 g, 592.82 mmol), N-hydroxyphthalimide (106 g, 649.78 mmol, 1.10 equivalent), DMAP (3.6 g, 29.47 mmol, 0.05 equivalent) in THF (2 L), DIC (138 mL, 1.54 equivalent) was added dropwise at 0°C under the nitrogen atmosphere. The reaction solution was stirred at 25°C for 16 hours, then solids were removed by filtration, and the solvent of the filtrate was distilled off under reduced pressure. The residue was diluted with toluene, and the resulting solid was removed by filtration, and the solvent of the filtrate was distilled off under reduced pressure. The residue was purified by recrystallization (acetone/heptane) to obtain compound aa132-a (1-O-benzyl-5-O-(1,3-dioxoisoindol-2-yl)(2S)-2[(2-methylpropan-2-yl)oxycarbonylamino]pentanedioate. (230 g, 80%)

LCMS(ESI) m/z = 505.2(M+Na)$^+$
Retention time: 0.992 minutes (analysis condition SMD method_16)

Nickel bromide trihydrate ($NiBr_2 \cdot 3H_2O$) (13.5 g, 49.7 mmol, 0.3 equivalent) and 4,4'-di-tert-butyl-2,2'-bipyridyl (dtbbpy, CAS No. 72914-19-3) (13.3 g, 49.7 mmol, 0.3 equivalent) was added to DMA (400 mL), and the mixture was stirred at 50°C for 3 hours under the nitrogen atmosphere to prepare a Ni solution.

A mixed solution of aa132-a (1-O-benzyl-5-O-(1,3-dioxoisoindol-2-yl)(2S)-2[(2-methylpropan-2-yl)oxycarbonylamino]pentanedioate (80 g, 166 mmol), zinc powder (54.2 g, 829 mmol, 5 equivalent), and 4-bromo-1,3-difluoro-2-(trifluoromethyl)benzene (CAS No. 156243-64-0, 86.6 g, 332 mmol, 2 equivalent) in DMA (400 mL) was stirred under the nitrogen atmosphere at room temperature for 1 hour. The previously prepared Ni solution was added thereto, and the mixture was stirred at room temperature for 16 hours. An aqueous EDTA/2Na solution (800 mL, 10%) was added

to the reaction solution, and solids were removed by filtration. The filtrate was extracted with ethyl acetate, then the combined organic layers were washed with saturated brine and dried over anhydrous sodium sulfate, and the solvent was distilled off under reduced pressure. The resulting residue was purified by silica gel column chromatography (ethyl acetate/petroleum ether) to obtain compound aa134-a. (57.2 g, 69%).

LCMS(ESI) m/z = 496(M+Na)$^+$
Retention time: 1.544 minutes (analysis condition SMD method_15)

A mixed solution of compound aa134-a (57.2 g, 121 mmol) in toluene (690 mL) was cooled to 0°C, and trifluoromethanesulfonic acid (TfOH) (54.4 g, 362 mmol, 3 equivalent) was added dropwise. The mixture was stirred at room temperature for 1 hour, then water (58 mL) was added. This mixed solution was extracted with water and the combined aqueous layers were extracted with ethyl acetate. The combined organic layers were washed with water, dried over anhydrous sodium sulfate, and the solvent was distilled off under reduced pressure to obtain 60 g of residue. To the residue, acetonitrile/water (400/400 mL) was added, and the pH of the mixture was adjusted to 7 with an aqueous sodium hydroxide solution (48%). Fmoc-OSu (36.6 g, 108.6 mmol, 0.9 equivalent) was added to this solution, the pH was adjusted to 8.0 with an aqueous sodium hydroxide solution (48%), and then the mixture was stirred at room temperature for 16 hours. The solid components were removed by filtering the reaction solution while washing with acetonitrile/water (1/1), and the filtrate was diluted with acetonitrile and adjusted to be acidic with 6 mol/L aqueous hydrochloric acid, then the precipitated solid was collected by filtration to obtain compound aa134 ((2S)-4-[3,5-difluoro-4-(trifluoromethyl)phenyl]-2-(9H-fluoren-9-ylmethoxycarbonylamino)butanoic acid, Fmoc-Hph (4-CF3-35-F2)-OH). (52 g, 83%)

LCMS(ESI) m/z = 528.45(M+Na)$^+$
Retention time: 3.538 minutes (analysis condition SMD method_14)
1H-NMR (300 MHz, DMSO-d6) δ 12.69 (s, 1H), 7.90 (d, J = 7.5 Hz, 2H), 7.78-7.54 (m, 3H), 7.48-7.20 (m, 6H), 4.33 (d, J = 6.3 Hz, 2H), 4.24 (t, J = 6.9 Hz, 1H), 3.97-3.84 (m, 1H), 2.79-2.65 (m, 2H), 2.15-2.00 (m, 1H), 2.00-1.83 (m, 1H)

<u>Synthesis of Compound aa113, (2S)-2-[ethyl(9H-fluoren-9-ylmethoxycarbonyl)amino]-3-(4-methylphenyl)propanoic acid (Fmoc-EtPhe(4-Me)-OH)</u>

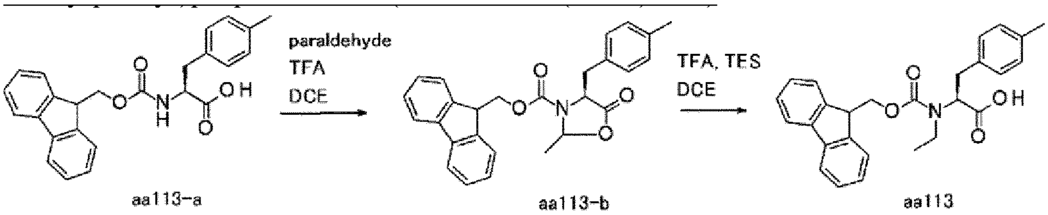


aa113-a aa113-b aa113

Under the nitrogen atmosphere, compound aa113 ((2S)-2-ethyl(9H-fluoren-9-ylmethoxycarbonyl)amino]-3-(4-methylphenyl)propanoic acid (Fmoc-EtPhe(4-Me)-OH) (5.62 g, 14.0 mmol, CAS number 199006-54-7) was suspended in dichloroethane (DCE) (17.5 mL), and para-aldehyde (5.61 mL, 42.0 mmol) and trifluoroacetic acid (TFA) (9.65 mL, 126 mmol) were added, and the mixture was stirred at 60 degrees for 6 hours. The reaction solution containing the obtained compound aa113-b was directly used in the next step.

LCMS(ESI) m/z = 428(M+H)$^+$
Retention time: 1.03 minutes (analysis condition SQDFA05)

To the obtained reaction solution of compound aa113-b, dichloroethane (DCE) (17.5 mL), trifluoroacetic acid (TFA) (19.3 mL, 252 mmol), triethylsilane (TES) (20.1 mL, 126 mmol) were added, and the mixture was stirred at 60 degrees for 17 hours. The mixture was cooled to room temperature and concentrated under reduced pressure, then the resulting residue was dissolved in ethyl acetate (40 mL). The organic layer was washed with saturated aqueous sodium hydrogen carbonate solution (40 mL) and saturated brine (40 mL) and dried over anhydrous sodium sulfate, then the solvent was distilled off under reduced pressure. The resulting residue was dissolved in acetonitrile (30 mL), and washed twice with hexane (15 mL), and the solvent was distilled off under reduced pressure. The resulting residue was purified by reverse-phase column chromatography (0.1%-acetonitrile with formic acid/0.1%-distilled water with formic acid) to obtain compound aa113 ((2S)-2-[ethyl(9H-fluoren-9-ylmethoxycarbonyl)amino]-3-(4-methylphenyl)propanoic acid (Fmoc-EtPhe(4-Me)-OH). (4.4 g, 73% in 2 steps)

LCMS(ESI) m/z = 430(M+H)+
Retention time: 0.95 minutes (analysis condition SQDFA05)

Subsequent extensions of Fmoc-Pro-OH (CAS number: 71989-31-6), Fmoc-Hph (4-CF3-35-F2)-OH (compound aa134), Fmoc-MeGly-OH (CAS number: 77128-70-2), Fmoc-EtPhe (4-Me)-OH (compound aa113), Fmoc-Aze(2)-OH (CAS number: 136552-06-2), Fmoc-MeAla-OH (CAS number: 84000-07-7), and Fmoc-Ile-OH (CAS number: 71989-23-6) were synthesized by Fmoc solid phase synthesis method using a peptide synthesizer (Multipep RSi) manufactured by Intavis Peptide Services GmbH. Detailed procedures of the manipulation were followed the manual attached to the synthesizer.

Compound 1217-c-resin (200 mg per 1 solid-phase reaction vessel) obtained above was added to 30 solid-phase reaction vessels, and the vessels were set in the peptide synthesizer. To all 30 solid-phase reaction vessels, dichloromethane (DCM) was added and allowed to stand for 1 hour to swell the resin. The solvent was then discharged from the frit.

Extension of Fmoc-Pro-OH

A solution of diazabicycloundecene (DBU) in DMF (2 v/v%, 1.4 mL per 1 solid-phase reaction vessel) was added to all 30 solid-phase reaction vessels, and the vessels were warmed to 30°C, and after 10 minutes, the solution was discharged from the frit. To all 30 solid-phase reaction vessels, DMF was added (1.4 mL per 1 solid-phase reaction vessel), and the solvent was discharged from the frit. This washing step of resin with DMF was repeated three more times. Subsequently, a solution of Fmoc-Pro-OH (CAS No. 71989-31-6) (0.6 mol/L) and HOAt (0.375 mol/L) in NMP (0.6 mL per 1 solid-phase reaction vessel) and a solution of N,N'-diisopropylcarbodiimide (DIC) in DMF (10 v/v%, 0.72 mL per 1 solid-phase reaction vessel) were mixed by a mixing vial of the synthesizer, then added to all 30 solid-phase reaction vessels and the vessels were allowed to stand still at 40°C for 4 hours. The solution was then discharged from the frit. To all 30 solid-phase reaction vessels, DMF was added (1.4 mL per 1 solid-phase reaction vessel), and the solvent was discharged from the frit. This washing step of resin was repeated two more times.

Extension of Fmoc-Hph(4-CF3-35-F2)-OH (compound aa134)

A solution of diazabicycloundecene (DBU) in DMF (2 v/v%, 1.4 mL per 1 solid-phase reaction vessel) was added to all 30 solid-phase reaction vessels containing the resin obtained above, and the vessels were warmed to 35°C, and after 10 minutes, the solution was discharged from the frit. To all 30 solid-phase reaction vessels, DMF was added (1.4 mL per 1 solid-phase reaction vessel), and the solvent was discharged from the frit. This washing step of resin with DMF was repeated three more times. Subsequently, a solution of Fmoc-Hph (4-CF3-35-F2)-OH (compound aa134) (0.45 mol/L) and HOAt (0.375 mol/L) in NMP (0.6 mL per 1 solid-phase reaction vessel) and a solution of N,N'-diisopropylcarbodiimide (DIC) in DMF (10 v/v%, 0.72 mL per 1 solid-phase reaction vessel) were mixed by a mixing vial of the synthesizer, then added to all 30 solid-phase reaction vessels and the vessels were allowed to stand still at 40°C for 2.5 hours. The solution was then discharged from the frit. To all 30 solid-phase reaction vessels, DMF was added (1.4 mL per 1 solid-phase reaction vessel), and the solvent was discharged from the frit. This washing step of resin was repeated two more times.

Extension of Fmoc-MeGly-OH

A solution of diazabicycloundecene (DBU) in DMF (2 v/v%, 1.4 mL per 1 solid-phase reaction vessel) was added to all 30 solid-phase reaction vessels containing the resin obtained above, and the vessels were warmed to 35°C, and after 10 minutes, the solution was discharged from the frit. To all 30 solid-phase reaction vessels, DMF was added (1.4 mL per 1 solid-phase reaction vessel), and the solvent was discharged from the frit. This washing step of resin with DMF was repeated three more times. Subsequently, a solution of Fmoc-MeGly-OH (0.6 mol/L) and HOAt (0.375 mol/L) in NMP (0.6 mL per 1 solid-phase reaction vessel) and a solution of N,N'-diisopropylcarbodiimide (DIC) in DMF (10 v/v%, 0.72 mL per 1 solid-phase reaction vessel) were mixed by a mixing vial of the synthesizer, then added to all 30 solid-phase reaction vessels and the vessels were allowed to stand still at 40°C for 2.5 hours. The solution was then discharged from the frit. To all 30 solid-phase reaction vessels, DMF was added (1.4 mL per 1 solid-phase reaction vessel), and the solvent was discharged from the frit. This washing step of resin was repeated two more times.

Extension of Fmoc-EtPhe (4-Me)-OH (compound aa113)

A solution of diazabicycloundecene (DBU) in DMF (2 v/v%, 1.4 mL per 1 solid-phase reaction vessel) was added to all 30 solid-phase reaction vessels containing the resin obtained above, and the vessels were warmed to 35°C,

and after 10 minutes, the solution was discharged from the frit. To all 30 solid-phase reaction vessels, DMF was added (1.4 mL per 1 solid-phase reaction vessel), and the solvent was discharged from the frit. This washing step of resin with DMF was repeated three more times. Subsequently, a solution of Fmoc-EtPhe (4-Me)-OH (0.6 mol/L) produced as described above and HOAt (0.375 mol/L) in NMP (0.6 mL per 1 solid-phase reaction vessel) and a solution of N,N'-diisopropylcarbodiimide (DIC) in DMF (10 v/v%, 0.72 mL per 1 solid-phase reaction vessel) were mixed by a mixing vial of the synthesizer, then added to all 30 solid-phase reaction vessels and the vessels were allowed to stand still at 40°C for 2.5 hours. The solution was then discharged from the frit. To all 30 solid-phase reaction vessels, DMF was added (1.4 mL per 1 solid-phase reaction vessel), and the solvent was discharged from the frit. This washing step of resin was repeated two more times.

Extension of Fmoc-Aze(2)-OH

A solution of diazabicycloundecene (DBU) in DMF (2 v/v%, 1.4 mL per 1 solid-phase reaction vessel) was added to all 30 solid-phase reaction vessels containing the resin obtained above, and the vessels were warmed to 35°C, and after 10 minutes, the solution was discharged from the frit. To all 30 solid-phase reaction vessels, DMF was added (1.4 mL per 1 solid-phase reaction vessel), and the solvent was discharged from the frit. This washing step of resin with DMF was repeated three more times. Subsequently, a mixed solution of Fmoc-Aze(2)-OH (0.6 mol/L) and HOOBt (0.375 mol/L) in NMP and DMSO (7 : 3) (0.6 mL per 1 solid-phase reaction vessel) and a solution of N,N'-diisopropylcarbodiimide (DIC) in DMF (10 v/v%, 0.72 mL per 1 solid-phase reaction vessel) were mixed by a mixing vial of the synthesizer, then added to all 30 solid-phase reaction vessels and the vessels were allowed to stand still at 60°C for 5 hours. A solution of N,N'-diisopropylcarbodiimide (DIC) in DMF (10 v/v%, 0.72 mL per 1 solid-phase reaction vessel) was then added to all 30 solid-phase reaction vessels and the vessels were allowed to stand still at 60°C for 5 hours. The solution was then discharged from the frit. To all 30 solid-phase reaction vessels, DMF was added (1.4 mL per 1 solid-phase reaction vessel), and the solvent was discharged from the frit. This washing step of resin was repeated two more times.

Extension of Fmoc-MeAla-OH

A solution of diazabicycloundecene (DBU) in DMF (2 v/v%, 1.4 mL per 1 solid-phase reaction vessel) was added to all 30 solid-phase reaction vessels containing the resin obtained above, and the vessels were warmed to 35°C, and after 10 minutes, the solution was discharged from the frit. To all 30 solid-phase reaction vessels, DMF was added (1.4 mL per 1 solid-phase reaction vessel), and the solvent was discharged from the frit. This washing step of resin with DMF was repeated three more times. Subsequently, a solution of Fmoc-MeAla-OH (0.6 mol/L) and HOAt (0.375 mol/L) in NMP (0.6 mL per 1 solid-phase reaction vessel) and a solution of N,N'-diisopropylcarbodiimide (DIC) in DMF (10 v/v%, 0.72 mL per 1 solid-phase reaction vessel) were mixed by a mixing vial of the synthesizer, then added to all 30 solid-phase reaction vessels and the vessels were allowed to stand still at 40°C for 2.5 hours. The solution was then discharged from the frit. To all 30 solid-phase reaction vessels, DMF was added (1.4 mL per 1 solid-phase reaction vessel), and the solvent was discharged from the frit. This washing step of resin was repeated two more times.

Extension of Fmoc-Ile-OH

A solution of diazabicycloundecene (DBU) in DMF (2 v/v%, 1.4 mL per 1 solid-phase reaction vessel) was added to all 30 solid-phase reaction vessels containing the resin obtained above, and the vessels were warmed to 35°C, and after 10 minutes, the solution was discharged from the frit. To all 30 solid-phase reaction vessels, DMF was added (1.4 mL per 1 solid-phase reaction vessel), and the solvent was discharged from the frit. This washing step of resin with DMF was repeated three more times. Subsequently, a solution of Fmoc-Ile-OH (0.6 mol/L) and HOAt (0.375 mol/L) in NMP (0.6 mL per 1 solid-phase reaction vessel) and a solution of N,N'-diisopropylcarbodiimide (DIC) in DMF (10 v/v%, 0.72 mL per 1 solid-phase reaction vessel) were mixed by a mixing vial of the synthesizer, then added to all 30 solid-phase reaction vessels and the vessels were allowed to stand still at 40°C for 10 hours. The solution was then discharged from the frit. To all 30 solid-phase reaction vessels, DMF was added (1.4 mL per 1 solid-phase reaction vessel), and the solvent was discharged from the frit. This washing step of resin was repeated two more times. Subsequently, dichloromethane (DCM) was added to all 30 solid-phase reaction vessels (1.6 mL per 1 solid-phase reaction vessel), and the solvent was discharged from the frit. This washing step of resin was repeated five more times. Resins were collected from all 30 solid-phase reaction vessels, and mixed, then subjected to the following procedures.

Extension of Fmoc-MeLeu-OH (CAS number: 103478-62-2)

The resin obtained above was added to a 200 mL plastic solid-phase reaction vessel, and DCM (60 mL) was added thereto. The vessel was shaken at 30°C for 5 minutes, then the solvent was discharged from the frit. Toluene (50

mL) was added to this solid-phase reaction vessel, and after shaking at 30°C for 5 minutes, the solvent was discharged from the frit. This washing step of resin with toluene was repeated one more time. A solution of diazabicycloundecene (DBU) in toluene (2 v/v%, 45 mL) was added to this solid-phase reaction vessel, and after shaking at 30°C for 5 minutes, the solution was discharged from the frit.

Toluene (50 mL) was added to this solid-phase reaction vessel, and after shaking at 30°C for 5 minutes, the solvent was discharged from the frit. This washing step of resin with toluene was repeated one more time. DCM (50 mL) was added to this solid-phase reaction vessel, and after shaking at 30°C for 5 minutes, the solvent was discharged from the frit. This washing step of resin with DCM was repeated one more time. A solution of Fmoc-MeLeu-OH (4.25 g, 11.57 mmol), [ethylcyano(hydroxyimino)acetat-O2]tri-1-pyrrolidinylphosphonium hexafluorophosphate (PyOxym) (6.10 g, 11.57 mmol), DIPEA (3.03 mL, 17.35 mmol) in DCM (45 mL) was added to this solid-phase reaction vessel, and the vessel was shaken at 30°C for 3 hours. The solution was then discharged from the frit. DMF (50 mL) was added to this solid-phase reaction vessel, and after shaking at 30°C for 5 minutes, the solvent was discharged from the frit. This washing step of resin with DMF was repeated four more times. DCM (50 mL) was added to this solid-phase reaction vessel, and after shaking at 30°C for 5 minutes, the solvent was discharged from the frit. This washing step of resin with DCM was repeated three more times. The obtained resin was then dried under reduced pressure.

DCM (60 mL) was added to the solid-phase reaction vessel, and after shaking at 30°C for 5 minutes, the solvent was discharged from the frit. DMF (50 mL) was added to this solid-phase reaction vessel, and after shaking at 30°C for 5 minutes, the solvent was discharged from the frit. This washing step of resin with DMF was repeated one more time. A solution of diazabicycloundecene (DBU) in DMF (2 v/v%, 45 mL) was added to this solid-phase reaction vessel, and after shaking at 30°C for 15 minutes, the solution was discharged from the frit. DMF (50 mL) was added to this solid-phase reaction vessel, and after shaking at 30°C for 5 minutes, the solvent was discharged from the frit. This washing step of resin with DMF was repeated four more times. DCM (50 mL) was added to this solid-phase reaction vessel, and after shaking at 30°C for 5 minutes, the solvent was discharged from the frit. This washing step of resin with DCM was repeated four more times to obtain a resin supporting Compound 1217-d.

Synthesis of Compound 1217-d (excision of peptide from resin)

To the solid-phase reaction vessel containing resin obtained above, a mixed solution of 2,2,2-trifluoroethanol (TFE) (60 mL), DCM (60 mL) and DIPEA (0.909 mL) was added, and the vessel was shaken at room temperature for 2 hours. The solution was then collected from the frit. To this solid-phase reaction vessel, a mixed solution of 2,2,2-trifluoroethanol (TFE) (30 mL) and DCM (30 mL) was added, and after shaking at room temperature for 5 minutes, the solution was collected from the frit. Furthermore, to this solid-phase reaction vessel, a mixed solution of 2,2,2-trifluoroethanol (TFE) (30 mL) and DCM (30 mL) was added, and after shaking at room temperature for 5 minutes, the solution was collected from the frit. All the collected solutions were mixed and the solvent was distilled off under reduced pressure to obtain Compound 1217-d as a crude product. (3.85 g)

LCMS(ESI) m/z = 1453.9(M-H)-
Retention time: 0.67 minutes (analysis condition SQDAA50)

Synthesis of Compound 1 (cyclization and purification of peptide)

Compound 1217-d (3.85 g) obtained above was dissolved in a mixed solution of isopropyl acetate (529 mL) and DIPEA (0.915 mL, 5.24 mmol). To this, HCTU (O-(1H-6-chlorobenzotriazol-1-yl)-1,1,3,3-tetramethyluronium hexafluorophosphate, CAS No. 330645-87-9) (1.805 g, 4.36 mmol) was added, and the mixture was stirred at room temperature for 21 hours. The solvent was then distilled off under reduced pressure until the solution volume was about half. To the resulting solution, a mixed solution of saturated aqueous ammonium chloride solution (40 mL) and water (40 mL) was added, and the mixture was extracted with isopropyl acetate (350 mL). The obtained organic phase was washed sequentially with a mixed solution of saturated aqueous sodium hydrogen carbonate solution (40 mL) and water (40 mL), and a mixed solution of saturated brine (40 mL) and water (40 mL) and then dried over sodium sulfate, and the solvent was distilled off under reduced pressure to obtain a 3.36 g of residue. The obtained residue was purified by reverse-phase silica gel column chromatography (with Daisogel SP-120-40/60-ODS-RPS, using acetonitrile containing 0.1% formic acid/water containing 0.1% formic acid as eluent) and the eluent containing the compound of interest was lyophilized to obtain Compound 1 in the amorphous state (1.36 g, yield 34%). The values in mass spectrum and retention time in liquid chromatography of the obtained Compound 1 were as follows.

LCMS(ESI) m/z = 1437.7$(M+H)^+$
Retention time: 7.496 minutes (analysis condition SSC-A-AF-01)

Preparation Example 2:

Preparation of seed crystals used in Preparation Example 3

Compound 1 (122.3 mg) in the amorphous state obtained in Preparative Example 1 was dissolved in DMSO (0.612 mL) and the dissolved solution (0.015 mL) was lyophilized at -20°C for 2 days. To the obtained lyophilized product, a water-acetonitrile mixed solution (3:1, 0.015 mL) was added, and the mixture was stirred with shaking at room temperature for 7 days to obtain a hydrate crystal (Form C) of Compound 1.

Preparation Example 3:

Crystallization of Compound 1: synthesis of hydrate crystal (Form C) of "(5S,8S,11S,15S,18S,23aS,29S,35S,37aS)-8-((S)-sec-butyl)-18-cyclopentyl-29-(3,5-difluoro-4-(trifluoromethyl)phenethyl)-36-ethyl-11-isobutyl-N,N,5,6,12,16,19,33-octamethyl-35-(4-methylbenzyl)-4,7,10,13,17,20,23,28,31,34,37-undecaoxotetratriacontahydro-2H,4H-spiro[azeto[2,1-u]pyrrolo[2,1-i][1,4,7,10,13,16,19,22,25,28,31]undecaazacyclotetratriacontin-21,1'-cyclopentane]-15-carboxamide"

A solution containing Compound 1 was added to a reaction tank replaced by nitrogen, and the outer temperature of the reaction tank was set to 40°C, then purified water (10.9 kg) filtered using a filter (CCF-G100-D1N) was added. To the reaction tank, a suspension obtained by adding ground crystals of Compound 1 (10.2 g) obtained by the same procedures as in Preparation Example 2 to a mixed solution of acetone (59.2 g)/water (61.2 g) was added. The suspension was added to the reaction tank while washing the container that contained the suspension with a mixture of acetone (59.2 g)/water (61.2 g), and then stirred for 2 hours and 1 minute. Purified water (2.7 kg) filtered using a filter (CCF-G100-D1N) was added and the mixture was stirred for 7 hours and 10 minutes. Further, a suspension obtained by adding ground crystals of Compound 1 (10.2 g) obtained by the same procedures as in Preparation Example 2 to a mixed solution of acetone (59.2 g)/water (61.2 g) was added to the reaction tank. The suspension was added to the reaction tank while washing the container that contained the suspension with a mixed solution of acetone (59.2 g)/water (61.2 g), and then stirred for 12 hours and 40 minutes. Purified water (2.7 kg) filtered using a filter (CCF-G100-D1N) was added and the mixture was stirred for 2 hours. After cooling the outside temperature of the reaction tank from 40°C to 25°C for 1 hour, the reaction mixture was stirred for 18 hours and 44 minutes. The reaction mixture was filtered under pressure using a filter cloth (PF-020), and the obtained crystal was washed while washing inside of the reaction tank and the filtration device with a mixed solution of acetone (7.5 kg) and purified water (7.5 kg) filtered using a filter (CCF-G100-E1N). The obtained crystals were washed with purified water (17.0 kg × 2) filtered using a filter (CCF-G100-E1N), the filtration device from which the crystals were collected was decompressed and the outside temperature of the filtration device was set to 70°C to dry the crystals for 17 hours. In addition, the crystals were dried at the outside temperature of room temperature to 30°C for 27 hours. The dried powder was collected from the filtration device to obtain a white powder (2.6 kg).

The obtained white powder was confirmed to have the same structure as the compound obtained in "Synthesis of Compound 1 (cyclization and purification of peptide)" of Preparation Example 1.

The retention time was confirmed by the HPLC analysis method shown below.

HPLC analysis conditions

Device: Waters ACQUITY UPLC H-Class
Column: ACQUITY UPLC CSH C18 (Waters), 2.1 mm ID×150 mm, 1.7 μm
Mobile phase: 0.05% TFA/water (A), 0.05% TFA/MeCN (B)
Elution method: B) 20%(0 min)→100%(24 min)→100%(29 min)→20%(29.1 min)→20%(34 min)
Flow rate: 0.3 mL/min
Column temperature: 50°C
Detection wavelength: 220 nm (PDA)
Retention time by HPLC analysis: 18.199 minutes

Powder X-ray analysis was performed using the XRPD device shown below.

XRPD measurement conditions

Measuring device: X' pert-pro MPD (manufactured by PANalytical Ltd.)
Source: CuKα
Tube voltage: 45 kV
Tube current: 40 mA
Scanning range: 3 to 40°
Scanning speed: 4.2°/min
Sampling width: 0.017°

As a result of the measurement, 2θ values were observed at 4.964°, 7.921°, 8.296°, 8.855°, 9.956°, 10.435°, 11.729°, 12.704°, 13.552°, 13.901°, 15.895°, 16.643°, and 17.813° (± 0.2°) as the major peaks. The analysis results are shown in Figure 1.

Thermal gravimetric and differential thermal analysis of the hydrate crystal (Form C) of Compound 1 obtained by the same method as in Preparation Example 3 was measured under the following conditions. The results are shown in Figure 2.

Measuring device: TGA/DSC 3+ (manufactured by Mettler-Toledo International Inc.)
Measurement range: 25 to 350°C
Heating rate: 10°C/ min
Atmosphere: Dry Nitrogen

The moisture content of the hydrate crystal (Form C) of Compound 1 obtained by the same method as in Preparation Example 3 was measured by the Karl Fischer titration method. Measurements were performed using CA-310 (manufactured by Nittoseiko Analytech Co., Ltd.) after the samples were conditioned in a laboratory environment. As a result of the measurement, the moisture content of the hydrate crystal (Form C) of Compound 1 was 6.50 wt%.

A single crystal X-ray structural analysis of the hydrate crystal (Form C) of Compound 1 obtained by the same method as in Preparation Example 3 was performed under the following conditions.

Measuring device: Rigaku R-AXIS RAPID-II with a VariMax Cu diffractometer (manufactured by Rigaku Corporation)
Counter cathode: Cu
Tube voltage: 40 kV
Tube current: 30 mA
Temperature: -180°C

Measurement: Measurements were made using strategy and exposure time that were believed to yield sufficient diffraction spots for structural analysis.

Structural analysis: Initial structural determination was done by the direct method (SIR2004, Crystal Structure, Rigaku Corporation), and structural refinement was done by full-matrix least-squares method (SHELXL-2017/1, APEX3, Bruker). All non-hydrogen atoms were refined with anisotropic temperature factors. The hydrogen atom of the water molecule was placed in an appropriate position using a restraint and refined with an isotropic temperature factor of 1.5 times the size of the attached oxygen atom. The other hydrogen atoms were placed in appropriate positions using the riding model, and refined with an isotropic temperature factor of 1.2 times the size of the attached non-hydrogen atom. The results are shown in Figure 3.

From the results of thermogravimetric and differential thermal analysis, Karl Fischer titration, and single crystal X-ray structural analysis, it was confirmed that the hydrate crystal (Form C) of Compound 1 was exactly a hydrate crystal having a water molecule in the crystal structure.

Dynamic moisture vapor adsorption measurement of the hydrate crystal (Form C) of Compound 1 obtained by the same method as in Preparation Example 3 was performed. The results are shown in Figure 4.

Measuring device: DVS Intrinsic (manufactured by Surface Measurement Systems Ltd.)
Temperature: 25°C
Relative humidity (%) measurement point:

Cycle 1: 0, 10, 20, 30, 40, 50, 60, 70, 80, 90, 95, 90, 80, 70, 60, 50, 40, 30, 20, 10, 0 (%);
Cycle 2: 10, 20, 30, 40, 50, 60, 70, 80, 90, 95, 90, 80, 70, 60, 50, 40, 30, 20, 10, 0 (%);

Threshold: 0.001 dm/dt (%/min)
Minimum sorption temperature: 10 minutes
Maximum sorption temperature: 1440 minutes

As a result of the measurement, it was confirmed that the hydrate crystal (Form C) of Compound 1 was a hydrate crystal that varies by 3.3% by weight depending on changes in hydration number at the relative humidity ranging from 0 to 95%.

The hydrate crystal (Form C) of Compound 1 obtained in Preparation Example 3 (hereinafter, sometimes referred to as "Compound I") was used in the preparation and assessment of the following composition or formulation. Hereinafter, the compound that is the active ingredient in Examples is described as "Compound I". Compound I dissolved in the liquid additive or the like means Compound 1 in the free form.

Search of composition containing Compound I

Test Example 1 (Solubility assessment of Compound I in various additives)

The solubility of Compound I in various additives was determined. Various additives in liquid form were added to Compound I and the mixture was stirred. When Compound I did not dissolve in an additive, the additive was further added and the mixture was stirred. This procedure was repeated until Compound I was dissolved, and the approximate solubility was determined from the amount of the additive required to dissolve Compound I. Since polyoxyethylene (15) hydroxystearate (Solutol HS15) was solid at room temperature (R.T.), it was heated to 37°C to be a liquid, then the solubility was determined. The solubility of the other additives was determined at room temperature. The results are shown in Table 2. As a result, it was found that Compound I has high solubility in PG monocaprylate.

[Table 2]

Solubility of Compound I in additive

| Additive | **Temperature** | **Solubility** (mg/mL) |
|---|---|---|
| MCT (Miglyol 810) | R. T. | <20 |
| Triacetin | R. T. | 100-150 |
| Oleic acid (Extra olein 99, Extra 0S-85) | R. T. | 70-80 |
| PG monocaprylate (Sefsol-218, Capryol 90) | R.T. | >200 |
| Glycerol monooleate (Peceol) | R. T. | <20 |
| Glyceryl monolinoleate (Maisine 35-1) | R. T. | <20 |
| Sorbitan trioleate (S0-30V) | R. T. | < 20 |
| Polyoxyl 35 castor oi (Kolliphor EL) | | < 20 |
| Polyoxyethylene (15) hydroxystearate (Solutol HS15) | 37° C | 40-60 |
| PEG-8 Capryl ic/Capric Glycerides (Labrasol) | R.T. | 80-100 |
| Polysorbate 80 | R. T. | 80-100 |
| Et0H R. T. | | >200 |
| PG | R. T. | >200 |
| PEG400 | R. T. | >200 |

Test Example 2 (Solubility assessment of Compound I in each formulation)

Using PG monocaprylate that was shown as high solubility, additive mixed solutions were prepared based on the prescription in Table 3. The solubility of Compound I in each additive mixed solution 1 to 4 was then measured. Considering the moisture transition from the capsule coating when prepared as a capsule formulation, the solubility for the additive mixed solution with addition of 3% water was also measured. As a result, it was found as shown in Table 4 that the additive mixed solutions 1 to 4 can dissolve Compound I at high concentration of 100 mg/mL or more. Since the solubility of Compound I decreased to 60 to 70 mg/mL when water was added to each additive mixed solution, the subsequent solubility assessments of Compound I in each additive mixed solution were performed with addition of water.

[Table 3]

Prescription of additive mixed solutions 1 to 4 (% by weight)

| | 1 | 2 | 3 | 4 |
|---|---|---|---|---|
| Propy I ene glycol monocaprylate | 53.0 | 29.3 | 53.3 | 29.4 |
| Oleic acid | 13.8 | 37.4 | 13.9 | 37.5 |
| Polyoxyl 35 castor oil | 16.6 | 27.7 | 31.8 | 32.1 |
| D-α-Tocopherol polyethylene glycol 1000 succinate | 16.6 | 5.6 | 0.0 | 0.0 |
| dl-α-tocopherol | 0.0 | 0.0 | 1.0 | 1.0 |

[Table 4]

Solubility of Compound I in additive mixed solutions 1 to 4

| Additive mixed solution | Temperature | Solubility (mq/mL) |
|---|---|---|
| Additive mixed solution 1 | R. T. | > 100 |
| Additive mixed solution 2 | R. T. | > 100 |
| Additive mixed solution 3 | R. T. | > 100 |
| Additive mixed solution 4 | R. T. | > 100 |
| Additive mixed solution 1 / Water (100/3 v/v) | R. T. | 60-70 |
| Additive mixed solution 2 / Water (100/3 v/v) | R. T. | 60-70 |
| Additive mixed solution 3 / Water (100/3 v/v) | R. T. | 60-70 |
| Additive mixed solution 4 / Water (100/3 v/v) | R. T. | 60-70 |

Further, additive mixed solutions 5 to 45 were prepared based on the prescription in Tables 5 to 10 in order to search for an optimal prescription.

[Table 5]

Prescription of additive mixed solutions 5 to 11 (% by weight)

| | 5 | 6 | 7 | 8 | 9 | 10 | 11 |
|---|---|---|---|---|---|---|---|
| Propylene glycol monocaprylate | 56.7 | 61. 8 | 67.0 | 45.7 | 50. 8 | 56. 1 | 61.5 |
| Tr iacetin | 0.0 | 0.0 | 0.0 | 22.8 | 17. 3 | 11. 7 | 5.9 |
| Polyoxyl 35 castor oil | 27.0 | 21.8 | 16.5 | 15.8 | 15.9 | 16.1 | 16.3 |
| D-α-Tocopherol polyethylene glycol 1000 succinate | 16.3 | 16.4 | 16.5 | 15.8 | 15.9 | 16.1 | 16.3 |

[Table 6]

Prescription of additive mixed solutions 12 to 18 (% by weight)

| | 12 | 13 | 14 | 15 | 16 | 17 | 18 |
|---|---|---|---|---|---|---|---|
| Propylene glycol monocaprylate | 56.4 | 61.5 | 66.8 | 45.5 | 50.7 | 55. 9 | 61.3 |
| Triacetin | 0.0 | 0.0 | 0.0 | 22.7 | 17.2 | 11.6 | 5.9 |
| D-α-Tocpherol polyethylene glycol 1000 succinate | 16.4 | 16.5 | 16.6 | 15.9 | 16.0 | 16.2 | 16.4 |
| Polysorbate 80 | 27.3 | 22.0 | 16.6 | 15.9 | 16.0 | 16.2 | 16.4 |

[Table 7]

Prescription of additive mixed solutions 19 to 27 (% by weight)

| | 19 | 20 | 21 | 22 | 23 | 24 | 25 | 26 | 27 |
|---|---|---|---|---|---|---|---|---|---|
| Propyleneglycol monocaprylate | 51.2 | 56.5 | 67.5 | 50.7 | 56.0 | 66.9 | 56.3 | 56.2 | 56.1 |
| Triacetin | 17.4 | 11.7 | 0.0 | 17.3 | 11.6 | 0.0 | 11.7 | 11.7 | 11.7 |
| Polyoxy 35 castor oil | 30.5 | 30.8 | 31.6 | 0.0 | 0.0 | 0.0 | 20.1 | 14.8 | 9.5 |
| Polysorbate 80 | 0.0 | 0.0 | 0.0 | 31.1 | 31.4 | 32.2 | 10.9 | 16.3 | 21.7 |
| dl-α-tocopherol | 0.9 | 0.9 | 1.0 | 0.9 | 0.9 | 1.0 | 0.9 | 0.9 | 0.9 |

[Table 8]

**Prescription of additive mixed solutions 28 to 31 (% by weight)**

| | 28 | 29 | 30 | 31 |
|---|---|---|---|---|
| Propylene glycol monocaprylate | 56.4 | 56.4 | 56.3 | 56. 2 |
| Triacetin | 11.7 | 11.7 | 11.7 | 11.7 |
| Polyoxyl 35 castor oil | 20.2 | 14.8 | 9.5 | 0.0 |
| PEG-8 Caprylic/Capric Glycerides | 10.8 | 16.1 | 21.5 | 31.1 |
| dl-α-tocopherol | 0.9 | 0.9 | 0.9 | 0.9 |

[Table 9]

Prescription of additive mixed solutions 32 to 39 (% by weight)

| | 32 | 33 | 34 | 35 | 3 6 | 37 | 38 | 39 |
|---|---|---|---|---|---|---|---|---|
| Propylene glycol monocaprylate | 52.9 | 47.9 | 51.5 | 46.6 | 56.6 | 51.6 | 61.6 | 51.0 |
| Oleic acid | 9. 2 | 9.2 | 0.0 | 0.0 | 0.0 | 0.0 | 0.0 | 0.0 |
| Triacetin | 0.0 | 0.0 | 11.7 | 11. 6 | 5.9 | 5.9 | 5.9 | 17.3 |
| Polyoxyl 35 castor oil | 31.5 | 31.3 | 30.7 | 30.5 | 0.0 | 0.0 | 0.0 | 0.0 |
| PEG-8 Caprylic/Capric Glycerides | 0.0 | 0.0 | 0.0 | 0.0 | 31.3 | 31.2 | 31.5 | 30.8 |
| Propylene glycol | 5.4 | 10.7 | 5.2 | 10.4 | 5.3 | 10.5 | 0.0 | 0.0 |
| dt-α-tocopherot | 1.0 | 10 | 0.9 | 0.9 | 0.9 | 0.9 | 0.9 | 0.9 |

[Table 10]

Prescription of additive mixed solutions 40 to 55 (% by weight)

| | 40 | 41 | 42 | 43 | 44 | 45 |
|---|---|---|---|---|---|---|
| Propylene glycol monocaprylate | 48.5 | 39.0 | 29.4 | 19.7 | 48.2 | 53.3 |
| Oleic acid | 18. 6 | 28.0 | 37.5 | 47.0 | 18.5 | 13.9 |
| Polyoxyl 35 castor | 32.9 | 33.0 | 33.2 | 33.3 | 16.6 | 32.8 |
| D-α-Tocopherol polyethylene glycol 1000 succinate | 0.0 | 0.0 | 0.0 | 0.0 | 16.6 | 0.0 |

The solubility of Compound I in each formulation 5 to 45 with addition of water was then measured. The results are shown in Table 11.

[Table 11]

Solubility of Compound I in additive mixed solutions 5 to 45

| Additive mixed solution | Temperature | Solubility (mg/mL) |
|---|---|---|
| Additive mixed solution 5 / Water (100/3 v/v) | R. T. | 40-50 |
| Additive mixed solution 6 / Water (100/3 v/v) | R. T. | 50-60 |
| Additive mixed solution 7 / Water (100/3 v/v) | R. T. | 60-70 |
| Additive mixed solution 8 / Water (100/3 v/v) | R. T. | 60-70 |
| Additive mixed solution 9 / Water (100/3 v/v) | R. T. | 70-80 |
| Additive mixed solution 10 / Water (100/3 v/v) | R. T. | 70-80 |
| Additive mixed solution 11 / Water (100/3 v/v) | R. T. | 50-60 |
| Additive mixed solution 12 / Water (100/3 v/v) | R. T. | 50-60 |
| Additive mixed solution 131 Water (100/3 v/v) | R. T. | 70-80 |
| Additive mixed solution 141 Water (100/3 v/v) | R. T. | 70-80 |
| Additive mixed solution 151 Water (100/3 v/v) | R. T. | 70-80 |
| Additive mixed solution 161 Water (100/3 v/v) | R. T. | 70-80 |
| Additive mixed solution 17 / Water (100/3 v/v) | R. T. | 70-80 |
| Additive mixed solution 18 / Water (100/3 v/v) | R. T. | 70-80 |
| Additive mixed solution 19 / Water (100/3 v/v) | R. T. | 70-80 |
| Additive mixed solution 20 / Water (100/3 v/v) | R. T. | 60-70 |
| Additive mixed solution 21 / Water (100/3 v/v) | R. T. | 70-80 |
| Additive mixed solution 22 / Water (100/3 v/v) | R. T. | 70-80 |
| Additive mixed solution 23 / Water (100/3 v/v) | R. T. | 80-100 |
| Additive mixed solution 24 / Water (100/3 v/v) | R. T. | 70-80 |
| Additive mixed solution 25 / Water (100/3 v/v) | R. T. | 70-80 |
| Additive mixed solution 26 / Water (100/3 v/v) | R. T. | 80-100 |
| Additive mixed solution 27 / Water (100/3 v/v) | R. T. | 70-80 |
| Additive mixed solution 28 / Water (100/3 v/v) | R. T. | 80-90 |
| Additive mixed solution 29 / Water (100/3 v/v) | R. T. | 90-100 |
| Additive mixed solution 30 / Water (100/3 v/v) | R. T. | 90-100 |
| Additive mixed solution 31 / Water (100/3 v/v) | R. T. | > 100 |
| Additive mixed solution 32 / Water (100/3 v/v) | R. T. | < 60 |
| Additive mixed solution 33 / Water (100/3 v/v) | R. T. | < 60 |
| Additive mixed solution 34 / Water (100/3 v/v) | R. T. | < 60 |
| Additive mixed solution 35 / Water (100/3 v/v) | R. T. | < 60 |
| Additive mixed solution 36 / Water (100/3 v/v) | R. T. | < 60 |
| Additive mixed solution 37 / Water (100/3 v/v) | R. T. | < 60 |
| Additive mixed solution 38 / Water (100/3 v/v) | R. T. | 100-125 |
| Additive mixed solution 39 / Water (100/3 v/v) | R. T. | 100-125 |
| Additive mixed solution 40 / Water (100/3 v/v) | R. T. | 60-70 |
| Additive mixed solution 41 / Water (100/3 v/v) | R. T. | 60-70 |
| Additive mixed solution 42 / Water (100/3 v/v) | R. T. | 60-70 |
| Additive mixed solution 43 / Water (100/3 v/v) | R. T. | 60-70 |
| Additive mixed solution 44 / Water (100/3 v/v) | R. T. | 60-70 |
| Additive mixed solution 45 / Water (100/3 v/v) | R. T. | 60-70 |

Examples 1 to 22

Based on the prescription in Table 12, formulations A to V, which are the compositions of the present invention, were prepared by the following methods.

[Table 12]

Prescription of formulations A to V

| Formulation name | | Composition | |
|---|---|---|---|
| Example 1 | A | Compound I 50 mg | Additive mixed solution 1 1 mL |
| Example 2 | B | Compound I 50 mg | Additive mixed solution 2 1 mL |
| Example 3 | C | Compound I 50 mg | Additive mixed solution 3 1 mL |
| Example 4 | D | Compound I 50 mg | Additive mixed solution 4 1 mL |
| Example 5 | E | Compound I 50 mg | Additive mixed solution 7 1 mL |
| Example 6 | F | Compound I 50 mg | Additive mixed solution 19 1 mL |
| Example 7 | G | Compound I 50 mg | Additive mixed solution 20 1 mL |
| Example 8 | H | Compound I 50 mg | Additive mixed solution 21 1 mL |
| Example 9 | I | Compound I 50 mg | Additive mixed solution 25 1 mL |
| Example 10 | J | Compound I 50 mg | Additive mixed solution 26 1 mL |
| Example 11 | K | Compound I 50 mg | Additive mixed solution 28 1 mL |
| Example 12 | L | Compound I 50 mg | Additive mixed solution 29 1 mL |
| Example 13 | M | Compound I 50 mg | Additive mixed solution 30 1 mL |
| Example 14 | N | Compound I 50 mg | Additive mixed solution 32 1 mL |
| Example 15 | O | Compound I 50 mg | Additive mixed solution 33 1 mL |
| Example 16 | P | Compound I 50 mg | Additive mixed solution 34 1 mL |
| Example 17 | Q | Compound I 50 mg | Additive mixed solution 35 1 mL |
| Example 18 | R | Compound I 50 mg | Additive mixed solution 40 1 mL |
| Example 19 | S | Compound I 50 mg | Additive mixed solution 41 1 mL |
| Example 20 | T | Compound I 50 mg | Additive mixed solution 42 1 mL |
| Example 21 | U | Compound I 50 mg | Additive mixed solution 44 1 mL |
| Example 22 | V | Compound I 50 mg | Additive mixed solution 45 1 mL |

Example 1 (Preparation of formulation A)

Compound I was weighed into a vial, and 1 mL of additive mixed solution 1 per 50 mg of Compound I was added. The mixture was stirred with a stirrer, and Compound I was confirmed to be completely dissolved to obtain formulation A.

Example 2 (Preparation of formulation B)

Compound I was weighed into a vial, and 1 mL of additive mixed solution 2 per 50 mg of Compound I was added. The mixture was stirred with a stirrer, and Compound I was confirmed to be completely dissolved to obtain formulation B.

Example 3 (Preparation of formulation C)

Compound I was weighed into a vial, and 1 mL of additive mixed solution 3 per 50 mg of Compound I was added. The mixture was stirred with a stirrer, and Compound I was confirmed to be completely dissolved to obtain formulation C.

Example 4 (Preparation of formulation D)

Compound I was weighed into a vial, and 1 mL of additive mixed solution 4 per 50 mg of Compound I was added. The mixture was stirred with a stirrer, and Compound I was confirmed to be completely dissolved to obtain formulation D.

Example 5 (Preparation of formulation E)

Compound I was weighed into a vial, and 1 mL of additive mixed solution 7 per 50 mg of Compound I was added. The mixture was stirred with a stirrer, and Compound I was confirmed to be completely dissolved to obtain

formulation E.

Example 6 (Preparation of formulation F)

Compound I was weighed into a vial, and 1 mL of additive mixed solution 19 per 50 mg of Compound I was added. The mixture was stirred with a stirrer, and Compound I was confirmed to be completely dissolved to obtain formulation F.

Example 7 (Preparation of formulation G)

Compound I was weighed into a vial, and 1 mL of additive mixed solution 20 per 50 mg of Compound I was added. The mixture was stirred with a stirrer, and Compound I was confirmed to be completely dissolved to obtain formulation G.

Example 8 (Preparation of formulation H)

Compound I was weighed into a vial, and 1 mL of additive mixed solution 21 per 50 mg of Compound I was added. The mixture was stirred with a stirrer, and Compound I was confirmed to be completely dissolved to obtain formulation H.

Example 9 (Preparation of formulation I)

Compound I was weighed into a vial, and 1 mL of additive mixed solution 25 per 50 mg of Compound I was added. The mixture was stirred with a stirrer, and Compound I was confirmed to be completely dissolved to obtain formulation I.

Example 10 (Preparation of formulation J)

Compound I was weighed into a vial, and 1 mL of additive mixed solution 26 per 50 mg of Compound I was added. The mixture was stirred with a stirrer, and Compound I was confirmed to be completely dissolved to obtain formulation J.

Example 11 (Preparation of formulation K)

Compound I was weighed into a vial, and 1 mL of additive mixed solution 28 per 50 mg of Compound I was added. The mixture was stirred with a stirrer, and Compound I was confirmed to be completely dissolved to obtain formulation K.

Example 12 (Preparation of formulation L)

Compound I was weighed into a vial, and 1 mL of additive mixed solution 29 per 50 mg of Compound I was added. The mixture was stirred with a stirrer, and Compound I was confirmed to be completely dissolved to obtain formulation L.

Example 13 (Preparation of formulation M)

Compound I was weighed into a vial, and 1 mL of additive mixed solution 30 per 50 mg of Compound I was added. The mixture was stirred with a stirrer, and Compound I was confirmed to be completely dissolved to obtain formulation M.

Example 14 (Preparation of formulation N)

Compound I was weighed into a vial, and 1 mL of additive mixed solution 32 per 50 mg of Compound I was added. The mixture was stirred with a stirrer, and Compound I was confirmed to be completely dissolved to obtain formulation N.

Example 15 (Preparation of formulation O)

Compound I was weighed into a vial, and 1 mL of additive mixed solution 33 per 50 mg of Compound I was added. The mixture was stirred with a stirrer, and Compound I was confirmed to be completely dissolved to obtain formulation O.

Example 16 (Preparation of formulation P)

Compound I was weighed into a vial, and 1 mL of additive mixed solution 34 per 50 mg of Compound I was added. The mixture was stirred with a stirrer, and Compound I was confirmed to be completely dissolved to obtain formulation P.

Example 17 (Preparation of formulation Q)

Compound I was weighed into a vial, and 1 mL of additive mixed solution 35 per 50 mg of Compound I was added. The mixture was stirred with a stirrer, and Compound I was confirmed to be completely dissolved to obtain formulation Q.

Example 18 (Preparation of formulation R)

Compound I was weighed into a vial, and 1 mL of additive mixed solution 40 per 50 mg of Compound I was added. The mixture was stirred with a stirrer, and Compound I was confirmed to be completely dissolved to obtain formulation R.

Example 19 (Preparation of formulation S)

Compound I was weighed into a vial, and 1 mL of additive mixed solution 41 per 50 mg of Compound I was added. The mixture was stirred with a stirrer, and Compound I was confirmed to be completely dissolved to obtain formulation S.

Example 20 (Preparation of formulation T)

Compound I was weighed into a vial, and 1 mL of additive mixed solution 42 per 50 mg of Compound I was added. The mixture was stirred with a stirrer, and Compound I was confirmed to be completely dissolved to obtain formulation T.

Example 21 (Preparation of formulation U)

Compound I was weighed into a vial, and 1 mL of additive mixed solution 44 per 50 mg of Compound I was added. The mixture was stirred with a stirrer, and Compound I was confirmed to be completely dissolved to obtain formulation U.

Example 22 (Preparation of formulation V)

Compound I was weighed into a vial, and 1 mL of additive mixed solution 45 per 50 mg of Compound I was added. The mixture was stirred with a stirrer, and Compound I was confirmed to be completely dissolved to obtain formulation V.

The % by weight of each ingredient contained in the formulations of Examples 1 to 22 based on the entire of the formulation was summarized in the table below.

[Table 13]

| Example No. | Example 1 | Example 2 | Example 3 | Example 4 | Example 5 | Example 6 | Example 7 | Example 8 | Example 9 | Example 10 | Example 11 |
|---|---|---|---|---|---|---|---|---|---|---|---|
| Formulation name | A | B | C | D | E | F | G | H | I | J | K |
| Compound I | 4.9 | 5.0 | 5.0 | 5.0 | 4.9 | 4.8 | 4.8 | 4.9 | 4.8 | 4.8 | 4.8 |
| Propylene glycol monocaprylate | 50.3 | 27.8 | 50 7 | 27 9 | 63.7 | 48.7 | 53.8 | 64.2 | 53.6 | 53.5 | 53.7 |
| Oleic acid | 13.1 | 35.5 | 13.2 | 35.6 | | | | | | | |
| Triacetin | | | | | | 16.6 | 11.2 | | 11.1 | 11.1 | 11.2 |
| Polyoxyl 35 castor oil | 15.8 | 26.3 | 30.2 | 30 5 | 15.7 | 29.0 | 29.3 | 30.0 | 19.2 | 14.1 | 192 |
| D-α-Tocopherol polyethylene glycol 1000 succinate | 15.8 | 5.3 | | | 15.7 | | | | | | |
| PEG-8 Caprylic/Capric Glycerides | | | | | | | | | | | 102 |
| Polysorbate 80 | | | | | | | | | 10.4 | 15.5 | |
| Propylene glycol | | | | | | | | | | | |
| dl-α-tocopherol | | | 0.9 | 0.9 | | 0.9 | 0.9 | 0.9 | 0.9 | 0.9 | 0.9 |
| Total | 100 | 100 | 100 | 100 | 100 | 100 | 100 | 100 | 100 | 100 | 100 |
| Example No. | Example 12 | Example 13 | Example 14 | Example 15 | Example 16 | Example 17 | Example 18 | Example 19 | Example 20 | Example 21 | Example 22 |
| Formulation name | L | M | N | O | P | Q | R | S | T | U | V |
| Compound I | 4.8 | 4.8 | 49 | 49 | 4.8 | 48 | 5.0 | 5.0 | 50 | 49 | 50 |
| Propylene glycol monocaprylate | 53.7 | 53.6 | 50.3 | 45 5 | 49.0 | 44.4 | 46.1 | 37.1 | 27.9 | 45.9 | 50.6 |
| Oleic acid | | | 8.8 | 8.7 | | | 11.7 | 26.6 | 35.6 | 17.6 | 13.2 |
| Triacetin | 11.2 | 11.1 | | | 11.1 | 11.1 | | | | | |
| Polyoxyl 35 castor oil | 14.1 | 91 | 30.0 | 29.8 | 29.2 | 29.0 | 31.3 | 31.4 | 31.5 | 15.8 | 31.2 |

(continued)

| Example No. | Example 12 | Example 13 | Example 14 | Example 15 | Example 16 | Example 17 | Example 18 | Example 19 | Example 20 | Example 21 | Example 22 |
|---|---|---|---|---|---|---|---|---|---|---|---|
| D-α-Tocopherol polyethylene glycol 1000 succinate | | | | | | | | | | 15.8 | |
| PEG-8 CaprylicICapric Glycerides | 15.4 | 20.5 | | | | | | | | | |
| Polysorbate 80 | | | | | | | | | | | |
| Propylene glycol | | | 5.1 | 10.1 | 5.0 | 99 | | | | | |
| dl-α-tocopherol | 0.9 | 09 | 0.9 | 0.9 | 0.9 | 0.9 | | | | | |
| Total | 100 | 100 | 100 | 100 | 100 | 100 | 100 | 100 | 100 | 100 | 100 |

Comparative Examples 1 to 19

Based on the prescription in Table 14, formulations W to AO, which are compositions of the present invention, were prepared by the following methods.

[Table 14]

Prescription of formulations W to AO

| | Formulation name | Composition |
|---|---|---|
| Comparative Example 1 | W | Compound I 50 mg Additive mixed solution 8 1 mL |
| Comparative Example 2 | X | Compound I 50 mg Additive mixed solution 9 1 mL |
| Comparative Example 3 | Y | Compound I 50 mg Additive mixed solution 10 1 mL |
| Comparative Example 4 | Z | Compound I 50 mg Additive mixed solution 13 1 mL |
| Comparative Example 5 | AA | Compound I 50 mg Additive mixed solution 14 1 mL |
| Comparative Example 6 | AB | Compound I 50 mg Additive mixed solution 15 1 mL |
| Comparative Example 7 | AC | Compound I 50 mg Additive mixed solution 16 1 mL |
| Comparative Example 8 | AD | Compound I 50 mg Additive mixed solution 17 1 mL |
| Comparative Example 9 | AE | Compound I 50 mg Additive mixed solution 18 1 mL |
| Comparative Example 10 | AF | Compound I 50 mg Additive mixed solution 22 1 mL |
| Comparative Example 11 | AG | Compound I 50 mg Additive mixed solution 23 1 mL |
| Comparative Example 12 | AH | Compound I 50 mg Additive mixed solution 24 1 mL |
| Comparative Example 13 | AI | Compound I 50 mg Additive mixed solution 27 1 mL |
| Comparative Example 14 | AJ | Compound 150 mg Additive mixed solution 31 1 mL |
| Comparative Example 15 | AK | Compound I 50 mg Additive mixed solution 36 1 mL |
| Comparative Example 16 | AL | Compound I 50 mg Additive mixed solution 37 1 mL |
| Comparative Example 17 | AM | Compound I 50 mg Additive mixed solution 38 1 mL |
| Comparative Example 18 | AN | Compound I 50 mg Additive mixed solution 39 1 mL |
| Comparative Example 19 | AO | Compound I 50 mg Additive mixed solution 43 1 mL |

Comparative Example 1 (Preparation of formulation W)

Compound I was weighed into a vial, and 1 mL of additive mixed solution 8 per 50 mg of Compound I was added. The mixture was stirred with a stirrer, and Compound I was confirmed to be completely dissolved to obtain formulation W.

Comparative Example 2 (Preparation of formulation X)

Compound I was weighed into a vial, and 1 mL of additive mixed solution 9 per 50 mg of Compound I was added. The mixture was stirred with a stirrer, and Compound I was confirmed to be completely dissolved to obtain formulation X.

Comparative Example 3 (Preparation of formulation Y)

Compound I was weighed into a vial, and 1 mL of additive mixed solution 10 per 50 mg of Compound I was added. The mixture was stirred with a stirrer, and Compound I was confirmed to be completely dissolved to obtain formulation Y.

Comparative Example 4 (Preparation of formulation Z)

Compound I was weighed into a vial, and 1 mL of additive mixed solution 13 per 50 mg of Compound I was added. The mixture was stirred with a stirrer, and Compound I was confirmed to be completely dissolved to obtain formulation Z.

Comparative Example 5 (Preparation of formulation AA)

Compound I was weighed into a vial, and 1 mL of additive mixed solution 14 per 50 mg of Compound I was added. The mixture was stirred with a stirrer, and Compound I was confirmed to be completely dissolved to obtain formulation AA.

Comparative Example 6 (Preparation of formulation AB)

Compound I was weighed into a vial, and 1 mL of additive mixed solution 15 per 50 mg of Compound I was added. The mixture was stirred with a stirrer, and Compound I was confirmed to be completely dissolved to obtain formulation AB.

Comparative Example 7 (Preparation of formulation AC)

Compound I was weighed into a vial, and 1 mL of additive mixed solution 16 per 50 mg of Compound I was added. The mixture was stirred with a stirrer, and Compound I was confirmed to be completely dissolved to obtain formulation AC.

Comparative Example 8 (Preparation of formulation AD)

Compound I was weighed into a vial, and 1 mL of additive mixed solution 17 per 50 mg of Compound I was added. The mixture was stirred with a stirrer, and Compound I was confirmed to be completely dissolved to obtain formulation AD.

Comparative Example 9 (Preparation of formulation AE)

Compound I was weighed into a vial, and 1 mL of additive mixed solution 18 per 50 mg of Compound I was added. The mixture was stirred with a stirrer, and Compound I was confirmed to be completely dissolved to obtain formulation AE.

Comparative Example 10 (Preparation of formulation AF)

Compound I was weighed into a vial, and 1 mL of additive mixed solution 22 per 50 mg of Compound I was added. The mixture was stirred with a stirrer, and Compound I was confirmed to be completely dissolved to obtain formulation AF.

Comparative Example 11 (Preparation of formulation AG)

Compound I was weighed into a vial, and 1 mL of additive mixed solution 23 per 50 mg of Compound I was added. The mixture was stirred with a stirrer, and Compound I was confirmed to be completely dissolved to obtain formulation AG.

Comparative Example 12 (Preparation of formulation AH)

Compound I was weighed into a vial, and 1 mL of additive mixed solution 24 per 50 mg of Compound I was added. The mixture was stirred with a stirrer, and Compound I was confirmed to be completely dissolved to obtain formulation AH.

Comparative Example 13 (Preparation of formulation AI)

Compound I was weighed into a vial, and 1 mL of additive mixed solution 27 per 50 mg of Compound I was added. The mixture was stirred with a stirrer, and Compound I was confirmed to be completely dissolved to obtain formulation AI.

Comparative Example 14 (Preparation of formulation AJ)

Compound I was weighed into a vial, and 1 mL of additive mixed solution 31 per 50 mg of Compound I was added. The mixture was stirred with a stirrer, and Compound I was confirmed to be completely dissolved to obtain

formulation AJ.

Comparative Example 15 (Preparation of formulation AK)

Compound I was weighed into a vial, and 1 mL of additive mixed solution 36 per 50 mg of Compound I was added. The mixture was stirred with a stirrer, and Compound I was confirmed to be completely dissolved to obtain formulation AK.

Comparative Example 16 (Preparation of formulation AL)

Compound I was weighed into a vial, and 1 mL of additive mixed solution 37 per 50 mg of Compound I was added. The mixture was stirred with a stirrer, and Compound I was confirmed to be completely dissolved to obtain formulation AL.

Comparative Example 17 (Preparation of formulation AM)

Compound I was weighed into a vial, and 1 mL of additive mixed solution 38 per 50 mg of Compound I was added. The mixture was stirred with a stirrer, and Compound I was confirmed to be completely dissolved to obtain formulation AM.

Comparative Example 18 (Preparation of formulation AN)

Compound I was weighed into a vial, and 1 mL of additive mixed solution 39 per 50 mg of Compound I was added. The mixture was stirred with a stirrer, and Compound I was confirmed to be completely dissolved to obtain formulation AN.

Comparative Example 19 (Preparation of formulation AO)

Compound I was weighed into a vial, and 1 mL of additive mixed solution 43 per 50 mg of Compound I was added. The mixture was stirred with a stirrer, and Compound I was confirmed to be completely dissolved to obtain formulation AO.

The % by weight of each ingredient contained in the formulations of Comparative Examples 1 to 19 based on the entire of the formulation was summarized in the table below.

[Table 15]

| Comparative Example No. | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 |
|---|---|---|---|---|---|---|---|---|---|---|
| Formulation name | W | x | Y | z | AA | AB | AC | AD | AE | AF |
| Compound I | 4.7 | 4.7 | 4.8 | 4.8 | 4.9 | 4.7 | 4.7 | 4.8 | 4.8 | 4.7 |
| Propylene glycol monocaprylate | 43.5 | 48.4 | 53.4 | 58.5 | 63.5 | 43.4 | 48.3 | 53.2 | 58.3 | 48.3 |
| Oleic acid | | | | | | | | | | |
| Triacetin | 21.7 | 16.5 | 11.1 | | | 21.7 | 16.4 | 11.1 | 5.6 | 16.4 |
| Polyoxyl 35 castor oil | 15.0 | 15.2 | 15.4 | | | | | | | |
| D-α-Tocopherol polyethylene glycol 1000 succinate | 150 | 15.2 | 15.4 | 15.7 | 15.8 | 15.1 | 15.3 | 15.5 | 15.6 | |
| PEG-8 Caprylic/Capric Glycerides | | | | | | | | | | |
| Polysorbate 80 | | | | 20.9 | 15.8 | 15.1 | 15.3 | 15.5 | 15.6 | 29.6 |
| Propylene glycol | | | | | | | | | | |
| di-α-tocopherol | | | | | | | | | | 0.9 |
| Total | 100 | 100 | 100 | 100 | 100 | 100 | 100 | 100 | 100 | 100 |
| **Comparative Example No.** | **Comparative Example 11** | **Comparative Example 12** | **Comparative Example 13** | **Comparative Example 14** | **Comparative Example 15** | **Comparative Example 16** | **Comparative Example 17** | **Comparative Example 18** | **Comparative Example 19** | |
| Formulation name | AG | AH | AI | AJ | AK | AL | AM | AN | AO | |
| Compound I | 4.8 | 4.9 | 4.8 | 4.8 | 4.8 | 4.8 | 4.9 | 4.7 | 5.0 | |

(continued)

| Comparative Example No. | Comparative Example 11 | Comparative Example 12 | Comparative Example 13 | Comparative Example 14 | Comparative Example 15 | Comparative Example 16 | Comparative Example 17 | Comparative Example 18 | Comparative Example 19 |
|---|---|---|---|---|---|---|---|---|---|
| Propylene glycol monocaprylate | 53.3 | 63.6 | 53.5 | 53.5 | 53.9 | 49.1 | 58.7 | 48.5 | 18.7 |
| Oleic acid | | | | | | | | | 44.7 |
| Triacetin | 11.1 | | 11.1 | 11.1 | 5.6 | 56 | 5.6 | 16.5 | |
| Polyoxyl 35 castor oil | | | 9.1 | 0.0 | | | | | 31.6 |
| D-α-Tocopherol polyethylene glycol 1000 succinate | | | | | | | | | |
| PEG-8 Caprylic/Capric Glycerides | | | | 29.6 | 29.8 | 29.7 | 30.0 | 29.3 | |
| Polysorbate 80 | 29.9 | 30.6 | 20.1 | | | | | | |
| Propylene glycol | | | | | 5.0 | 9.9 | | | |
| dl-α-tocopherol | 0.9 | 0.9 | 0.9 | 0.9 | 0.9 | 0.9 | 0.9 | 0.9 | |
| Total | 100 | 100 | 100 | 100 | 100 | 100 | 100 | 100 | 100 |

Test Example 3: Particle size distribution measurement by DLS

Next, water was added to formulations A to V prepared in Examples 1 to 22 and formulations W to AO prepared in Comparative Examples 1 to 19 to generate dispersions. The particle size of the droplets generated in the dispersion was measured and the particle size distribution at room temperature (about 25 °C) was assessed. 4 μL of each formulation was added to the vial, and then 396 μL of water was added, and then the mixture was stirred. The obtained samples were subjected to particle size distribution by dynamic scattering (DLS) according to the method shown in Test method 1. The average particle size (Z-average size) and polydispersity index (PDI) were used as indicators for the particle size distribution. In DLS, PDI is an indicator representing the width of the particle size distribution, and is shown in the range of 0 to 1. PDI = 0 represents a suspension with no distribution of particle size. A dispersion having PDI of 0.1 or less is considered as having monodispersity, and a dispersion having PDI between 0.1 and 0.5 is considered to have a narrow distribution. While, a dispersion having PDI of greater than 0.5 is considered as having polydispersity. As shown in Table 16, formulations A to V were all good dispersions having a small average particle size (less than 200 nm) and narrow distribution of PDI of less than 0.5. While, formulations W to AO had a large average particle size (200 nm or more) and polydispersity with PDI of greater than 0.5.

[Table 16]

DLS Results

| | Formulation name | Average particle size (nm) | PDI |
|---|---|---|---|
| Example 1 | A | 158.4 | 0.158 |
| Example 2 | B | 116.9 | 0.455 |
| Example 3 | C | 92.23 | 0.265 |
| Example 4 | D | 105.7 | 0.496 |
| Example 5 | E | 157.5 | 0.233 |
| Example 6 | F | 114.7 | 0.235 |
| Example 7 | G | 89.66 | 0.207 |
| Example 8 | H | 81.73 | 0.155 |
| Example 9 | I | 156.4 | 0.238 |
| Example 10 | J | 164.7 | 0.25 |
| Example 11 | K | 139.7 | 0.195 |
| Example 12 | L | 170.7 | 0.276 |
| Example 13 | M | 142.2 | 0.426 |
| Example 14 | N | 76.01 | 0.256 |
| Example 15 | O | 64.52 | 0.24 |
| Example 16 | P | 94.48 | 0.2 |
| Example 17 | Q | 95.09 | 0.234 |
| Example 18 | R | 92.67 | 0.282 |
| Example 19 | S | 99.12 | 0.451 |
| Example 20 | T | 198.1 | 0.451 |
| Example 21 | U | 153.9 | 0.166 |
| Example 22 | V | 93.53 | 0.37 |
| Comparative Example 1 | W | 2314 | 1 |
| Comparative Example 2 | X | 2450 | 0.843 |
| Comparative Example 3 | Y | 291.6 | 0.661 |
| Comparative Example 4 | Z | 1267 | 0.743 |
| Comparative Example 5 | AA | 3492 | 1 |
| Comparative Example 6 | AB | 3439 | 0.874 |
| Comparative Example 7 | AC | 5166 | 0.358 |
| Comparative Example 8 | AD | 1160 | 1 |
| Comparative Example 9 | AE | 1290 | 0.999 |
| Comparative Example 10 | AF | 1391 | 1 |
| Comparative Example 11 | AG | 2226 | 0.971 |
| Comparative Example 12 | AH | 325.7 | 0.894 |

(continued)

DLS Results

| | Formulation name | Average particle size (nm) | PDI |
|---|---|---|---|
| Comparative Example 13 | AI | 244.5 | 0.573 |
| Comparative Example 14 | AJ | 1707 | 0.411 |
| Comparative Example 15 | AK | 2889 | 0.49 |
| Comparative Example 16 | AL | 4400 | 0.386 |
| Comparative Example 17 | AM | 3963 | 0.558 |
| Comparative Example 18 | AN | 3338 | 0.559 |
| Comparative Example 19 | AO | 207.5 | 0.737 |

Test Example 4 (Stability test at 80°C)

Stability tests were conducted to estimate the long-term stability of the pharmaceuticals. First, 10 mg each of formulations A, C, F, H, and J prepared in Examples 1, 3, 6, 8, and 10 was taken into a glass vial. The vial was placed in a brown bottle containing silica gel and deoxidizer, and sealed. Each brown bottle was placed in an 80°C thermostatic bath and stored for 2 weeks. The concentration of Compound I before and after storage was quantified by UPLC, and the residual rate was determined by the following equation.

Residual rate (%) = (Concentration of Compound I after storage/Concentration of Compound I before storage) X 100

As shown in Table 17, any formulation has a high Compound I residual rate of 95% or more and was found to be a formulation that could be developed as a pharmaceutical.

[Table 17]

Residual rate of Compound I

| | Formulation name | 80° C 2 weeks |
|---|---|---|
| Example 1 | A | 95. 3 ± 0. 6 |
| Example 3 | C | 96. 8 ± 0.8 |
| Example 6 | F | 100. 3 ± 0.7 |
| Example 8 | H | 100.4 ± 0.3 |
| Example 10 | J | 100.7 ± 1.0 |

Test Example 5 (Stability test at 40°C)

Since the self-emulsifying formulation is a liquid, it is mainly filled into a capsule formulation to be developed as a pharmaceutical. Then, 10 mg each of formulations A, C, and F prepared in Examples 1, 3, and 6 was taken into a hard capsule. The vial was placed in a brown bottle containing silica gel and deoxidizer, and sealed. Each brown bottle was placed in a thermostatic bath at 40°C and stored for 6 months. The concentration of Compound I before and after storage was quantified by UPLC, and the residual rate was determined by the following equation.

Residual rate (%) = (Concentration of Compound I after storage/Concentration of Compound I before storage) X 100

As shown in Table 18, any formulation has a high Compound I residual rate of 95% or more and was found to be a formulation that could be developed as a pharmaceutical.

[Table 18]

Residual rate of Compound I after storage

| | Formulation name | 40° C 6 months |
|---|---|---|
| Example 1 | A | 99. 9 ± 0. 9 |
| Example 3 | C | 101. 2 ± 1. 4 |
| Example 6 | F | 101. 1 ± 0.7 |

Test Example 6 (Dispersity assessment)

To estimate the dispersity of formulations C and F and formulation AJ for comparison into fasted state simulated intestinal fluid (FaSSIF) according to the dispersity assessment shown in Test method 2, the dispersion profile was analyzed using a pDISS device that provides a controlled temperature and stirring rate (37°C, 200 rpm). 100 μL of each formulation was added to 10 mL of FaSSIF surface warmed at 37 °C and stirred at a rate of 200 rpm. At the timepoints of 5, 10, 15, 20, 25, 30 and 60 minutes, 50 μL of the solution was aliquoted from the center of the container through the guide plastic tube, and the concentration of Compound I was determined by UPLC. The dispersity (%) was determined by the following equation.

Dispersity (%) = (concentration of Compound I in aliquoted solution/concentration of Compound I when entire formulation is uniformly dispersed) X 100

As shown in Figure 5, it was found that the entire amount of formulation C dispersed immediately after the start of the test. Formulation F also showed a dispersity of more than 80% at the timepoint of 60 minutes, and was found to be a good formulation compared to formulation AJ.

The formulations AP to AS were then prepared for absorption assessment in rats.

Example 23 (Preparation of capsule formulation AP for administration to rat)

Compound I was weighed into a vial, and 1 mL of additive mixed solution 3 per 60 mg of Compound I was added. The mixture was stirred with a stirrer, and Compound I was confirmed to be completely dissolved. The solution in which the compound was dissolved was filled into a No. 9 gelatin capsule to prepare formulation AP.

Example 24 (Preparation of capsule formulation AQ for administration to rat)

Compound I was weighed into a vial, and 1 mL of additive mixed solution 19 per 60 mg of Compound I was added. The mixture was stirred with a stirrer, and Compound I was confirmed to be completely dissolved. The solution in which the compound was dissolved was filled into a No. 9 gelatin capsule to prepare formulation AQ.

Comparative Example 20 (Preparation of capsule formulation AR for administration to rat)

Compound I was weighed into a vial, and 1 mL of additive mixed solution 31 per 60 mg of Compound I was added. The mixture was stirred with a stirrer, and Compound I was confirmed to be completely dissolved. The solution in which the compound was dissolved was filled into a No. 9 gelatin capsule to prepare formulation AR.

Comparative Example 21 (Preparation of solution formulation AS for administration to rat)

Compound I was weighed into a vial, and 1 mL of dimethyl sulfoxide (DMSO) per 10 mg of Compound I was added and dissolved. Further, 0.5 mL of polyoxyl 35 castor oil and 8.5 mL of water were added, well stirred, and mixed to prepare solution formulation AS.

The % by weight of each ingredient contained in the formulations of Examples 23 and 24 and Comparative Examples 20 based on the entire of the formulation was summarized in the table below.

[Table 19]

| Example No. | Example 23 | Example 24 | Comparative Example 20 |
|---|---|---|---|
| Formulation name | AP | AQ | AR |

(continued)

| Example No. | Example 23 | Example 24 | Comparative Example 20 |
|---|---|---|---|
| Compound I | 5.9 | 5.7 | 5.7 |
| Propylene glycol monocaprylate | 50.2 | 48.3 | 53.0 |
| Oleic acid | 13.1 | | |
| Triacetin | | 16.4 | 11.0 |
| Polyoxyl 35 castor oil | 29.9 | 28.7 | |
| D-α-Tocopherol polyethylene glycol 1000 succinate | | | |
| PEG-8 Caprylic/Capric Glycerides | | | 29.4 |
| Polysorbate 80 | | | |
| Propylene glycol | | | |
| dl-α-tocopherol | 0.9 | 0.9 | 0.9 |
| Total | 100.0 | 100.0 | 100.0 |

Test Example 7 (Absorption assessment in rat)

Capsule formulations AP and AQ of Examples 23 and 24, and capsule formulation AR and solution formulation AS of Comparative Examples 20 and 21 were orally administered to rats (Wister Hannover, male) at a volume of 10 mg/kg in 4 cases for each formulation. Formulations AP, AQ, and AR were directly administered orally as capsule formulations, and water was further administered orally at a volume of 5 mL/kg. Solution formulation AS was directly administered orally. Blood sampling was performed at about 0.5, 1, 2, 4, 7, 24, and 48 hours after administration, and the concentration of Compound I contained in plasma was quantified by LC-MS/MS according to the method shown in Test method 3, and the plasma drug concentration-area under the time curve (AUC) and the maximum plasma concentration (Cmax) were calculated.

The AUC and Cmax after administration of each formulation are shown in Table 20. Capsule formulations AP and AQ of Examples 23 and 24 were found to exhibit higher AUC and Cmax than capsule formulation AR of Comparative Example 20. It was also found that capsule formulation AP of Example 23 exhibited comparable AUC and Cmax compared to solution formulation AS of Comparative Example 21.

[Table 20]

AUC and Cmax on administrations of formulations AP to AS (mean ± SD in 4 cases)

| | Formulation | AUC (ng*h/mL) | $C_{max}$ (ng/mL) |
|---|---|---|---|
| Example 23 | AP | 10,400±1,930 | 2, 500±456 |
| Example 24 | AQ | 5,510±1,160 | 1, 280±250 |
| Comparative Example 20 | AR | 3, 360±700 | 802±210 |
| Comparative Example 21 | AS | 10, 600±3, 400 | 1, 900±990 |

Next, capsule formulation AT and solution formulation AU were prepared for absorption assessment in monkeys.

Example 25 (Preparation of capsule formulation AQ for administration to monkey)

Compound I was weighed into a vial, and 1 mL of additive mixed solution 3 per 50 mg of Compound I was added. The mixture was stirred with a stirrer, and Compound I was confirmed to be completely dissolved. The solution in which the compound was dissolved was filled into a No. 1 gelatin capsule to prepare formulation AT.

Comparative Example 22 (Preparation of solution formulation AU for administration to monkey)

Compound I was weighed into a vial, and 1 mL of dimethyl sulfoxide (DMSO) per 15 mg of Compound I was added and dissolved. Further, 0.25 mL of polyoxyl 35 castor oil and 8.75 mL of water were added, well stirred, and mixed to prepare solution formulation AU.

Test Example 8 (Absorption assessment in monkey)

Capsule formulation AT of Example 25 and solution formulation AU of Comparative Example 22 were orally administered to cynomolgus monkey at a volume of 3 mg/kg in 4 cases for each formulation. Capsule formulation AT was directly administered orally and water was further administered orally at a volume of 4 mL/kg. Solution formulation AU was directly administered orally. Blood sampling was performed at about 0.5, 1, 2, 4, 7, 24, and 48 hours after administration, and the concentration of Compound I contained in plasma was quantified by LC-MS/MS according to the method shown in Test method 3, and the plasma drug concentration-area under the time curve (AUC) and the maximum plasma concentration (Cmax) were calculated.

The AUC and Cmax after administration of each formulation are shown in Table 21. It was found that capsule formulation AT of Example 25 exhibited comparable AUC and Cmax compared to solution formulation AU of Comparative Example 22.

[Table 21]

AUC and Cmax on administrations of formulations AT and AU (mean ± SD in 4 cases)

| | Formulation | AUC (ng*h/mL) | $C_{max}$ (ng/mL) |
|---|---|---|---|
| Example 25 | AT | 5,420±2,750 | 915±530 |
| Comparative Example 22 | AU | 3, 390±2, 310 | 634±448 |

Example 26 (Production 1 of capsule formulation (hard capsule))

Compound I was weighed into a stainless steel container, then 51.5 mg of propylene glycol monocaprylate, 13.3 mg of oleic acid, 31.1 mg of polyoxyl 35 castor oil, and 0.2 mg of dl-α-tocopherol per 0.5 mg of Compound I were added, and the mixture was stirred and mixed with a homogenizer. The solution in which the compound was dissolved was filled into a No. 3 gelatin capsule, and the capsule was band-sealed to prepare formulation AV.

Example 27 (Production 2 of capsule formulation (hard capsule))

Compound I was weighed into a stainless steel container, then 123.6 mg of propylene glycol monocaprylate, 31.9 mg of oleic acid, 74.6 mg of polyoxyl 35 castor oil, and 0.5 mg of dl-α-tocopherol per 12 mg of Compound I were added, and the mixture was stirred and mixed with a homogenizer. The solution in which the compound was dissolved was filled into a No. 3 gelatin capsule, and the capsule was band-sealed to prepare formulation AW.

Example 28 (Production 3 of capsule formulation (soft capsule))

Compound I was weighed into a stainless steel container, then 206.4 mg of propylene glycol monocaprylate, 53.3 mg of oleic acid, 124.6 mg of polyoxyl 35 castor oil, and 0.8 mg of dl-α-tocopherol per 19.1 mg of Compound I were added, and the mixture was stirred and mixed with a stirring blade. The solution in which the compound was dissolved was filled into a soft gelatin capsule to prepare formulation AX.

Example 29 (Production 4 of capsule formulation (soft capsule))

Compound I was weighed into a stainless steel container, then 46.4 mg of propylene glycol monocaprylate, 12.0 mg of oleic acid, and 28.0 mg of polyoxyl 35 castor oil per 4.5 mg of Compound I were added, and the mixture was stirred and mixed with a stirring blade. The solution in which the compound was dissolved was filled into a soft gelatin capsule to prepare formulation AY.

Example 30 (Production 5 of capsule formulation (soft capsule))

Compound I was weighed into a stainless steel container, then 61.8 mg of propylene glycol monocaprylate, 16.0 mg of oleic acid, and 37.3 mg of polyoxyl 35 castor oil per 6.0 mg of Compound I were added, and the mixture was stirred and mixed with a stirring blade. The solution in which the compound was dissolved was filled into a soft gelatin capsule to prepare formulation AZ.

The % by weight of each ingredient contained in the formulations of Examples 25 to 30 based on the entire of the formulation was summarized in the table below.

[Table 22]

| Example No. | Example 25 | Example 26 | Example 27 | Example 28 | Example 29 | Example 30 |
|---|---|---|---|---|---|---|
| Formulation name | AT | AV | AW | AX | AY | AZ |
| Compound I | 5.0 | 0.5 | 5.0 | 4.7 | 5.0 | 5.0 |
| Propylene glycol monocaprylate | 50.7 | 53.3 | 50.9 | 51.1 | 51.0 | 51.0 |
| Oleic acid | 13.2 | 13.8 | 13.1 | 13.2 | 13.2 | 13.2 |
| Triacetin | | | | | | |
| Polyoxyl 35 castor oil | 30.2 | 32.2 | 30.8 | 30.8 | 30.8 | 30.8 |
| D-α-Tocopherol polyethylene glycol 1000 succinate | | | | | | |
| PEG-8 Caprylic/Capric Glycerides | | | | | | |
| Polysorbate 80 | | | | | | |
| Propylene glycol | | | | | | |
| dl-α-tocopherol | 0.9 | 0.2 | 0.2 | 0.2 | | |
| Total | 100.0 | 100.0 | 100.0 | 100.0 | 100.0 | 100.0 |

Test Example 9 (Particle size distribution measurement by DLS)

Next, the particle size distribution of formulation AV, formulation AW, formulation AX, formulation AY, and formulation AZ prepared in Examples 26 to 30 when dispersed in water was assessed. To the vial, 9.9 mL of Japanese Pharmacopeia 1st Fluid for dissolution test, pH 1.2 was added, and further 100 μL of each formulation was added, and then the mixture was stirred. The stirred sample was aliquoted by 100 μL, and added to a vial containing 0.9 mL of Japanese Pharmacopeia 1st Fluid for dissolution test, pH 1.2, and then the mixture was stirred. The particle size distribution by dynamic scattering (DLS) was determined for these samples according to the method shown in Test method 1. The average particle size (Z-average size) and polydispersity index (PDI) are used as indicators for the particle size distribution as in Test Example 3. As shown in Table 23, formulation AV, formulation AW, formulation AX, formulation AY, and formulation AZ were all good dispersions having a small average particle size (less than 200 nm) and a narrow distribution with PDI of less than 0.5.

[Table 23]

| DLS Results | | | |
|---|---|---|---|
| | Formulation name | Average particle size (nm) | PDI |
| Example 26 | AV | 78.16 | 0.20 |
| Example 27 | AW | 78.35 | 0.21 |
| Example 28 | AX | 79.49 | 0.17 |
| Example 29 | AY | 79.54 | 0.15 |
| Example 30 | AZ | 80.53 | 0.18 |

Test Example 10 (Pharmacological test example)

Kras-SOS1 protein-protein interaction inhibition (KrasG12D-SOS PPI) using AlphaScreen

Kras-SOS1 protein-protein interaction inhibition (PPI) can be performed by a method known to those skilled in the art using a device, a reagent, or the like available from commercial suppliers. Specifically, biotin-tagged human Kras

and His-tagged human SOS1 enzymes expressed in E. coli and loaded with GDP after purification were used to measure PPI by energy transfer from nickel-bound donor beads to streptavidin-bound acceptor beads. This measurement is performed by irradiating the donor beads with light of 680 nm to induce energy transfer to the acceptor beads via singlet oxygen, and detecting light of 520 to 620 nm from the acceptor beads. Based on the inhibition of the control group without Compound 1, 50% inhibitory concentration ($IC_{50}$ value) was calculated.

The $IC_{50}$ value of Compound 1 was 0.0010 μM. Compound 1 was shown to inhibit the protein-protein interaction between Kras and SOS1. Inhibition of the binding of K-ras and SOS1 is known to inhibit signal transduction downstream of Kras. It was thus suggested that Compound 1 has cell proliferation inhibitory activity.

Measurement of AsPC-1 cell growth inhibitory activity (AsPC-1CGI)

AsPC-1 cell growth inhibitory activity can be performed by methods known to those skilled in the art using devices, reagents, cell lines, and the like available from commercial suppliers. Specifically, Compound 1 was serially diluted with dimethyl sulfoxide, then 200 nL of the dilution was dispensed into the U-bottom 96 well plate with Labcyte Echo. Human pancreatic cancer strain AsPC-1 was added to RPMI-1640 medium supplemented with 15% bovine fetal serum to prepare a cell suspension of 1000 cells/100 μL. The cell suspension was dispensed by 100 μL per well to Compound 1-added plates, and cultured in a 37°C, 5% carbon dioxide gas incubator. After 96 hours, 80 μL of CellTiter-Glo (Promega Corporation) was added to each well, and fluorescence was measured. Cell growth inhibition (CGI) of the test compound was calculated as 50% growth inhibition concentration ($IC_{50}$ value) from the growth inhibition rate of the case with addition of Compound 1 to the control without addition of Compound 1. The $IC_{50}$ value (μM) of Compound 1 was 0.02 μM or less, thus it was shown that Compound 1 has an anti-tumor effect.

[Industrial Applicability]

The composition according to the present invention contains specific ingredients for specific compounds, thereby the compositions of the present invention can be excellent in various properties required as a formulation. For example, the composition containing such specific ingredients has desirable particle properties, excellent stability and dispersity of the active ingredient, and excellent absorption into the body. Thus, the composition according to the present invention is suitable for use as a pharmaceutical formulation.

## Claims

1. A composition comprising: a compound represented by the following Formula (1):

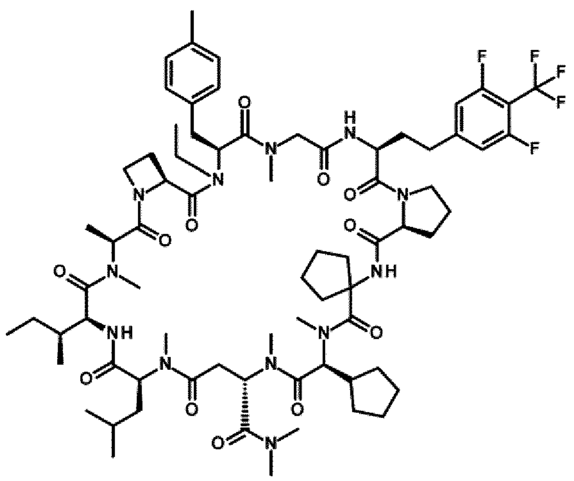

(1)

or a salt thereof, or a solvate thereof; a liquid additive; and optionally an oily component.

2. The composition according to claim 1, wherein the liquid additive is a surfactant.

3. The composition according to claim 2, wherein the surfactant is a combination of a hydrophobic surfactant and a hydrophilic surfactant.

4. The composition according to claim 3, wherein an HLB value of the hydrophobic surfactant is 0 or more and less than 10, and an HLB value of the hydrophilic surfactant is 10 or more and 30 or less.

5. The composition according to claim 3 or 4, wherein the hydrophobic surfactant is at least one selected from the group consisting of a propylene glycol fatty acid ester, a glyceryl fatty acid ester, a polyglyceryl fatty acid ester, a sorbitan fatty acid ester, and a hydrophobic polyoxyethylene hydrogenated castor oil.

6. The composition according to any one of claims 3 to 5, wherein the hydrophilic surfactant is at least one selected from the group consisting of a polyethylene glycol fatty acid ester, a polyoxyethylene castor oil, a hydrophilic polyoxyethylene hydrogenated castor oil, a polyoxyethylene sorbitan fatty acid ester, D-α-tocopheryl polyethylene glycol 1000 succinate, a caprylocaproyl polyoxyl-8 glyceride, and a combination thereof.

7. The composition according to any one of claims 1 to 6, wherein the composition comprises an oily component.

8. The composition according to any one of claims 1 to 7, further comprising an antioxidant.

9. The composition according to any one of claims 1 to 8, wherein the compound represented by Formula (1) or a salt thereof, or a solvate thereof, is a compound represented by Formula (1) or a hydrate thereof.

10. The composition according to any one of claims 1 to 9, wherein a content of the compound represented by Formula (1) or a salt thereof, or a solvate thereof, based on the total composition is 10% by weight or less.

11. The composition according to any one of claims 1 to 10, wherein a content of the liquid additive based on the total composition is 50% by weight or more and 97% by weight or less.

12. The composition according to any one of claims 1 to 11, wherein a weight ratio of the liquid additive to the compound represented by Formula (1) or a salt thereof, or a solvate thereof, is 5 or more.

13. A pharmaceutical formulation comprising the composition according to any one of claims 1 to 12.

14. A method for producing the composition according to any one of claims 1 to 12, comprising the steps of:

(1) providing a compound represented by the following Formula (1):

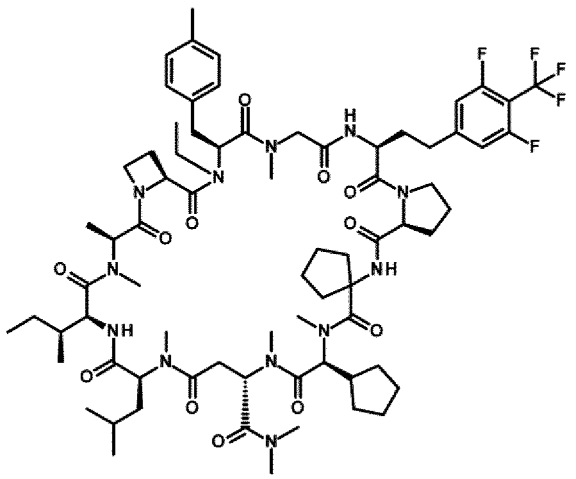

(1)

or a salt thereof, or a solvate thereof, a liquid additive, and optionally an oily component;
(2) mixing the compound represented by Formula (1) or a salt thereof, or a solvate thereof, the liquid additive, and optionally the oily component; and
(3) obtaining a composition in which the compound represented by Formula (1) or a salt thereof, or a solvate thereof is dissolved.

15. The method according to claim 14, wherein the compound represented by Formula (1) or a salt thereof, or a solvate

thereof, provided in step (1) is a hydrate of the compound represented by Formula (1).

Fig.1

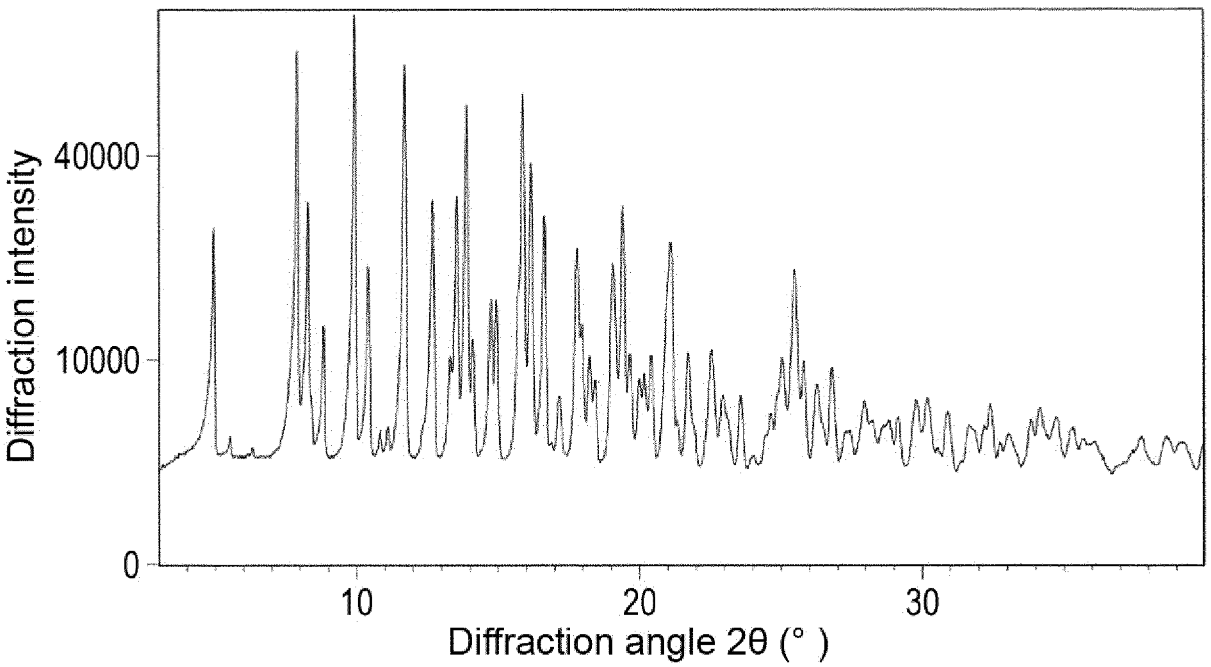
Diffraction intensity
40000
10000
0
10
20
30
Diffraction angle 2θ (° )

Fig.2

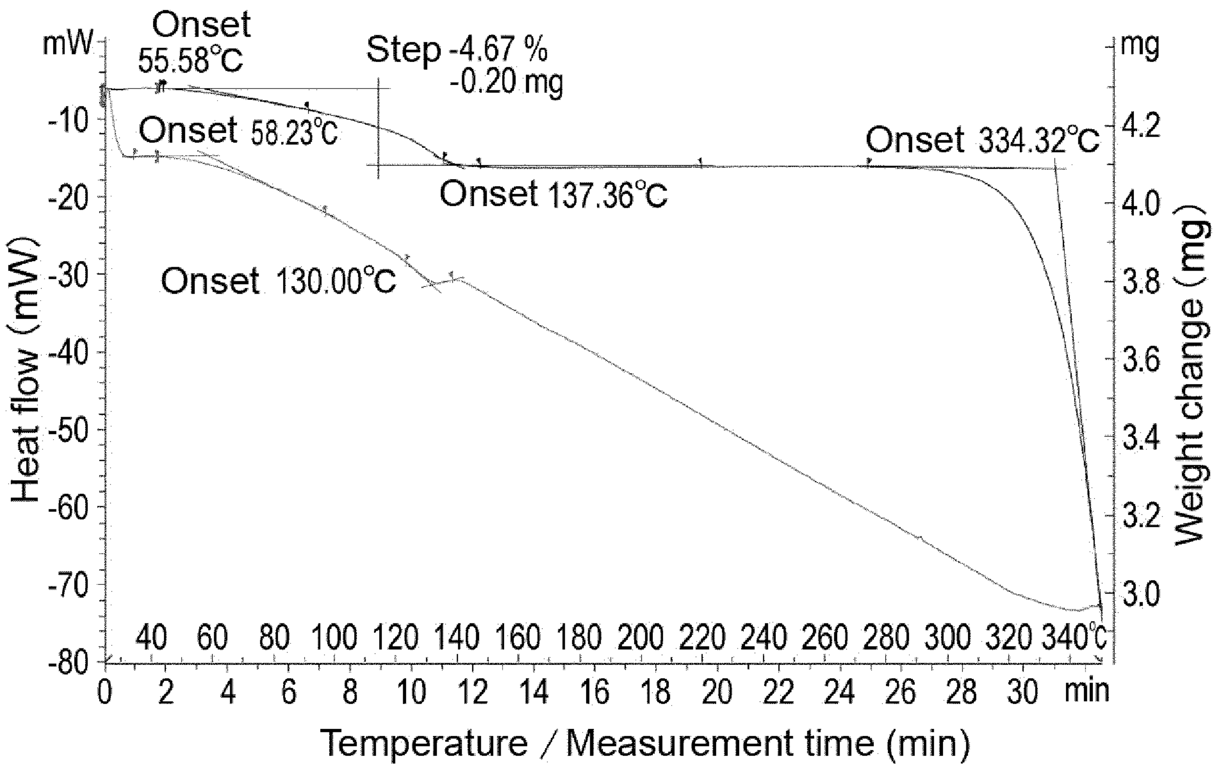
mW
Onset
55.58℃
Step -4.67 %
-0.20 mg
mg
Onset 58.23℃
Onset 334.32℃
Onset 137.36℃
Onset 130.00℃
-10
-20
-30
-40
-50
-60
-70
-80
4.2
4.0
3.8
3.6
3.4
3.2
3.0
Heat flow (mW)
Weight change (mg)
40 60 80 100 120 140 160 180 200 220 240 260 280 300 320 340℃
0 2 4 6 8 10 12 14 16 18 20 22 24 26 28 30 min
Temperature / Measurement time (min)

Fig.3

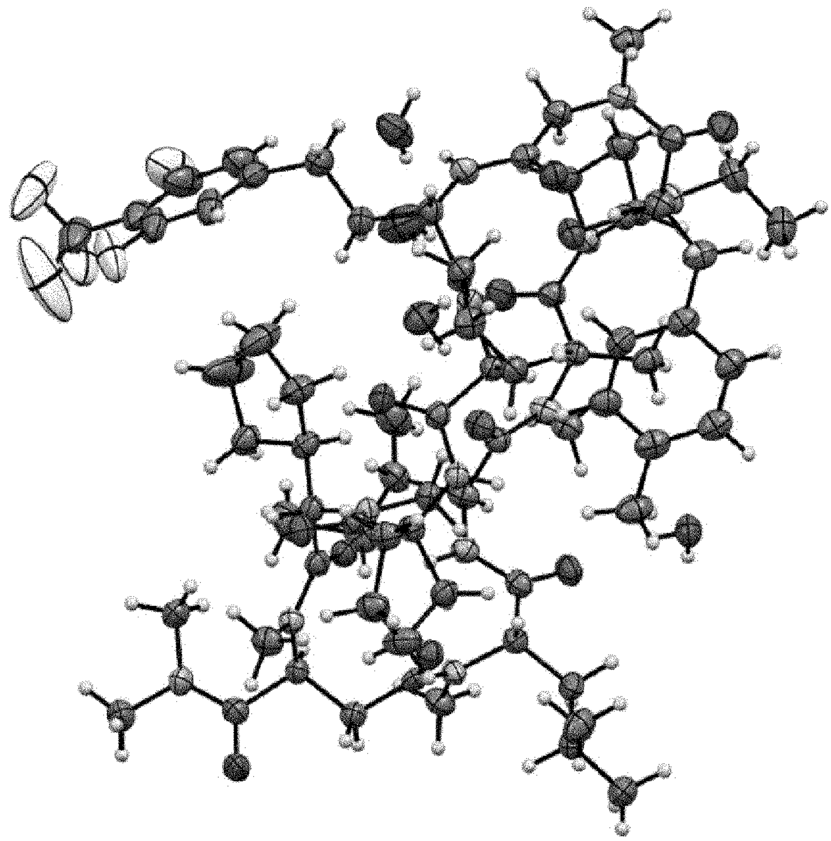

*Fig.4*

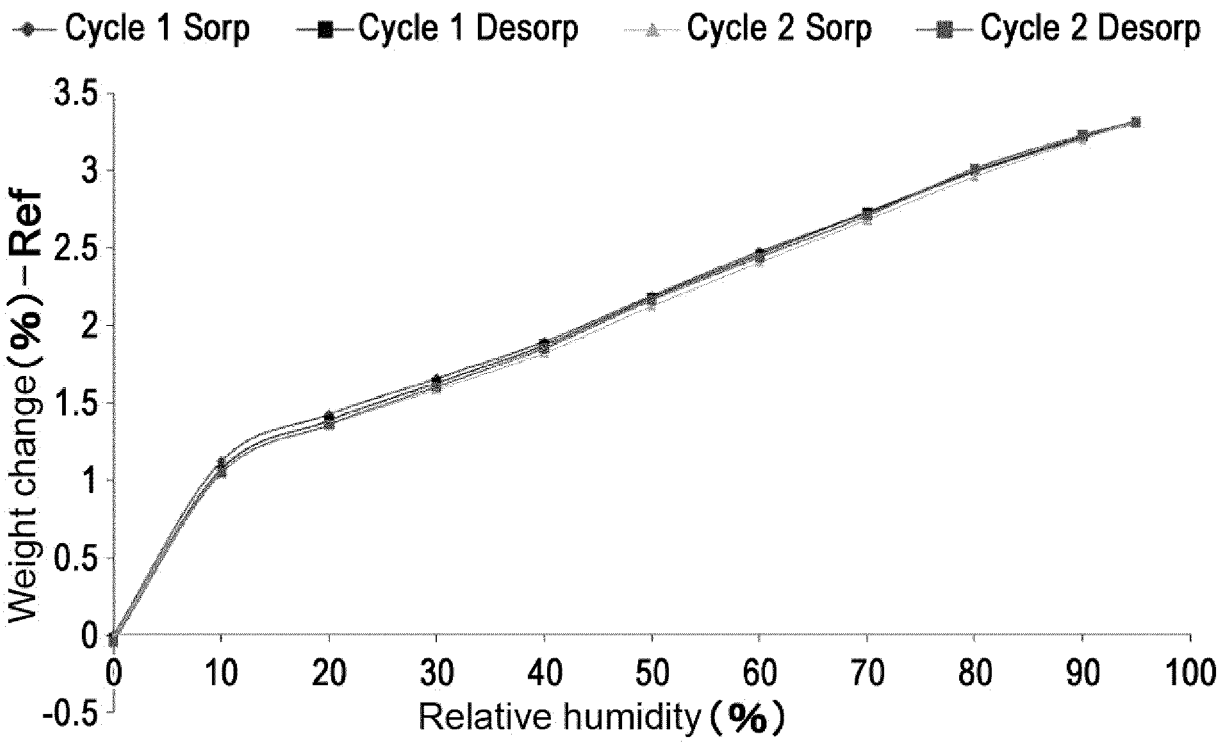
Cycle 1 Sorp
Cycle 1 Desorp
Cycle 2 Sorp
Cycle 2 Desorp
Weight change (%) - Ref
3.5
3
2.5
2
1.5
1
0.5
0
-0.5
0
10
20
30
40
50
60
70
80
90
100
Relative humidity (%)

*Fig.5*

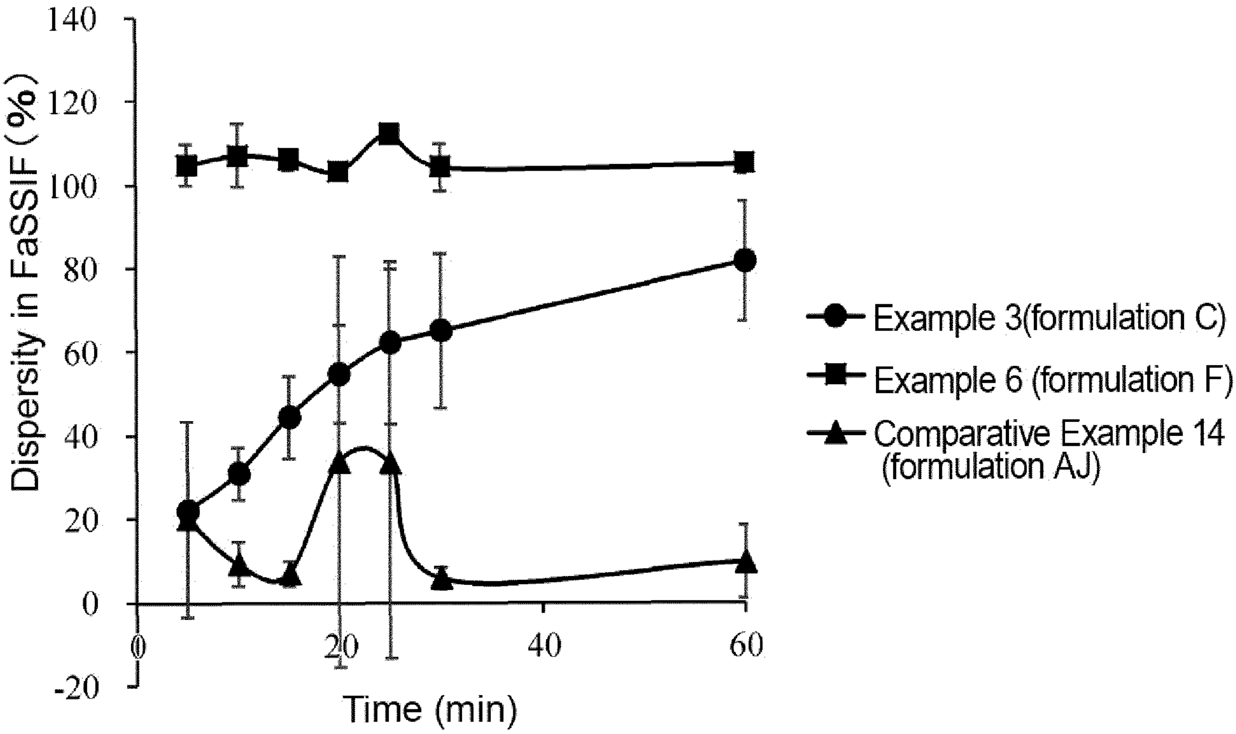
Dispersity in FaSSIF (%)
140
120
100
80
60
40
20
0
-20
0
20
40
60
Time (min)
Example 3(formulation C)
Example 6 (formulation F)
Comparative Example 14
(formulation AJ)

**INTERNATIONAL SEARCH REPORT**

International application No.
**PCT/JP2022/019540**

**A. CLASSIFICATION OF SUBJECT MATTER**

***A61K 38/12***(2006.01)i; ***A61K 47/10***(2006.01)i; ***A61K 47/14***(2006.01)i; ***A61K 47/26***(2006.01)i; ***A61K 47/44***(2017.01)i; ***A61P 35/00***(2006.01)i; ***A61P 43/00***(2006.01)i
FI: A61K38/12; A61P35/00; A61P43/00 111; A61K47/14; A61K47/10; A61K47/26; A61K47/44

According to International Patent Classification (IPC) or to both national classification and IPC

**B. FIELDS SEARCHED**

Minimum documentation searched (classification system followed by classification symbols)

A61K38/12; A61K47/10; A61K47/14; A61K47/26; A61K47/44; A61P35/00; A61P43/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| P, X | WO 2021/090855 A1 (CHUGAI PHARMACEUTICAL CO LTD) 14 May 2021 (2021-05-14) in particular, claims, compound 1217 | 1-15 |
| A | 古本健太朗，他, 難溶性化合物の溶解性改善を目的としたハイスループット添加剤スクリーニング法の確立, 薬剤学, vol. 70, suppl., 2010, p. 192 (P-5) in particular, 2nd paragraph, (FURUMOTO, Kentaro et al. Journal of Pharmaceutical Science and Technology, Japan.), non-official translation (Establishment of the high-throughput additive screening method for the purpose of improving solubility of the insoluble compound) | 1-15 |
| A | 青木雅英，他, 原薬－添加剤間のCocrystal形成による溶解性低下減少の解明, 日本薬剤学会年会講演要旨集, vol. 29, 2014, p. 193 pp. 20-32) in particular, 2nd, 3rd paragraphs, (AOKI, Masahide et al.), non-official translation (Elucidation of decrease in deterioration of solubility due to cocrystal formation between drug substance and additives. Lecture abstracts of the Annual Meeting of the Academy of Pharmaceutical Science and Technology Japan.) | 1-15 |

☑ Further documents are listed in the continuation of Box C.
☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 July 2022** | **19 July 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2022/019540**

**C. DOCUMENTS CONSIDERED TO BE RELEVANT**

| Category* | Citation of document, with indication, where appropriate, of the relevant passages | Relevant to claim No. |
|---|---|---|
| A | WO 2013/100132 A1 (CHUGAI PHARMACEUTICAL CO LTD) 04 July 2013 (2013-07-04) in particular, claims, paragraph [0015], etc. | 1-15 |

Form PCT/ISA/210 (second sheet) (January 2015)

**INTERNATIONAL SEARCH REPORT**
**Information on patent family members**

International application No.
**PCT/JP2022/019540**

| Patent document cited in search report | Publication date (day/month/year) | Patent family member(s) | Publication date (day/month/year) |
|---|---|---|---|
| WO 2021/090855 A1 | 14 May 2021 | JP 6880352 B<br>in particular, claim 2, compound 1217<br>KR 10-2021-0064234 A | |
| WO 2013/100132 A1 | 04 July 2013 | US 2015/0080549 A1<br>claims<br>EP 2813512 A1<br>TW 201333029 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013100132 A
- WO 2018225864 A
- WO 2020122182 A
- WO 2012122059 A
- WO 2017181061 A
- WO 2018031730 A
- WO 2016071515 A


### Non-patent literature cited in the description


- *Adv. Drug Del. Rev.,* 1997, vol. 23, 3-25
- *Future Med. Chem.,* 2009, vol. 1, 1289-1310
- *Acc. Chem. Res.,* 2008, vol. 41, 1331-1342
- *Angew. Chem. Int. Ed.,* 2013, vol. 52, 254-269
- *Chem. Rev.,* 2019, vol. 119, 10360-10391
- *Ther. Deliv.,* 2017, vol. 8 (10), 867-878
- Japanese Pharmacopeia
- *CHEMICAL ABSTRACTS,* 146982-24-3
- *CHEMICAL ABSTRACTS,* 220497-61-0
- *CHEMICAL ABSTRACTS,* 117322-30-2
- *CHEMICAL ABSTRACTS,* 30924-93-7
- *CHEMICAL ABSTRACTS,* 72914-19-3
- *CHEMICAL ABSTRACTS,* 156243-64-0
- *CHEMICAL ABSTRACTS,* 199006-54-7
- *CHEMICAL ABSTRACTS,* 71989-31-6
- *CHEMICAL ABSTRACTS,* 77128-70-2
- *CHEMICAL ABSTRACTS,* 136552-06-2
- *CHEMICAL ABSTRACTS,* 84000-07-7
- *CHEMICAL ABSTRACTS,* 71989-23-6
- *CHEMICAL ABSTRACTS,* 330645-87-9